# EUROPÄISCHE PATENTSCHRIFT

(11) **EP 0 572 852 B1**
(45) Veröffentlichungstag und Bekanntmachung des Hinweises auf die Patenterteilung: **21.10.1998**
(21) Anmeldenummer: 93107984.2
(22) Anmeldetag: 17.05.1993
(51) Int. Cl.: C07D 403/04, C07D 487/14, A61K 31/495

(54) **Chinoxalin-2,3(1H,4H)-dione als Arzneimittel**
Quinoxalin-2,3(1H,4H)-diones used as medicine
Quinoxalines-2,3(1H,4H)-diones utiles comme médicament

(30) Priorität: 30.05.1992 DE 4217952
(43) Veröffentlichungstag der Anmeldung: 08.12.1993
(73) Patentinhaber: BASF Aktiengesellschaft, 67063 Ludwigshafen (DE)
(72) Erfinder: Lubisch, Wilfried, Dr., W-6800 Mannheim 1 (DE); Behl, Berthold, Dr., 67117 Limbergerhof (DE); Hofmann, Hans Peter, Dr., W-6703 Limburgerhof (DE)

(56) Entgegenhaltungen:
- EP-A- 0 311 135

## Beschreibung

Die vorliegende Erfindung betrifft neue Chinoxalin-2,3-(1H,4H)-dione der allgemeinen Formel I 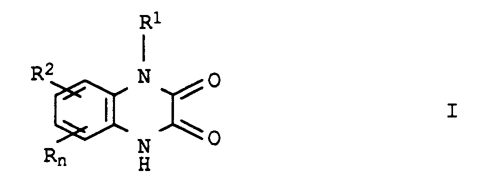 und ihre tautomeren und enantiomeren Formen sowie ihre physiologisch verträglichen Salze, wobei die Variablen folgende Bedeutung haben:
- R¹: - Wasserstoff,
- ein cycloaliphatischer Rest mit bis zu 8 C-Atomen,
- eine Phenylgruppe,
- ein aliphatischer Rest mit 1 bis 12 C-Atomen, der einen oder zwei gleiche oder verschiedene der Substituenten Phenyl, Cyclopentyl, Cyclohexyl
-CO-R³, -CO-O-R³, -CO-NH-R³, -OR³, -NR⁷R³, 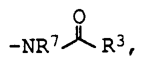 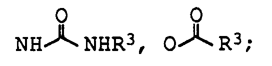 =N-OR³, -CN
tragen kann, wobei R³ und R⁷ unabhängig voneinander Wasserstoff, C₁-C₄-Alkyl, Phenyl, Benzyl, 1-Phenylethyl oder 2-Phenylethyl bedeuten, und
wobei die in R¹ enthaltenen oder R¹ darstellenden aliphatischen und aromatischen Cyclen bis zu drei gleiche oder verschiedene der folgenden Substituenten tragen können: C₁-C₄-Alkyl, C₁-C₄-Halogenalkyl, C₁-C₄-Alkoxy, C₁-C₄-Halogenalkoxy, Halogen, Nitro, Cyano, -CO-OR⁹, - CO-NH-R⁹, -OH, 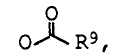 -NHR⁹, -NR⁹R¹⁰, 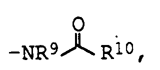 =N-OR⁹, =O; wobei R⁹ und R¹⁰ unabhängig voneinander Wasserstoff, C₁-C₄-Alkyl, Phenyl, Benzyl, 1-Phenylethyl und 2-Phenylethyl bedeuten,
- R²: - eine Pyrrol-1-ylgruppe, die einen oder zwei der folgenden Substituenten tragen kann:C₁-C₄-Alkyl, Phenyl, Phenylsulfonyl, Nitro, Cyano oder einen Substituent aus der Gruppe
-CO-O-R³, -CO-NH-R³, -CH₂-O-R³, -O-R³, -CH=NO-R³, -C(O)R³, 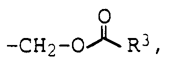 -CH₂NR⁷R³, 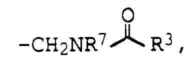 -CH=CH-R⁸, -CH=N-R³,
wobei R⁸ -COOR³, -CONH-R³, CN und Phenyl sein kann;
- R: - gleiche oder verschiedene der folgenden Reste: C₁-C₄-Alkyl, C₁-C₄-Alkoxy, Trifluormethyl, Trichlormethyl, Trifluormethoxy, Trichlormethoxy, Fluor, Chlor, Brom, Jod, Nitro, Cyano und eine der Gruppen -CO-O-R³, -CO-NH-R³, -SO₂R³ und 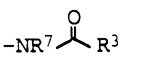 sowie einen anellierten Benzolring, der seinerseits bis zu drei der für R genannten Reste tragen kann;
- n: - eine ganze Zahl von 0 bis 3, für den anellierten Benzolring 0 oder 1.

Ferner betrifft die Erfindung Chinoxalin-2,3(1H,4H)-dione der allgemeinen Formel I' 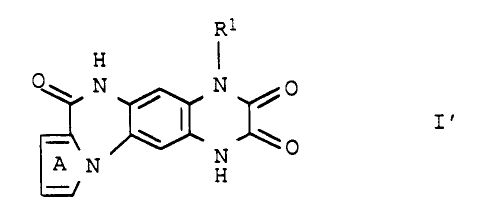 und ihre tautomeren und enantiomeren Formen sowie ihre physiologisch verträglichen Salze, wobei R¹ die in Anspruch 1 genannte Bedeutung hat und wobei der Ring A die in Anspruch 1 als Substituenten an der Pyrrol-1-ylgruppe genannten Reste tragen kann.

Außerdem betrifft die Erfindung Verfahren zur Herstellung der Verbindungen I und I' sowie deren Verwendung als Arzneimittel für die Human- und Veterinärmedizin.

Derivate des Chinoxalin-2,3(1H,4H)-dions 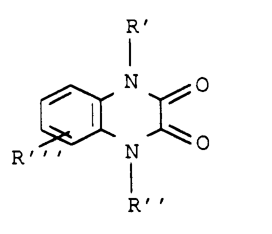 werden in zahlreichen Veröffentlichungen zur Behandlung von Erkrankungen des zentralen Nervensystems sowie als Schlaf- und Beruhigungsmittel vorgeschlagen. Beispielsweise werden in EP-A 315 858, EP-A 374 534 und EP-A 377 112 Verbindungen beschrieben, in denen R''' Halogen, Nitro, Cyano, Hydroxy, Trifluormethyl, C₁-C₄-Alkoxy und die Gruppen -SO₂H, SO₂R^{x}, -SONH₂, -SO₂NHR^{x} und SO₂NR₂^{x}, wobei R^{x} für C₁-C₄-Alkyl steht, sowie einen anellierten Benzolring, der auch substituiert sein kann, bedeuten. In US-A 3 992 378 handelt es sich bei dem Substituenten R''' um einen C₁-C₂-Fluoralkylrest und in PCT 91/13878 neben Halogen und Nitro um C₁-C₆-Alkyl, Alkoxy, Aryloxy und Aralkoxy.

Darüber hinaus sind in der EP-A 8864 auch Piperidinyl, Pyrrolidinyl und Piperazinyl als Substituenten R''' erwähnt. Verbindungen der letztgenannten Art sind auch aus Ind. J. Chem. 28B (1989), S. 888-890 bekannt; in einer darüber hinaus hier noch erwähnten Überprüfung ihrer Verwendbarkeit als Wurmmittel zur Bekämpfung von Haken- und Bandwürmern erwiesen sich diese Verbindungen als ungeeignet.

Die bekannten Verbindungen zeigen den Nachteil, daß sie die Blut-Hirn-Schranke nicht oder nur schlecht überwinden können und daher in ihrer Wirkung zu wünschen übrig lassen.

Der Erfindung lagen daher neue, wirksamere Chinoxalin-2,3-(1H,4H)-dione, ihre tautomeren und enantiomeren Formen sowie ihre physiologisch verträglichen Salze als Aufgabe zugrunde.

Demgemäß wurden die eingangs definierten Verbindungen I und I' gefunden.

Ferner wurden verschiedene, im folgenden näher beschriebene Verfahren für die Herstellung der Chinoxalin-2,3(1H,4H)-dione I und I' sowie deren Verwendung als Arzneimittel für die Human- und Veterinärmedizin gefunden.

Unter den Verbindungen I und I' sind solche Verbindungen zu verstehen, in denen die Variablen folgende Bedeutung haben:
- R¹: Wasserstoff,
ein aliphatischer Rest mit bis zu 12 C-Atomen, wie Methyl, Ethyl, n-Propyl, Isopropyl, n-Butyl, Isobutyl, sec.-Butyl, tert.-Butyl, n-Pentyl, tert.-Amyl, 1-Methylbut-1-yl, 2,2-Dimethylprop-1-yl, n-Hexyl, 1-Methylpent-1-yl, 1,1-Dimethylbut-1-yl, 1,1,2-Trimethylprop-1-yl, 1-Ethyl-1-methylprop-1-yl, 2,2-Diethyleth-1-yl, n-Heptyl, 1-Methylhex-1-yl, 1,1-Dimethylpent-1-yl, 1-Ethyl-1-methylbut-1-yl, 1,1-Diethylprop-1-yl, 1-Methyl-1-propylprop-1-yl, 1-Ethyl-1-propeth-1-yl, 1-Methyl-1-butyleth-1-yl, 1,1,2,2-Tetramethylprop-1-yl, n-Octyl, 1,1-Dimethylhex-1-yl, 1-Ethyl-1-methylpent-1-yl, 1,1-Diethylbut-1-y1, 1-Methyl-1-propylbut-1-yl, 1-Ethyl-1-propylprop-1-yl, 1,1,3,3-Tetramethylbut-1-yl, 1,1-Dimethylhept-1-yl, 1,1-Dimethyloct-1-yl, 1,1-Dimethylnon-1-yl, 1,1-Dimethyldec-1-yl, der einen der folgenden Substituenten tragen kann: Phenyl, Cyclopentyl, Cyclohexyl oder eine Gruppe
-CO-R³,
-CO-O-R³ oder
-CO-NH-R³
wobei R³ Wasserstoff, Methyl, Ethyl, n-Propyl, Isopropyl, n-Butyl, Isobutyl, sec.-Butyl, tert.-Butyl, Phenyl, Benzyl oder 1-Phenylethyl bedeutet;
ein cycloaliphatischer Rest mit bis zu 12 C-Atomen, wie Cyclopropyl, Cyclobutyl, Cyclopentyl, Cyclohexyl, Cycloheptyl, Cyclooctyl, Cyclononyl, Cyclodecyl, Cycloundecyl, Cyclododecyl, Cyclopentan-2-on-1-yl, Cyclohexan-2-on-1-yl, Cyclooctan-2-on-1-yl und die entsprechenden ungesättigten cycloaliphatischen Reste oder
eine Phenylgruppe,
wobei die in R¹ enthaltenen cyclischen Gruppen bis zu drei der folgenden Substituenten tragen können:
C₁-C₄-Alkyl wie Methyl, Ethyl, n-Propyl, Isopropyl, n-Butyl, Isobutyl, sec.-Butyl, tert.-Butyl, C₁-C₄-Alkoxy wie Methoxy, Ethoxy, n-Propoxy, Isopropoxy, n-Butoxy, Isobutoxy, sec.-Butoxy, tert.-Butoxy, C₁-C₄-Halogenalkyl, C₁-C₄-Halogenalkoxy, Halogen, Nitro, Cyano, -CO-O-R³ und -CO-NH-R³;
- R²: eine Pyrrol-1-ylgruppe, die bis zu zwei der folgenden Substituenten tragen kann:
Methyl, Ethyl, n-Propyl, Isopropyl, n-Butyl, Isobutyl, sec.-Butyl, tert.-Butyl, Phenyl, Phenylsulfonyl, Nitro, Cyano und eine der Gruppen
-CO-O-R³,
-CO-NH-R³,
-CH₂-O-R³,
-O-R³ und
-CH=NO-R³
- R: gleiche oder verschiedene der folgenden Reste: Methyl, Ethyl, n-Propyl, Isopropyl, n-Butyl, Isobutyl, sec.-Butyl, tert.-Butyl, C₁-C₄-Alkoxy, Trifluormethyl, Trichlormethyl, Trifluormethoxy, Trichlormethoxy, Fluor, Chlor, Brom, Jod, Nitro, Cyano und eine der Gruppen
-CO-O-R³ und
-CO-NH-R³
sowie einen anellierten Benzolring, der seinerseits bis zu drei der für R vorgenannten Reste tragen kann;
- n: 0 bis 3.

Unter Alkyl in einer Halogenalkyl- oder Halogenalkoxygruppe sind je nach der genannten Zahl der Kohlenstoffatome Methyl, Ethyl, Propyl, Isopropyl, Butyl, Isobutyl, sec.-Butyl und tert.-Butyl zu verstehen.

Halogen oder Halogen in einer Halogenalkyl- oder Halogenalkoxygruppe bedeutet Fluor, Chlor, Brom oder Jod.

Besonders bevorzugt sind Verbindungen, in denen
- R¹: Wasserstoff,
Methyl, Ethyl, n-Propyl, Isopropyl, n-Butyl, Isobutyl, sec.-Butyl, tert.-Butyl,
eine der Gruppen -(CH₂)ₘ-R^{4,} wobei m für einen Wert von 0 bis 2 steht und R⁴ die Cyclohexylgruppe oder die Phenylgruppe bedeutet, welche Fluor, Chlor, Brom, Nitro, Trifluormethyl, Trifluormethoxy, Methyl, Ethyl, n-Propyl, Isopropyl, n-Butyl, Isobutyl, sec.-Butyl oder tert.-Butyl als Substituenten tragen kann, oder die Gruppe -CHR⁵-(CH₂)ₘ-CO-O-R⁶ oder -CHR⁵-(CH₂)ₘ-CO-NH-R⁶, wobei R⁵ Wasserstoff, Methyl, Ethyl, n-Propyl, Isopropyl, n-Butyl, Isobutyl, sec.-Butyl oder tert.-Butyl und R⁶ den Rest R⁵ oder die Gruppe -(CH₂)ₘ-R⁴ bedeuten, insbesondere Wasserstoff und Cyclohexyl,
- R²: eine Pyrrol-1-ylgruppe, die bis zu zwei der Substituenten C₁-C₄-Alkyl, C₁-C₄-Alkoxy, Phenyl, Formyl, Acetyl, Propionyl und eine der Gruppen -CO-O-R⁶ und -CO-NH-R⁶ tragen kann,
insbesondere Pyrrol-1-yl, 2,5-Dimethylpyrrol-1-yl, 2-Methoxypyrrol-1-yl, 2,5-Diphenylpyrrol-1-yl, 2-Formylpyrrol-1-yl, 2-Propionylpyrrolyl-1-yl, 2-Carboxypyrrol-1-yl und 2-Methoxycarbonylpyrrol-1-yl,
- R: Methyl, Ethyl, n-Propyl, Isopropyl, n-Butyl, Isobutyl, sec.-Butyl, tert.-Butyl, Fluor, Chlor, Brom, Nitro, Trifluormethyl, Trifluormethoxy oder einen anellierten Benzolring, der seinerseits einen für R vorgenannten Rest tragen kann, insbesondere Chlor, Nitro, Trifluormethyl und einen anellierten Benzolring, und
- n: 0 oder 1 bedeuten und die Maßgabe gilt, daß n im Falle des anellierten Benzolrings 2 bedeutet.

Prinzipiell kann man die erfindungsgemäßen Verbindungen I auf zwei Wegen herstellen, indem man ein Aminophenylen-1,2-diamin II 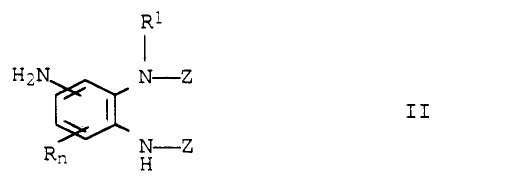 wobei Z für eine als Schutzgruppe fungierende Acylgruppe steht, mit Bernsteindialdehyd oder seinem acyclischen oder cyclischen Acetal oder Hemiacetal (Verbindung III) zu IV 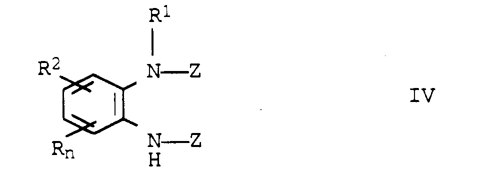 umsetzt, und nach Abspaltung der Schutzgruppe Z den Ringschluß zu I in an sich bekannter Weise mit Oxalsäure oder einem ihrer funktionellen Derivate vornimmt,
oder indem man II in schutzgruppenfreier Form zunächst mit Oxalsäure oder einem ihrer funktionellen Derivate in die entsprechenden Aminochinoxalin-2,3-(1H,4H)-dione überführt und diese danach mit III zu I umsetzt.

Im einzelnen sind die erfindungsgemäßen Verbindungen I folgendermaßen erhältlich:
Man geht von einem Aminophenylen-1,2-diamin II aus, in welchem Z für eine als Schutzgruppe fungierende Acylgruppe, vorzugsweise Acetyl oder Trifluoracetyl, steht, und setzt dieses in bekannter Weise, z.B. A.R. Katritzky und C.W. Rees, "Comprehensive Heterocyclic Chemistry", Bd. 4, Teil 306, S. 313 ff, in Gegenwart von katalytischen Mengen einer Säure, wie Essigsäure, mit einer Verbindung III, vorzugsweise dem cyclischen Acetal, unter Wasserabspaltung zu IV um.

Die Säure kann auch als Lösungsmittel dienen, wenn sie in größeren Mengen verwendet wird.

Im allgemeinen ist es jedoch üblich, die Umsetzung in einem Lösungsmittel wie Toluol oder in einem Lösungsmittelgemisch wie Toluol/Dimethylformamid unter Säurekatalyse bei einer Reaktionstemperatur von 50 bis 150°C, vorzugsweise 100 bis 150°C, vorzunehmen.

Nach Abspaltung der Schutzgruppe erfolgt in an sich bekannter Weise der Ringschluß von IV mit Oxalsäure oder einem ihrer funktionellen Derivate, vorzugsweise Oxalsäurediester wie Oxalsäuredimethylester und Oxalsäurediethylester, zu I. Der Ringschluß ist hinsichtlich der Reaktionstemperatur und Reaktionszeit allgemein bekannt.

Aminophenylen-1,2-diamine II sind z.B. aus US-A 3 992 378 bekannt oder hiernach erhältlich. Ferner lassen sie sich aus kommerziell erhältlichen o-Phenylendiaminen nach Einführung von Schutzgruppen auf übliche Weise durch Nitrierung und anschließende Reduktion der Nitrogruppe herstellen.

Eine weitere Möglichkeit zur Herstellung der Ausgangsverbindung II besteht aus der allgemein bekannten Umsetzung von 2-Nitro-chlorbenzolen mit Aminen wie Ethylamin, Cyclohexylamin, 1-Phenylethylamin und α-Aminoessigsäureester zu den entsprechenden 2-Nitroanilinen, der anschließenden Reduktion der Nitrogruppe, Einführung von Schutzgruppen sowie Nitrierung und Reduktion der weiteren Nitrogruppe.

Die Umsetzung der 2-Nitro-chlorbenzole mit Aminen ist allgemein bekannt und wird üblicherweise in polaren Lösungsmitteln wie Dimethylformamid, Dimethylsulfoxid und Ethanol in Gegenwart von basischen Salzen wie Kaliumcarbonat bei einer Reaktionstemperatur von 25 bis 180°C, vorzugsweise von 25 bis 140°C durchgeführt.

Die Einführung und Abspaltung der Schutzgruppen erfolgt jeweils nach allgemein üblichen Methoden, welche beispielsweise in T.W. Greene, "Protective Groups in Organic Synthesis", Wiley and Sons, New York 1982, Kap. 7, S. 249ff, zusammenfassend beschrieben sind.

Die organischen Syntheseschritte Nitrierung und Reduktion können jeweils nach den üblichen, in Houben-Weyl, "Methoden der organischen Chemie", Bd. 10/1 bzw. Bd. 11/1 beschriebenen Methoden durchgeführt werden. Für die Nitrierung eignen sich Gemische aus Essigsäure-Salpetersäure und Schwefelsäure-Natriumnitrat.

Die Reduktion kann nach der chemischen und nach der katalytischen Methode erfolgen. Bei der katalytischen Variante wird z.B. mit Wasserstoff an den Katalysatoren Palladium/Aktivkohle und Platin/Aktivkohle in Gegenwart eines Lösungsmittels reduziert; bei der chemischen Variante kann die Reduktion mit Natriumborhydrid/Kupfersulfat in Dimethylformamid und in Alkoholen wie Ethanol durchgeführt werden. Ferner ist es auch üblich, die Nitrogruppen mit Redoxsystemen wie Eisen/Salzsäure und Zink/Salzsäure zu reduzieren.

Eine weitere Möglichkeit zur Herstellung der erfindungsgemäßen Verbindungen I besteht darin, daß man die bereits oben beschriebenen Syntheseschritte - Umsetzung mit III und Ringschluß - in umgekehrter Reihenfolge durchführt.

Man führt zunächst den Ringschluß durch, indem man ein in schutzgruppenfreier Form vorliegendes Aminophenylen-1,2-diamin II mit Oxalsäure oder einem ihrer funktionellen Derivate wie Oxalsäuredichlorid und Oxalsäurediester nach den üblichen Methoden zu den entsprechenden Aminochinoxalin-2,3(1H,4H)-dionen umsetzt. Vor der sich nun anschließenden Umsetzung mit III zu I empfiehlt es sich, wenn weitere Substituenten R, beispielsweise Nitro, in das Aminochinoxalindion eingeführt werden, die Aminogruppe durch eine Acylgruppe zu schützen, um dann - nach Abspaltung der Schutzgruppe in Gegenwart von Salzsäure - entweder die freien Aminochinoxalin-2,3(1H,4H)-dione oder ihre Hydrochloride mit III umzusetzen.

Die erfindungsgemäßen Verbindungen I' werden hergestellt, indem man von einem nach dem oben beschriebenen erfindungsgemäßen Verfahren hergestellten Chinoxalin-2,3(1H,4H)-dion I'' 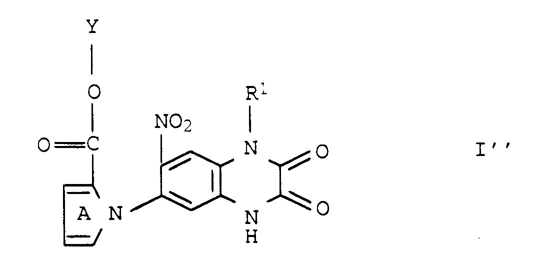 ausgeht und die Nitrogruppe von I'' reduziert. Die Reduktion erfolgt in an sich bekannter Weise unter sauren Bedingungen, beispielsweise mit Wasserstoff an Palladium/Aktivkohle oder Eisessig/Eisen, unter Abspaltung von YOH, wobei Y Wasserstoff oder eine C₁-C₄-Alkylgruppe, vorzugsweise Methyl, bedeutet.

Die pharmakologische Wirksamkeit der erfindungsgemäßen Verbindungen I und I' wurde an isoliertem Membranmaterial von Rattengroßhirnen untersucht. Hierzu wurde das Membranmaterial in Gegenwart der erfindungsgemäßen Verbindungen mit den radioaktiv markierten Substanzen ³H-2-Amino-3-hydroxy-5-methyl-4-isoxazolpropionsäure (³H-AMPA) und ³H-5,7-Dichlorkynurensäure behandelt, wobei sich diese an spezifische Rezeptoren (AMPA- bzw. NMDA-Rezeptor (N-Methyl-D-aspartat)) binden. Anschließend wurde durch Scintillationszählung die Radioaktivität der behandelten Membrane gemessen. Über die gebundene Radioaktivität ließen sich die Mengen an gebundener ³H-AMPA und ³H-5,7-Dichlorkynurensäure bzw. jeweils die verdrängten Mengen dieser radioaktiv markierten Substanzen bestimmen. Die sich hieraus ergebende Dissoziationskonstante K_{I} (I=Inhibitor), welche ein Maß für die Verdrängungswirkung des erfindungsgemäßen Wirkstoffes ist, wurde durch iterative nichtlineare Regressionsanalyse mit dem Statistical Analysis System (SAS) an einem IBM-Rechner, ähnlich dem Programm "Ligand" von P.J. Munson und D. Rodbard (Analytical Biochem. 107, 220 (1980), Ligand: Versatile Computerized Approach for Charakterization of Ligand Binding Systems) ermittelt.

Folgende In-Vitro-Untersuchungen wurden durchgeführt:

### 1. Bindung von ³H-2-Amino-3-hydroxy-5-methyl-4-isoxazolpropionsäure (³H-AMPA)

Für die Präparation des Membranmaterials wurden frisch entnommene Rattengroßhirne zusammen mit dem ca. 15fachen Volumen einer Pufferlösung A aus 30 mM α,α,α-Tris-(hydroxymethyl)-methylamin-Hydrochlorid (TRIS-HCl) und 0,5 mM Ethylendiamintetraessigsäure (EDTA) - pH 7,4 - mittels eines Ultra-Turrax-Rührers homogenisiert. Die Suspension wurde 20 Minuten bei 48 000 · g zentrifugiert. Nach Abtrennung der überstehenden Flüssigkeit wurde das im Bodensatz enthaltene proteinhaltige Membranmaterial dreimal durch Suspendieren in der Pufferlösung A und anschließendes, jeweils 20-minütiges Zentrifugieren bei 48 000 · g gewaschen. Danach wurde das Membranmaterial in einem 15fachen Volumen der Pufferlösung A suspendiert und 30 Minuten bei 37°C inkubiert. Anschließend wurde das Proteinmaterial zweimal durch Zentrifugieren und Suspendieren gewaschen und bis zur Verwendung bei -70°C eingefroren.

Für den Bindungstest wurde das bei 37°C aufgetaute Proteinmaterial zweimal durch Zentrifugieren bei 48 000 · g (20 Minuten) und anschließendes Suspendieren in einer Pufferlösung B aus 50 mM TRIS-HCl, 0,1 M Kaliumthiocyanat und 2,5 mM Calciumchlorid - pH 7,1 - gewaschen. Anschließend wurden 0,25 mg Membranmaterial, 0,1 µCi ³H-AMPA (60 Ci/mmol) sowie Verbindung I bzw. I' in 1 ml Pufferlösung B gelöst und 60 Minuten auf Eis inkubiert. Die inkubierte Lösung wurde über einen CF/B-Filter (Firma Whatman), der zuvor mindestens 2 Stunden mit einer 0,5 %igen wäßrigen Lösung von Polyethylenimin behandelt worden war, filtriert. Anschließend wurde der Membranrückstand mit 5 ml kalter Pufferlösung B gewaschen, um gebundene und freie ³H-AMPA voneinander zu trennen. Nach Messung der Radioaktivität der gebundenen ³H-AMPA im Membranmaterial durch Scintillationszählung wurde durch Auswertung der Verdrängungskurven mittels Regressionsanalyse der K_{I}-Wert bestimmt. Für 7-Chlor-6-(1-pyrrolyl)-chinoxalin-2,3(1H,4H)-dion (Beispiel 1) wurde ermittelt: K_{I} < 10 µM.

### 2. Bindung von ³H-5,7-Dichlorkynurensäure

Für die Präparation des Membranmaterials wurden frisch entnommene Rattengroßhirne zusammen mit dem ca. 10fachen Volumen einer Pufferlösung A' aus 50 mM TRIS-HCl und 10 mM EDTA - pH 7,4 - homogenisiert. Die Suspension wurde 20 Minuten bei 48 000 · g zentrifugiert. Nach Abtrennung der überstehenden Flüssigkeit wurde das im Bodensatz enthaltene Membranmaterial zweimal durch Suspendieren in der Pufferlösung A' und anschließendes jeweils 20-minütiges Zentrifugieren und Suspendieren gewaschen. Nach erneutem Suspendieren der Membrane in der Pufferlösung A' und Einfrieren in flüssigem Stickstoff wurde die Suspension wieder bei 37°C aufgetaut und nach einem weiteren Waschvorgang 15 Minuten bei 37°C inkubiert. Anschließend wurde das Proteinmaterial viermal durch Zentrifugieren und Suspendieren gewaschen und bis zur Verwendung bei -70°C eingefroren.

Für den Bindungstest wurde das bei 37°C aufgetaute Proteinmaterial zweimal durch Zentrifugieren bei 48 000 · g (20 Minuten) und anschließendes Suspendieren in einer Pufferlösung B' aus 50 mM TRIS-HCl - pH 7,4 - gewaschen. Anschließend wurden 0,15 mg Membranmaterial, 0,3 µCi ³H-5,7-Dichlorkynurensäure (16 Ci/mmol) sowie Verbindung I bzw. I' in 1 ml Pufferlösung B' gelöst und 30 Minuten auf Eis inkubiert. Die inkubierte Lösung wurde 2 Minuten bei 150 000 · g zentrifugiert. Nach Abtrennung der überstehenden Flüssigkeit wurden die Bodensätze zweimal mit je 1,5 ml kalter Pufferlösung B' suspendiert. Nach Messung der Radioaktivität der an die Membranen gebundenen ³H-5,7-Dichlorkynurensäure im Bodensatz ergab sich der K_{I}-Wert durch Auswertung der Verdrängungskurven mittels der Regressionsanalyse.
Für 6-(1-Pyrrolyl)-7-trifluormethylchinoxalin-2,3(1H,4H)-dion (Beispiel 2) wurde ermittelt: K_{I} < 10 µM.

Die erfindungsgemäßen Verbindungen I und I' sind als Arzneimittel für die Human- und Veterinärmedizin geeignet und können zur Herstellung von Arzneimitteln zur Behandlung neurodegenerativer Erkrankungen und neurotoxischer Störungen des zentralen Nervensystems sowie zur Herstellung von Spasmolytika, Antiepileptika, Anxiolytika und Antidepressiva verwendet werden.

Die erfindungsgemäßen Arzneimittelzubereitungen enthalten neben den üblichen Arzneimittelhilfsstoffen eine therapeutisch wirksame Menge der Verbindungen I und I'. Für die lokale äußere Anwendung, z.B. in Puder und Salben, können die Wirkstoffe in den üblichen Konzentrationen enthalten sein.

In der Regel sind die Wirkstoffe in einer Menge von 0,0001 bis 1 Gew.-%, vorzugsweise 0,001 bis 0,1 Gew.-%, enthalten.

Bei der inneren Anwendung werden die Präparationen in Einzeldosen verabreicht. In einer Einzeldosis werden pro kg Körpergewicht 0,1 bis 50 mg, vorzugsweise 0,1 bis 10 mg Wirkstoff gegeben. Die Zubereitungen können täglich in einer oder mehreren Dosierungen je nach Art und Schwere der Erkrankung verabreicht werden.

Entsprechend der gewünschten Applikationsart enthalten die erfindungsgemäßen Arzneimittelzubereitungen neben dem Wirkstoff die üblichen Trägerstoffe und Verdünnungsmittel. Für die lokale äußere Anwendung können pharmazeutisch-technische Hilfsstoffe, wie Ethanol, Isopropanol, oxethyliertes Ricinusöl, oxethyliertes hydriertes Ricinusöl, Polyacrylsäure, Polyethylenglykol, Polyethylenglykolstearat, ethoxylierte Fettalkohole, Paraffinöl, Vaseline und Wollfett verwendet werden. Für die innere Anwendung eignen sich z.B. Milchzucker, Propylenglykol, Ethanol, Stärke, Talk und Polyvinylpyrrolidon.
Ferner können Antioxidationsmittel wie Tocopherol und butyliertes Hydroxyanisol sowie butyliertes Hydroxytoluol, geschmacksverbessernde Zusatzstoffe, Stabilisierungs-, Emulgier- und Gleitmittel enthalten sein.

Die neben dem Wirkstoff in der Zubereitung enthaltenen Stoffe sowie die bei der Herstellung der pharmazeutischen Zubereitung verwendeten Stoffe sind toxikologisch unbedenklich und mit dem jeweiligen Wirkstoff verträglich.

Die Herstellung der Arzneimittelzubereitungen erfolgt in üblicher Weise, z.B. durch Vermischen des Wirkstoffes mit den anderen üblichen Trägerstoffen und Verdünnungsmittel.

Die Arzneimittelzubereitungen können in verschiedenen Applikationsweisen verabreicht werden, wie
peroral, parenteral, subkutan, intraperitoneal und topisch. So sind Zubereitungsformen wie
Tabletten, Emulsionen, Infusions- und Injektionslösungen, Pasten, Salben, Gele, Cremes, Lotionen, Puder und Sprays möglich.

### Beispiel 1

### 7-Chlor-6-(1-pyrrolyl)-chinoxalin- 2,3(1H,4H)-dion

a) Herstellung von 2-Acetamido-4-chlor-acetanilid
   28,4 g (0,2 mol) 4-Chlor-1,2-phenylendiamin wurden in 130 ml 3-molarer Salzsäure gelöst und mit 300 ml Wasser verdünnt. Nach dem Zutropfen von 51 g (0,5 mol) Essigsäureanhydrid wurde die Reaktionsmischung 30 Minuten bei Raumtemperatur gerührt, dann mit 41 g (0,6 mol) Natriumacetat versetzt und mit Essigester extrahiert. Nach der Aufarbeitung der organischen Phase wurde das Produkt in einer Ausbeute von 21 % erhalten.
   Schmp. 216°C
b) Herstellung von 2-Acetamido-4-chlor-5-nitro-acetanilid
   Eine Lösung von 5 g (22 mmol) 2-Acetamido-4-chlor-acetanilid in 50 ml konzentrierter Schwefelsäure wurde auf 0°C abgekühlt, portionsweise mit 1,9 g (8,5 mmol) Natriumnitrat versetzt und 15 Minuten bei Raumtemperatur gerührt. Anschließend wurde die Reaktionsmischung in Eiswasser gegossen, das Rohprodukt wurde abgesaugt und mit Wasser gewaschen. Das Produkt wurde in einer Ausbeute von 63 % erhalten.
   Schmp. 232°C
c) Herstellung von 2-Acetamido-4-chlor-5-amino-acetanilid
   Eine Lösung von 4 g (14,7 mmol) 2-Acetamido-4-chlor-5-nitro-acetanilid in 200 ml Ethanol wurde mit einer Lösung von 0,4 g Kupfer-II-sulfat x 5 H₂O in 2 ml Wasser und anschließend portionsweise mit 1,1 g (29,4 mmol) Natriumborhydrid versetzt. Die Reaktionsmischung wurde 3 Stunden unter Rückfluß erhitzt und anschließend filtriert. Das Filtrat wurde eingeengt und das Rohprodukt mit Wasser und Methylenchlorid gewaschen. Nach der Aufarbeitung der organischen Phase wurde das Produkt in einer Ausbeute von 94 % erhalten.
   Schmp. 237°C
d) Herstellung von N-(2-Chlor-4,5-diacetamidophenyl)-pyrrol
   3 g (12,4 mmol) 2-Acetamido-4-chlor-5-amino-acetanilid und 1,6 g (12,4 mmol) 2,5-Dimethoxytetrahydrofuran wurden in 150 ml Essigsäure 30 Minuten unter Rückfluß erhitzt. Nach dem Einengen der Reaktionsmischung wurde der Rückstand mit Wasser und Essigester gewaschen, und die organische Phase wurde aufgearbeitet.
   Ausbeute: 81 %
   Schmp. 206°C
e) Herstellung von N-(2-Chlor-4,5-diaminophenyl)-pyrrol
   Eine Lösung von 1,4 g (4,8 mmol) N-(2-Chlor-4,5-diacetamidophenyl)-pyrrol und 0,4 g (9,8 mmol) Kaliumhydroxid in 25 ml Ethylenglykol wurde 30 Minuten bei 60°C gerührt. Danach wurde die Reaktionsmischung auf Wasser gegossen, mit Essigester extrahiert und aufgearbeitet.
   Ausbeute: 81 %
   Schmp. 206°C
   ¹H-NMR (D₆-DMSO):
      - δ (ppm):: 2,6 (4H); 6,2 (2H); 7,5 (1H); 7,7 (1H)
f) Herstellung von 7-Chlor-6-(1-pyrrolyl)-chinoxalin-2,3-(1H,4H)-dion
   0,6 g (2,9 mmol) N-(2-Chlor-4,5-diaminophenyl)-pyrrol und 32 g (221 mmol) Oxalsäurediethylester wurden 3 Stunden unter Rückfluß erhitzt. Nach Abkühlen der Reaktionsmischung wurde das Rohprodukt abfiltriert und mit Petrolether gewaschen.
   Ausbeute: 40 %
   Schmp. > 320°C
   ¹H-NMR (D₆-DMSO):
      - δ (ppm):: 6,25 (2H); 6,95 (2H); 7,10 (1H); 7,25 (1H); 12,0 (2H)

### Beispiel 2

### 6-(1-Pyrrolyl)-7-trifluormethylchinoxalin-2,3(1H,4H)-dion

Entsprechend der Vorschrift von Beispiel 1f wurde aus 0,8 g (3,3 mmol) N-(2-Trifluormethyl-4,5-diaminophenyl)-pyrrol und 32 g (221 mmol) Oxalsäurediethylester 6-(1-Pyrrolyl)-7-trifluormethylchinoxalin-2,3(1H,4H)-dion hergestellt.
Ausbeute: 43 %
Schmp. > 300°C
¹H-NMR (D₆-DMSO):
   - δ(ppm):: 6,2 (2H); 6,9 (2H); 7,0 (1H); 7,5 (1H); 12,5 (2H)

### Beispiel 3

### 1-Cyclohexyl-6-trifluormethyl-7-(1-pyrrolyl)-chinoxalin-2,3-(1H,4H)-dion

a) Herstellung von N-Cyclohexyl-2-nitro-4-trifluormethylanilin
   Eine Lösung von 50 g (0,22 mol) 2-Nitro-4-trifluormethylchlorbenzol, 22 g (0,22 mol) Cyclohexylamin und 61 g (0,44 mol) Kaliumcarbonat in 600 ml Ethanol wurden 4 Stunden unter Rückfluß erhitzt. Nach dem Einengen der Reaktionsmischung wurde das Rohprodukt mit Wasser und Ether gewaschen. Die organische Phase wurde aufgearbeitet, und das Produkt wurde durch Säulenchromatographie an Kieselgel gereinigt (Laufmittel: Heptan/Essigester = 5/1).
   Ausbeute: 51 %
   Schmp. 81°C
b) Herstellung von N-Cyclohexyl-2-amino-4-trifluormethylanilin
   Mit Wasserstoff wurden in Gegenwart von 1,5 g Palladium/Aktivkohle (10 Gew.-% Pd) 32 g (0,11 mol) N-Cyclohexyl-2-nitro-4-trifluormethylanilin - gelöst in 250 ml Methanol - bei Raumtemperatur bei einem Druck von ca. 1 bar Wasserstoff hydriert. Die Reaktionsmischung wurde filtriert. Die übliche Aufarbeitung des Filtrats lieferte die obengenannte Verbindung in 100 %iger Ausbeute.
   Schmp. 70°C.
c) Herstellung von 1-Cyclohexyl-6-trifluormethylchinoxalin-2,3(1H,4H)-dion
   28 g (0,11 mol) N-Cyclohexyl-2-amino-4-trifluormethylanilin und 322 g (2,2 mol) Oxalsäurediethylester wurden 2 Stunden unter Rückfluß erhitzt. Danach wurde die Reaktionsmischung eingeengt, und das Rohprodukt wurde aus Ethanol umkristallisiert.
   Ausbeute: 59 %
   Schmp. 183°C
d) Herstellung von 1-Cyclohexyl-6-trifluormethyl-7-nitrochinoxalin-2,3(1H,4H)-dion
   Eine Lösung von 20 g (64 mmol) 1-Cyclohexyl-6-trifluormethyl-chinoxalin-2,3(1H,4H)-dion in 400 ml konzentrierter Schwefelsäure wurde auf 0°C abgekühlt, innerhalb einer Stunde portionsweise mit 5,4 g (64 mmol) Natriumnitrat versetzt und 3 Stunden bei Raumtemperatur gerührt. Anschließend wurde die Reaktionsmischung in Eiswasser gegossen, das Rohprodukt wurde abgesaugt und aus Methanol umkristallisiert.
   Ausbeute: 78 %
   Zersetzungspunkt 272°C
e) Herstellung von 1-Cyclohexyl-6-trifluormethyl-7-aminochinoxalin-2,3(1H,4H)-dion
   Mit Wasserstoff wurden in Gegenwart von 1 g Palladium/Aktivkohle (10 Gew.-% Pd) 8 g (22,4 mmol) 1-Cyclohexyl-6-trifluormethyl-7-nitrochinoxalin-2,3(1H,4H)-dion - gelöst in 200 ml Tetrahydrofuran - bei Raumtemperatur bei einem Druck von ca. 1 bar Wasserstoff hydriert. Die Reaktionsmischung wurde filtriert. Die übliche Aufarbeitung des Filtrats lieferte die obengenannte Verbindung in 100 %iger Ausbeute.
   Zersetzungspunkt > 260°C
   ¹H-NMR (D₆-DMSO):
      - δ(ppm):: 1,4-1,6 (3H); 1,6-1,8 (3H); 1,8-2,0 (2H); 2,3-2,6 (2H); 4,2-4,5 (1H); 5,0-5,4 (2H); 7,1 (1H); 7,2 (1H); 11,8 (1H)
f) Herstellung von 1-Cyclohexyl-6-trifluormethyl-7-(1-pyrrolyl)-chinoxalin-2,3(1H,4H)-dion
   Eine Lösung von 2,4 g (7,3 mmol) 1-Cyclohexyl-6-trifluormethyl-7-aminochinoxalin-2,3(1H,4H)-dion und 1 g (7,3 mmol) 2,5-Dimethoxytetrahydrofuran in 100 ml Eisessig wurden 30 Minuten unter Rückfluß erhitzt. Anschließend wurde die Reaktionsmischung in Eiswasser gegossen. Das entstandene Produkt wurde abgesaugt.
   Ausbeute: 69 %
   Schmp. > 320°C
   ¹H-NMR (D₆-DMSO):
      - δ(ppm):: 1,15 (1H); 1,45 (2H); 1,6 (1H); 1,7 (2H); 1,75 (2H); 2,4 (2H); 4,4 (1H); 6,2 (2H); 6,9 (2H); 7,55 (1H); 7,6 (1H)

### Beispiel 4

### 1-Cyclohexyl-6-trifluormethyl-7-(2-methoxycarbonyl-1-pyrrolyl)-chinoxalin-2,3(1H,4H)-dion

Anstelle von 2,5-Dimethoxytetrahydrofuran wurden 1,2 g (6,1 mmol) 2-Methoxycarbonyl-2,5-dimethoxytetrahydrofuran mit 2 g (6,1 mmol) 1-Cyclohexyl-6-trifluormethyl-7-aminochinoxalin-2,3(1H,4H)-dion in 100 ml Essigsäure gelöst und 30 Minuten unter Rückfluß erhitzt. Die weitere Aufarbeitung erfolgte nach Beispiel 3, f.
Ausbeute: 61 %
Schmp. 200°C
¹H-NMR (D₆-DMSO):
   - δ(ppm):: 1,2-1,4 (m, 2H); 1,7-2,0 (m, 6H); 2,4-2,6 (m,2H); 3,7 (s,3H); 4,2-4,3 (m,1H); 6,4 (dd,1H); 6,95 (m,1H); 7,15 (dd,1H); 7,3 (1H); 7,6 (1H)

### Beispiel 5

### 6-(2-Methoxycarbonyl-1-pyrrolyl)-7-nitrochinoxalin-2,3-(1H,4H)-dion

a) Herstellung von 6-Trifluoracetamidochinoxalin-2,3(1H,4H)-dion
   Eine Lösung von 28 g (0,16 mol) 6-Aminochinoxalin-2,3(1H,4H)-dion in 200 ml Trifluoressigsäure wurde mit 35,7 g (0,17 mol) Trifluoressigsäureanhydrid eine Stunde unter Rückfluß erhitzt. Nach Abkühlung der Reaktionsmischung wurde das angefallene Rohprodukt abfiltriert und nach den üblichen Methoden aufgearbeitet.
   Ausbeute: 83 %
   Schmp. > 330°C
   ¹H-NMR (D₆-DMSO):
      - δ(ppm):: 7,1 (1H); 7,3 (1H); 7,6 (1H); 11,3 (1H); 12,1 (1H)
b) Herstellung von 6-Trifluoracetamido-7-nitrochinoxalin-2,3(1H,4H)-dion
   Eine Lösung von 39 g (0,14 mol) 6-Trifluoracetamidochinoxalin-2,3(1H,4H)-dion in 500 ml konzentrierter Schwefelsäure wurde auf 0°C abgekühlt, portionsweise mit 12,1 g (0,14 mol) Natriumnitrat versetzt und 30 Minuten bei Raumtemperatur gerührt. Anschließend wurde die Reaktionsmischung in Eiswasser gegossen, das Rohprodukt wurde abfiltriert und nach den üblichen Methoden aufgearbeitet.
   Ausbeute: 94 %
   Schmp. > 320°C
   ¹H-NMR (D₆-DMSO):
      - δ (ppm):: 7,3 (1H); 7,8 (1H); 11,6 (1H); 12,2 (1H); 12,4 (1H)
c) Herstellung von 6-Amino-7-nitrochinoxalin-2,3(1H,4H)-dion
   Eine Lösung von 41 g (0,13 mol) 6-Trifluoracetamido-7-nitrochinoxalin-2,3(1H,4H)-dion in 300 ml Ethanol und 700 ml 3-molare Salzsäure wurden 3 Stunden unter Rückfluß erhitzt. Nach Abkühlung der Reaktionsmischung wurde das angefallene Rohprodukt abfiltriert und nach den üblichen Methoden aufgearbeitet.
   Ausbeute: 84 %
   Schmp. > 330°C
   ¹H-NMR (D₆-DMSO):
      - δ (ppm):: 6,6 (1H); 7,2-7,6 (3H); 7,8 (1H); 11,7 (1H); 12,1 (1H)
d) Herstellung von 6-(2-Methoxycarbonyl-1-pyrrolyl)-7-nitrochinoxalin-2,3(1H,4H)- dion
   7 g (27,1 mmol) 6-Amino-7-nitrochinoxalin-2,3(1H,4H)-dion, 5,1 g (27,1 mmol) 2-Methoxycarbonyl-2,5-dimethoxytetrahydrofuran und 4,5 g (54,2 mmol) Natriumacetat wurden eine Stunde in 500 ml Eisessig unter Rückfluß erhitzt. Nach dem Einengen der Reaktionsmischung wurde das Rohprodukt durch Säulenchromatographie an Kieselgel (Laufmittel: Toluol/Aceton/Eisessig = 10/10/1) gereinigt.
   Ausbeute: 59 %
   Schmp. > 310°C
   ¹H-NMR (D₆-DMSO):
      - δ(ppm):: 3,6 (s,3H); 6,4 (dd,1H); 7,05 (m,2H); 7,3 (dd,1H); 7,95 (s,1H); 12,4 (m,2H)

### Beispiel 6

### 6-(2,5-Dimethyl-1-pyrrolyl)-7-nitrochinoxalin-2,3(1H,4H)-dion

Entsprechend der Vorschrift von Beispiel 5d wurden 13,5 mmol 6-Amino-7-nitrochinoxalin-2,3(1H,4H)-dion mit 13,5 mmol 2,5-Dimethyl-2,5-dimethoxytetrahydrofuran umgesetzt.
Ausbeute: 42 %
Schmp. > 320°C
¹H-NMR (D₆-DMSO):
   - δ(ppm):: 1,9 (6H); 5,8 (2H); 7,0 (1H); 7,9 (1H); 12 (2H)

### Beispiel 7

### 6-(1-Pyrrolyl)-7-nitrochinoxalin- 2,3(1H,4H)-dion

Entsprechend der Vorschrift von Beispiel 5d wurden 23,2 mmol 6-Amino-7-nitrochinoxalin-2,3(1H,4H)-dion mit 23,2 mmol 2,5-Dimethoxytetrahydrofuran umgesetzt.
Ausbeute: 66 %
Schmp. > 310°C
¹H-NMR (D₆-DMSO):
   - δ (ppm):: 6,2 (2H); 6,8 (2H); 7,1 (1H); 7,8 (1H); 12,5 (2H)

### Beispiel 8

### 6-(1-Pyrrolyl)-7-trifluormethylchinoxalin- 2,3(1H,4H)-dion

Entsprechend der Vorschrift von Beispiel 5d wurden 12,2 mmol 6-Amino-7-trifluormethylchinoxalin-2,3(1H,4H)-dion mit 12,2 mmol 2,5-Dimethoxytetrahydrofuran umgesetzt.
Ausbeute: 49 %
Schmp. > 300°C
¹H-NMR (D₆-DMSO):
   - δ (ppm):: 6,2 (2H); 6,9 (2H); 7,0 (1H); 7,5 (1H); 12,5 (2H)

### Beispiel 9

### 6-(2-Methoxycarbonyl-1-pyrrolyl)-7-trifluormethylchinoxalin-2,3(1H,4H)-dion

Entsprechend der Vorschrift von Beispiel 5d wurden 51 mmol 6-Amino-7-trifluormethylchinoxalin-2,3(1H,4H)-dion mit 51 mmol 2-Methoxycarbonyl-2,5-dimethoxytetrahydrofuran umgesetzt.
Ausbeute: 86 %
Schmp. > 310°C
¹H-NMR (D₆-DMSO):
   - δ(ppm):: 3,6 (3H); 6,3 (1H); 7,0 (2H); 7,1 (1H); 7,5 (1H); 12,5 (2H)

### Beispiel 10

### 6-(2-Carboxy-1-pyrrolyl)-7-trifluormethylchinoxalin-2,3(1H,4H)-dion

13 g (36,8 mmol) 6-(2-Methoxycarbonyl-1-pyrrolyl)-7-trifluormethylchinoxalin- 2,3(1H,4H)-dion wurden mit 75 ml 2-molarer Natronlauge versetzt und 16 Stunden bei Raumtemperatur gerührt. Nach Neutralisation mit 2-molarer Salzsäure wurde das Gemisch mit Methylenchlorid extrahiert und auf übliche Weise aufgearbeitet.
Ausbeute: 79 %
Schmp. > 320°C
¹H-NMR (D₆-DMSO):
   - δ(ppm):: 6,3 (1H); 6,95 (1H); 7,0 (1H); 7,1 (1H); 7,5 (1H); 12,0 (2H)

### Beispiel 11

### 6-(2,5-Dimethyl-1-pyrrolyl)-7-trifluormethyl-chinoxalin-2,3(1H,4H)-dion

Entsprechend der Vorschrift von Beispiel 5d wurden 12,2 mmol 6-Amino-7-trifluormethylchinoxalin-2,3-(1H,4H)-dion mit 12,2 mmol 2,5-Dimethyl-2,5-dimethoxytetrahydrofuran umgesetzt.
Ausbeute: 54 %
Schmp. > 350°C
¹H-NMR (D₆-DMSO):
   - δ(ppm):: 1,9 (6H); 5,8 (2H); 6,9 (1H); 7,6 (1H); 12,5 (2H)

### Beispiel 12

### 6-(2,5-Dimethyl-1-pyrrolyl)-7-chlorchinoxalin-2,3(1H,4H)-dion

Entsprechend der Vorschrift von Beispiel 5d wurden 14,2 mmol 6-Ammonio-7-chlorchinoxalin-2,3-(1H,4H)-dionchlorid mit 14,2 mmol 2,5-Dimethyl-2,5-dimethoxytetrahydrofuran umgesetzt.
Ausbeute: 57 %
Schmp. > 300°C
¹H-NMR (D₆-DMSO):
   - δ(ppm):: 1,9 (6H); 5,8 (2H); 7,0 (1H); 7,3 (1H); 12,0 (2H)

### Beispiel 13

### 6-(2,5-Diphenyl-1-pyrrolyl)-7-chlorchinoxalin-2,3(1H,4H)-dion Entsprechend der Vorschrift von Beispiel 5d wurden 16,5 mmol 6-Ammonio-7-chlorchinoxalin-2,3-(1H,4H)-dionchlorid mit 16,5 mmol 2,5-Diphenyl-2,5-dimethoxytetrahydrofuran umgesetzt.

Ausbeute: 13 %
Schmp. > 230°C
¹H-NMR (D₆-DMSO):
   - δ(ppm):: 6,5 (2H); 7,2 (1H); 12,0 (2H)

### Beispiel 14

### 6-(3-Formyl-1-pyrrolyl)-7-chlorchinoxalin-2,3(1H,4H)-dion

Entsprechend der Vorschrift von Beispiel 5d wurden 16,5 mmol 6-Ammonio-7-chlorchinoxalin-2,3-(1H,4H)-dionchlorid mit 16,5 mmol 3-Formyl-2,5-dimethoxytetrahydrofuran umgesetzt.
Ausbeute: 77 %
Schmp. > 300°C
¹H-NMR (D₆-DMSO):
   - δ(ppm):: 6,6 (s,1H); 7,15 (s,1H); 7,2 (s,1H); 7,3 (s,1H); 7,9 (s,1H); 9,8 (s,1H); 12,5 (m,2H)

### Beispiel 15

### 6-(3-Propionyl-1-pyrrolyl)-7-chlorchinoxalin-2,3(1H,4H)-dion

Entsprechend der Vorschrift von Beispiel 5d wurden 14,2 mmol 6-Ammonio-7-chlorchinoxalin-2,3-(1H,4H)-dionchlorid mit 14,2 mmol 3-Propionyl-2,5-dimethoxytetrahydrofuran umgesetzt.
Ausbeute: 69 %
Schmp. > 300°C
¹H-NMR (D₆-DMSO):
   - δ(ppm):: 1,05 (t,3H); 2,7 (q,2H); 6,6 (dd,1H); 7,0 (dd,1H); 7,1 (s,1H); 7,25 (s,1H); 7,8 (m,1H); 12,0 (2H)

### Beispiel 16

### 6-(2-Methoxy-1-pyrrolyl)-chinoxalin-2,3(1H,4H)-dion

Entsprechend der Vorschrift von Beispiel 5d wurden 28 mmol 6-Aminochinoxalin-2,3-(1H,4H)-dion mit 28 mmol 2,2,5-Trimethoxytetrahydrofuran umgesetzt.
Ausbeute: 97 %
Schmp. > 310°C
¹H-NMR (D₆-DMSO):
   - δ(ppm):: 3,6 (3H); 6,3 (1H); 7,0 (2H); 7,1 (1H); 7,2 (2H); 12,0 (2H)
Beispiel 17

### 5-(1-Pyrrolyl)-7-trifluormethylchinoxalin-2,3(1H,4H)-dion

Entsprechend der Vorschrift von Beispiel 5d wurden 12,2 mmol 5-Amino-7-trifluormethylchinoxalin-2,3-(1H,4H)-dion mit 12,2 mmol 2,5-Dimethoxytetrahydrofuran umgesetzt.
Ausbeute: 48 %
Schmp. > 320°C
¹H-NMR (D₆-DMSO):
   - δ(ppm):: 6,3 (2H); 7,0 (2H); 7,4 (1H); 7,7 (1H); 11,3 (1H); 12,2 (1H)

### Beispiel 18

### 9-(2-Methoxycarbonyl-1-pyrrolyl)-benzo[f]-chinoxalin-2,3(1H,4H)-dion

Entsprechend der Vorschrift von Beispiel 5d wurden 8,8 mmol 9-Amino-benzo[f]-chinoxalin-2,3-(1H,4H)-dion mit 8,8 mmol 2-Methoxycarbonyl-2,5-dimethoxytetrahydrofuran umgesetzt.
Ausbeute: 58 %
Schmp. > 300°C
¹H-NMR (D₆-DMSO):
   - δ(ppm):: 3,5 (3H); 6,5 (1H); 7,0 (1H); 7,1 (1H); 7,3 (2H); 7,5 (1H); 7,6 (1H); 8,6 (1H); 12,5 (2H)

### Beispiel 19

### 9-(2,5-Dimethyl-1-pyrrolyl)-benzo[f]-chinoxalin-2,3-(1H,4H)-dion

Entsprechend der Vorschrift von Beispiel 5d wurden 8,8 mmol 9-Amino-benzo[f]-chinoxalin-2,3-(1H,4H)-dion mit 8,8 mmol 2,5-Dimethyl-2,5-dimethoxytetrahydrofuran umgesetzt.
Ausbeute: 27 %
Schmp. > 320°C
¹H-NMR (D₆-DMSO):
   - δ(ppm):: 1,8 (6H); 5,95 (2H); 6,95 (1H); 7,3 (1H); 7,5 (1H); 7,7 (1H); 8,7 (1H); 12,5 (2H)

### Beispiel 20

### 9-(1-Pyrrolyl)-benzo[f]-chinoxalin-2,3(1H,4H)-dion

Entsprechend der Vorschrift von Beispiel 5d wurden 8,8 mmol 9-Amino-benzo[f]-chinoxalin-2,3-(1H,4H)-dionhydrochlorid mit 8,8 mmol 2,5-Dimethoxytetrahydrofuran umgesetzt.
Ausbeute: 62 %
Schmp. > 300°C
¹H-NMR (D₆-DMSO):
   - δ(ppm):: 6,3 (2H); 7,0 (2H); 7,3 (1H); 7,5 (2H); 7,7 (1H); 8,7 (1H); 12,5 (2H)

### Beispiel 21

### 7,8(6H,9H)-Dioxopyrazino-[5,6-g]-pyrrolo[1,2-c]-chinoxalin-2(4H)-on

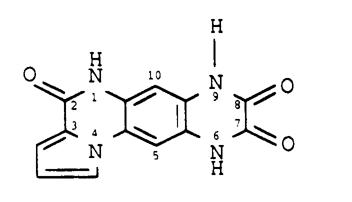

Eine Lösung von 4,8 g (14,5 mmol) 6-(2-Methoxycarbonyl-1-pyrrolyl)-7-nitrochinoxalin-2,3(1H,4H)-dion in 250 ml Eisessig wurde auf 80°C erwärmt, portionsweise mit 8 g (145,3 mmol) Eisenpulver versetzt und 2 Stunden unter Rückfluß erhitzt. Nach dem Einengen der Reaktionsmischung wurde das Rohprodukt mit verdünnter Salzsäure versetzt. Das Produkt wurde abfiltriert und getrocknet.
Ausbeute: 72 %
Schmp. > 320°C
¹H-NMR (D₆-DMSO):
   - δ(ppm):: 6,7 (1H); 7,1 (1H); 7,4 (1H); 7,6 (1H); 7,9 (1H); 11,3 (1H); 12,0-12,2 (2H)

### Beispiel 22

### 6-Chlor-7-(2-methoxycarbonyl-1-pyrrolyl)-chinoxalin-2,3-(1H,4H)-dion

10 g (47,25 mmol) 7-Amino-6-chlor-chinoxalin-2,3-(1H,4H)-dion und 8,9 g (47,25 mmol) 2,5-Dimethoxytetrahydrofuran-2-ylcarbonsäuremethylester wurden in 150 ml Essigsäure 2 h unter Rückfluß gekocht. Anschließend wurde der Niederschlag abgesaugt. Man erhielt 6,6 g (44 %) des
Produktes. Schmp. > 260°C.
¹H-NMR (D₆-DMSO):
δ= 3,6 (3H); 6,35 (1H); 7,0 (1H); 7,1 (1H); 7,15 (1H); 7,25 (1H) und 12,1 (breit, 2H) ppm.

### Beispiel 23

### 1-(Methoxycarbonylmethyl)-7-(2,5-dimethyl-1-pyrrolyl)-6-trifluormethyl-chinoxalin-2,3-(1H,4H)-dion

2,2 g (6,8 mmol) 6-(2,5-Dimethyl-1-pyrrolyl)-7-trifluormethyl-chinoxalin-2,3-(1H,4H)-dion (Beispiel 11) wurden unter N₂-Schutzgas in 25 ml wasserfreiem Dimethylformamid gelöst und portionsweise mit 0,245 (8,2 mmol) 80 %igem Natriumhydrid versetzt. Nachdem man noch eine Stunde bei Raumtemperatur gerührt hatte, wurde die Lösung auf -25°C gekühlt und 1,4 g (8,9 mmol) Bromessigsäureethylester, in 3 ml Dimethylformamid gelöst, zugetropft. Alles wurde 90 Minuten gerührt. Anschließend wurde der Ansatz auf Eis gegossen, mit verdünnter Salzsäure sauer gestellt und mit Essigsäureethylester extrahiert. Die organische Phase wurde getrocknet und im Vakuum einrotiert. Der Rückstand wurde chromatographisch an Kieselgel (Fließmittel: n-Heptan/Essigsäureethylester = 2/1) gereinigt, wobei 0,8 g (30 %) des Produktes anfielen.
Schmp. > 220°C (Z.)
¹H-NMR (CDCl₃): δ= 1,85 (6H); 3,65 (3H); 5,1 (2H); 5,8 (2H); 7,4 (1H) und 7,7 (1H) ppm.

### Beispiel 24

### 1-(Methoxycarbonylmethyl)-7-(1-pyrrolyl)-6-trifluormethyl-chinoxalin-2,3-(1H,4H)-dion

35 g (118,6 mmol) 6-(1-Pyrrolyl)-7-trifluormethyl-chinoxalin-2,3-(1H,4H)-dion (Beispiel 2) wurden analog Beispiel 23 mit 23,8 g (155,5 mmol) Bromessigsäuremethylester umgesetzt. Die chromatographische Reinigung erfolgte mit dem Fließmittel Methylenchlorid/Essigsäureethylester = 3/2. Man erhielt 17,3 g (40 %) des Produktes.
Schmp. 141-142°C.
¹H-NMR (D₆-DMSO):
δ= 3,7 (3H); 5,0 (2H); 6,2 (2H); 6,85 (2H); 7,6 (1H); 7,65 (1H) und 12,5 (breit) ppm.

### Beispiel 25

### 1-(Carboxymethyl)-7-(1-pyrrolyl)-6-trifluormethyl-chinoxalin-2,3-(1H,4H)-dion

2 g (5,45 mmol) 1(Methoxycarbonylmethyl)-7-(1-pyrrolyl)-6-trifluormethyl-chinoxalin-2,3-(1H,4H)-dion (Beispiel 24) wurden in 30 ml Tetrahydrofuran gelöst und mit 0,4 g (16,4 mmol) Lithiumhydroxid, gelöst in 4 ml Wasser, versetzt. Man rührte 16 h bei Raumtemperatur. Anschließend wurde das organische Lösungsmittel im Vakuum entfernt. Die wäßrige Phase wurde mit 1 M Salzsäure sauer gestellt und mit Essigsäureethylester extrahiert. Die organische Phase wurde getrocknet und im Vakuum eingeengt. Der Rückstand wurde aus Methanol umkristallisiert, wobei man 1,3 g (69 %) des Produktes erhielt. Schmp. 161-163°C (Z.)
¹H-NMR (D₆-DMSO):
δ= 4,95 (2H); 5,0 (2H); 6,2 (2H); 6,9 (2H); 7,5 (1H); 7,6 (1H) und 11,5 (breit) ppm.

### Beispiel 26

### (7-(1-Pyrrolyl)-6-trifluormethyl-chinoxalin-2,3-(1H,4H)-dion-1-yl)-essigsäuremethylamid

3 g (8,2 mmol) 1-(Methoxycarbonylmethyl)-7-(1-pyrrolyl)-6-trifluormethyl-chinoxalin-2,3-(1H,4H)-dion wurden in 100 ml ca. 5 M methanolischer Ammoniak-Lösung gelöst und bei Raumtemperatur für 2 h gerührt. Anschließend wurde der Ansatz im Vakuum eingeengt und der Rückstand chromatographisch an Kieselgel (Fließmittel: Methylenchlorid/Methanol/Tetrahydrofuran = 4:2:1) gereinigt. Man erhielt 1,8 g (63 %) des Produktes. Schmp. > 250°C.
¹H-NMR (D₆-DMSO):
δ= 2,3 (3H); 4,4 (2H); 6,2 (2H); 6,85 (2H); 7,0 (1H) und 7,5 (1H) ppm.

### Beispiel 27

### 1-(7-Chlor-chinoxalin-2,3-(1H,4H)-dion-6-yl)-pyrrolyl-2-carbonsäurebenzylamid

2,0 g (6,5 mmol) 1-Carboxymethyl)-7-(1-pyrrol)-6-trifluormethyl-chinoxalin-2,3-(1H,4H)-dion und 0,64 g (5,9 mmol) Benzylamin wurden in 50 ml wasserfreiem Dimethylformamid gelöst. Bei 0°C wurden nacheinander 1,6 g (5,9 mmol) Phosphorsäurediphenylesterazid gelöst in wenig Dimethylformamid, und 1,3 g (12,5 mmol) Triethylamin zugetropft. Man rührte alles für 4 h bei Raumtemperatur. Danach wurde der Ansatz auf Wasser gegossen, das mit Natriumhydrogenkarbonat abgepuffert war. Der entstandene Niederschlag wurde abgesaugt. Man erhielt 1,6 (62 %) des Produktes. Schmp. 241-242°C.
¹H-NMR (D₆-DMSO):
δ= 4,3 (2H); 6,3 (1H); 6,9 (1H); 7,0 (1H); 7,05 (1H); 7,1-7,4 (6H) und 8,7 (1H) ppm.

### Beispiel 28

### 1-Cyclohexyl-7-nitro-6-(1-pyrrolyl)-chinoxalin-2,3-(1H,4H)dion

a) N-Cyclohexyl-2,4-dinitroanilin
   50 g (0,247 Mol) 1-Chlor-2,4-dinitrobenzol, 73,5 g (0,74 Mol) Cyclohexylamin, 68 g (0,5 Mol) Kaliumkarbonat und 20 ml Wasser wurden in 400 ml Dimethylformamid 3 h auf 80-90°C erwärmt. Anschließend wurde der Ansatz im Vakuum eingeengt und der Rückstand zwischen Wasser und Essigester verteilt. Die organische Phase wurde getrocknet und im Vakuum eingeengt. Man erhielt 63,9 g (98 %) des Produktes. Schmp. 155°C.
   ¹H-NMR (D₆-DMSO):
   δ= 1,2-2,1 (10H); 3,8 (1H); 7,3 (1H); 8,2 (1H); 8,5 (NH) und 8,9 (1H) ppm.
b) N-Cyclohexyl-2,4-diaminoanilin
   60,0 g (0,23 Mol) N-Cyclohexyl-2,4-dinitroanilin wurden in 500 ml Tetrahydrofuran gelöst und nach Zugabe von 3 g Palladium/Kohle (10 %) hydriert. Anschließend wurde filtriert und das Filtrat im Vakuum eingeengt. Man erhielt 47,2 g (100 %) des Produktes.
   ¹H-NMR (D₆-DMSO):
   δ= 1,0-2,0 (10H); 2,8 (1H); 4,0-4,0 (3H,NH); 5,75 (1H); 5,9 (1H) und 6,2 (1H) ppm.
c) N-(1-Cyclohexyl-chinoxalin-2,3-(1H,4H)-dion-6-yl)-oxalsäuremonoethylesteramid
   46 g (0,224 Mol) N-Cyclohexyl-2,4-diaminoanilin wurden in 500 ml Oxalsäurediethylester 2 h unter Rückfluß gekocht. Nach dem Abkühlen wurde mit Ether versetzt und der anfallende Niederschlag abgesaugt. Man erhielt 48,3 g (60 %) des Produktes. Schmp. 271-272°C (Z.).
   ¹H-NMR (D₆-DMSO):
   δ= 1,3 (3H); 1,3-2,0 (8H); 2,3-2,5 (2H); 4,4 (2H); 4,5 (1H); 7,5 (1H); 7,6 (1H); 7,8 (1H), 10,9 (1H) und 12,1 (1H) ppm.
d) N-(1-Cyclohexyl-7-nitro-chinoxalin-2,3-(1H,4H)-dion-6-yl)-oxalsäuremonoethylesteramid
   48 g (0,133 Mol) N-(1-Cyclohexyl-chinoxalin-2,3-(1H,4H)-dion-6-yl)-oxalsäuremonoethylesteramid wurden in 1 l konzentrierter Schwefelsäure gelöst und bei 0°C portionsweise mit 11,35 g (0,133 Mol) Natriumnitrat versetzt. Der Ansatz wurde für weitere 2 h bei 0°C gerührt. Anschließend wurde alles auf viel Eis gegossen und der anfallende Niederschlag abgesaugt. Man erhielt 50,1 g (93 %) des Produktes. Schmp. 260°C.
   ¹H-NMR (D₆-DMSO):
   δ= 1,4-2,0 (11H); 2,3 (2H); 4,3 (2H); 4,5 (1H); 8,1 (1H); 8,2 (1H); 11,5 (1H) und 12,5 (1H) ppm.
e) 6-Amino-1-cyclohexyl-7-nitro-chinoxalin-2,3-(1H,4H)-dion
   42 g (0,1 Mol) N-(1-cyclohexyl-7-nitro-chinoxalin-2,3-(1H,4H)-dion-6-yl)-oxalsäuremonoethylesteramid wurden in einem Gemisch aus 1 l konzentrierter Schwefelsäure und 500 ml Ethanol für 4 h unter Rückfluß gekocht. Der Niederschlag wurde abgesaugt und man erhielt 34 g (100 %) des Produktes. Schmp. > 250°C (Z.)
   ¹H-NMR (D₆-DMSO):
   δ= 1,1-2,0 (8H); 2,3 (2H); 4,4 (1H); 6,7 (1H); 7,0-7,5 (2H, breit, NH₂); 8,0 (1H) und 12,2 (1H) ppm.
f) 1-Cyclohexyl-7-nitro-6-(1-pyrrolyl)-chinoxalin-2,3-(1H,4H)-dion
   3,0 g (99 mmol) 6-Amino-1-cyclohexyl-7-nitro-chinoxalin-2,3-(1H,4H)-dion und 1,3 g (99 mmol) 2,5-Dimethoxytetrahydrofuran wurden in 70 ml Essigsäure für 30 Minuten unter Rückfluß gekocht. Anschließend wurde alles mit Wasser verdünnt und der Niederschlag abgesaugt. Man erhielt 2,4 g (69 %) des Produktes. Schmp. 197°C.
   ¹H-NMR (D₆-DMSO):
   δ= 1,1-2,0 (8H); 2,3 (2H); 4,5 (1H); 6,3 (2H); 6,9 (2H); 7,2 (1H); 8,2 (1H) und 12,4 (1H) ppm.

### Beispiel 29

### 1-Cyclohexyl-6-(2-methoxycarbonyl-1-pyrrolyl)-7-nitro-chinoxalin-2,3-(1H,4H)-dion

10 g (33 mmol) 6-Amino-1-cyclohexyl-7-nitro-chinoxalin-2,3-(1H,4H)-dion (Beispiel 28c), 6,9 g (36 mmol) 2,5-Dimethoxytetrahydrofuran-2-yl-carbonsäuremethylester und eine Spatelspitze p-Toluolsulfonsäure wurden in einem Gemisch aus 50 ml Dimethylformamid und 50 ml Toluol am Wasserabscheider unter Rückfluß gekocht. Nach vollständiger Reaktion wurde der Ansatz im Vakuum eingeengt und der Rückstand mit Wasser behandelt. Der Niederschlag wurde abgesaugt. Man erhielt 11 g (79 %) des Produktes. Schmp. 211-212°C.
¹H-NMR (D₆-DMSO):
δ= 1,2-2,0 (8H); 2,4 (2H); 3,6 (3H); 4,5 (1H); 6,4 (1H); 7,05 (1H); 7.3 (1H); 8,0 (1H); 8,3 (1H) und 12,5 (1H) ppm.

### Beispiel 30

### 1-Ethyl-7-nitro-6-(1-pyrrolyl)-chinoxalin-2,3-(1H,4H)-dion

a) 6-Amino-1-ethyl-7-nitro-chinoxalin-2,3-(1H,4H)-dion
   Analog Beispiel 28 a-d wurde aus 1-Chlor-2,4-dinitrobenzol und Ethylamin das N-(1-Ethyl-7-nitro-chinoxalin-2,3-(1H,4H)-dion-6-yl)-oxalsäuremonoethylesteramid hergestellt.
   24 g (68,6 mmol) dieses Amids wurden in einem Gemisch aus 13 ml konzentrierter Schwefelsäure, 240 ml Wasser und 250 ml Ethanol für 3 h unter Rückfluß gekocht. Anschließend wurde der Niederschlag abgesaugt. Man erhielt 16,2 g (98 %) des Produktes.
   ¹H-NMR (D₆-DMSO):
   δ= 1,2 (3H); 4,1 (2H); 6,7 (1H); 7,2-7,6 (2H, NH₂); 7.8 (1H) und 12,2 (1H) ppm.
b) 1-Ethyl-7-nitro-6-(1-pyrrolyl)-chinoxalin-2,3-(1H,4H)-dion
   4,0 g (16 mmol) 6-Amino-1-ethyl-7-nitrochinoxalin-2,3-(1H,4H)-dion, 2,4 g (18 mmol) 2,5-Dimethoxytetrahydrofuran und eine Spatelspitze p-Toluolsulfonsäure wurden in einem Gemisch aus 50 ml Dimethylformamid und 50 ml Toluol für 3 h unter Rückfluß am Wasserabscheider erhitzt. Danach wurde der Ansatz im Vakuum eingeengt und der Rückstand mit Methanol behandelt. Der Niederschlag wurde abgesaugt, so daß man 18 g (3 %) des Produktes erhielt. Schmp. 248-250°C.
   ¹H-NMR (D₆-DMSO):
   δ= 1,2 (3H); 4,2 (2H); 6,2 (2H); 6,9 (2H); 7,2 (1H); 8,0 (1H) und 12,4 (1H) ppm.

### Beispiel 31

### 9-(2,5-Dimethyl-1-pyrrolyl)-1-(ethoxycarbonylmethyl)-benzo[f]chinoxalin-2,3-(1H,4H)-dion

a) 2-Methoxy-1-nitro-naphthalin
   100 g (0,63 Mol) 2-Methoxynaphthalin wurden in 1,2 l Essigsäure gelöst und bei 10°C langsam 100 ml 65 %iger Salpetersäure zugetropft. Man rührte noch 2 h bei 10°C. Anschließend wurde der anfallende Niederschlag abgesaugt. Man erhielt 72,5 g (57 %). Schmp. 129-130°C.
   ¹H-NMR (D₆-DMSO):
   δ= 4,1 (3H); 7,5-7,8 (4H); 8,05 (1H) und 8,2 (1H) ppm.
b) N-(1-Nitro-2-naphthyl)-aminoessigsäure
   50 g (0,246 Mol) des Produktes 31 a, 100 g (1,3 Mol) Glycin und 100 g (0,7 Mol) Kaliumkarbonat wurden in 400 ml Diethylenglykol für 10 Min. auf 140°C erwärmt. Anschließend wurde der Ansatz sofort auf Eiswasser gegossen, mit konzentrierter Salzsäure sauer gestellt und mit 1,5 l Essigsäureethylester extrahiert. Der resultierende Niederschlag wurde abgesaugt. Man erhielt 29,6 g (49 %) des Produktes. Schmp. > 155°C (Z.).
   ¹H-NMR (D₆-DMSO):
   δ= 4,3 (2H); 7,2 (1H); 7,35 (1H); 7,6 (1H); 7,8 (1H); 8,0 (1H); 8,4 (1H); 8,7 (1H,NH) und ca. 12 (breit) ppm.
c) N-(1-Amino-2-naphthyl)-aminoessigsäure
   28 g (0,11 Mol) des Produktes 31 b wurden in 300 ml Ethanol gelöst und nach Zugabe von 10 ml Essigsäure und 2 g Palladium/Kohle (10 %) bei Raumtemperatur und 1 bar hydriert. Anschließend wurde filtriert und das Filtrat im Vakuum eingeengt. Man erhielt 23,9 g (98 %) des Produktes.
   ¹H-NMR (D₆-DMSO):
   δ= 3,8 (2H); 6,2 (NH); 7,1 (1H); 7,2 (1H); 7,3-7,5 (2H); 7,7 (1H); 8,1 (1H) und 10,5 (1H, CO₂H) ppm.
d) 1-(Carboxymethyl)-benzo[f]chinoxalin-2,3-(1H,4H)-dion
   22 g (=,1 Mol) des Produktes 31 c und 28 ml (0,2 Mol) Triethylamin wurden in 300 ml wasserfreiem Tetrahydrofuran gelöst. Bei 0 bis 5°C wurden danach 12,5 ml (0,11 Mol) Oxalsäureethylesterchlorid, gelöst in 50 ml wasserfreiem Tetrahydrofuran, zugetropft. Man rührte alles für 1 h bei 0 bis 50°C. Anschließend wurde der Ansatz im Vakuum eingeengt und der Rückstand zwischen Essigsäureethylester und Wasser verteilt. Die organische Phase wurde getrocknet und im Vakuum eingeengt. Dieser Rückstand wurde in einem Gemisch aus 50 ml Ethanol und 200 ml konzentrierter Salzsäure für 1,5 h unter Rückfluß gekocht. Anschließend wurde alles auf Eiswasser gegossen und der Niederschlag abgesaugt. Man erhielt 16,8 g (61 %) des Produktes. Schmp. 288-291°C (Z.).
   ¹H-NMR (D₆-DMSO):
   δ= 5,0 (2H); 7,4-7,6 (3H); 7,7 (1H); 7,9 (1H); 8,6 (1H); 12,3 (1H) und ca. 13 (breit) ppm.
e) 1-(Ethoxycarbonylmethyl)-benzo[f]chinoxalin-2,3-(1H,4H)-dion
   17 g (62,9 mmol) des Produktes 31 d wurden in einem Gemisch aus 250 ml konzentrierter Schwefelsäure und 70 ml Ethanol für 3 h bei 55°C gerührt. Anschließend wurde alles auf Eiswasser gegossen und der Niederschlag abgesaugt. Man erhielt 18,3 g (98 %) des Produktes.
   Schm. > 250°C.
   ¹H-NMR (D₆-DMSO):
   δ= 1,2 (3H); 4,2 (2H); 5,1 (2H); 7,3-7,6 (4H); 7,85 (1H) und 8,7 (1H) ppm.
f) 1-(Ethoxycarbonylmethyl)-9-nitro-benzo[f]chinoxalin-2,3-(1H,4H)-dion
   18 g (60,3 mmol) des Produktes 31 e wurden in 200 ml Essigsäure suspendiert und bei Raumtemperatur vorsichtig mit 50 ml 65 %iger Salpetersäure versetzt. Danach wurde alles auf 80°C erwärmt. Nach Beendigung der Reaktion (Farbumschlag der Lösung von dunkel auf hellrot) wurde alles auf Eis gegossen und der Niederschlag abgesaugt. Man erhielt 16,9 g (82 %) des Produktes. Schmp. > 250°C.
   ¹H-NMR (D₆-DMSO):
   δ= 1,3 (3H); 4,2 (2H); 5,2 (2H); 7,7 (2H); 8,3-8,4 (2H); 8,8 (1H) und 12,7 (1H) ppm.
g) 9-Amino-1-(ethoxycarbonylmethyl)-benzo[f]chinoxalin-2,3-(1H,4H)-dion
   16,5 g (48 mmol) des Produktes 31 f wurden in 150 ml Dimethylformamid gelöst und nach Zusatz von 1,5 g (Palladium/Kohle (10 %) bei 1 bar und Raumtemperatur hydriert. Anschließend wurde filtriert und das Filtrat im Vakuum eingeengt. Man erhielt 13,1 g (88 %) des Produktes.
   Schmp. > 250°C.
   ¹H-NMR (D₆-DMSO):
   δ= 1,2 (3H); 4,2 (2H); 5,0 (2H); 5,7-6,0 (2H, NH, breit); 6,6 (1H); 7,4 (1H); 7,5 (1H); 8,2 (1H); 8,5 (1H) und 12,0 (1H) ppm.
h) 9-(2,5-Dimethyl-1-pyrrolyl)-1-(ethoxycarbonylmethyl-benzo[f]chinoxalin-2,3-(1H,4H)-dion
   12.5 g (40 mmol) des Produktes 31 g und 4,7 ml (40 mmol) 2,5-Hexandion wurden in 130 ml Essigsäure für 15 Minuten unter Rückfluß gekocht. Anschließend wurde alles im Vakuum eingeengt, der Rückstand in wenig Ethanol dispergiert und abgesaugt. Man erhielt 10,0 g (65 %) des Produktes.
   ¹H-NMR (D₆-DMSO):
   δ= 1,2 (3H); 1,8 (6H); 4,15 (2H); 5,2 (2H); 5,9 (2H); 6,9 (1H); 7,55 (1H); 7,7 (1H); 7,75 (1H); 8,75 (1H); 12,0 (breit, 1H) und 12,5 (1H) ppm.

### Beispiel 32

### 1-Ethyl-6-nitro-7-(1-pyrrolyl)-chinoxalin-2,3-(1H,4H)-dion

a) 1-Ethyl-7-nitro-chinoxalin-2,3-(1H,4H)-dion
   14,2 g (57 mmol) 6-Amino-1-ethyl-7-nitro-chinoxalin-2,3-(1H,4H)-dion (Beispiel 30 a) wurden in 340 ml Essigsäure suspendiert. Dazu tropfte man bei einer Temperatur von 10-20°C eine Lösung aus 5,1 g (74 mmol) Natriumnitrit und 80 ml konzentrierter Schwefelsäure. Man rührte alles noch ca. 30 Minuten. Danach wurde diese Lösung zu einer weiteren Suspension aus 16,3 g (114 mmol) Kupfer-I-oxid und 140 ml Ethanol getropft. Man rührte noch weitere 10 Minuten und goß danach den Ansatz auf 1 l Eiswasser. Danach wurde filtriert und das Filtrat mit Methylenchlorid extrahiert. Die organische Phase wurde getrocknet und im Vakuum eingeengt. Man erhielt 8,5 g (64 %) des Produktes. Schmp. > 300°C.
   ¹H-NMR (D₆-DMSO):
   δ= 1,2 (3H); 4,2 (2H); 7,3 (1H); 8,1 (2h) und 12,5 (1H) ppm.
b) 7-Amino-1-ethyl-chinoxalin-2,3-(1H,4H)-dion
   8,3 g (35 mmol) 1-Ethyl-6-nitro-chinoxalin-2,3-(1H,4H)-dion wurden in 300 ml Dimethylformamid gelöst und nach Zugabe von 2 g Palladium/Kohle (10 %ig) hydriert. Anschließend wurde filtriert und das Filtrat im Vakuum eingeengt. Man erhielt 5,9 g (81 %) des Produktes.
   Schmp. 271-273°C (Z.).
   ¹H-NMR (D₆-DMSO):
   δ= 1,2 (3H); ca. 3,3 (breit, 1H); 4,05 (2H); 6,4 (1H); 6,6 (1H); 6,9 (1h) und ca. 11,7 (breit) ppm.
c) 7-Acetamido-1-ethyl-chinoxalin-2,3-(1H,4H)-dion
   5,5 g (27 mmol) 7-Amino-1-ethyl-chinoxalin-2,3-(1H,4H)-dion wurden in 75 ml Essigsäureanhydrid 1 h unter Rückfluß erhitzt. Anschließend wurde der Ansatz auf Eiswasser gegossen und filtriert. Das Filtrat wurde im Vakuum eingeengt. Man erhielt 5,7 g (86 %) des Produktes. Schmp. 303-304°C (Z.).
   ¹H-NMR (D₆-DMSO):
   δ= 1,2 (3H); 4,0 (2H); 7,0 (1H); 7,3 (1H); 7,7 (1H); 10,0 (1h) und ca. 12 (breit) ppm.
d) 7-Amino-1-ethyl-6-nitro-chinoxalin-2,3-(1H,4H)-dion
   5,2 g (21 mmol) 7-Acetamin-1-ethyl-chinoxalin-2,3-(1H,4H)-dion wurden in 75 ml konzentrierter Schwefelsäure gelöst und bei 0-5°C mit 2 g (23 mmol) Natriumnitrat versetzt. Anschließend wurde noch für 2 h bei 0-5°C gerührt. Der Ansatz wurde auf Eiswasser gegossen und für 16 h bei Raumtemperatur gerührt. Danach wurde mit verdünnter Natronlauge und Natriumhydrogenkarbonat-Lösung neutralisiert und alles im Vakuum eingeengt. Der Rückstand wurde mit Dimethylformamid extrahiert und anschließend die organische Phase erneut eingeengt. Der Rückstand wurde an Kieselgel (Fließmittel: Toluol:Aceton:Essigsäure = 10:10:1) gereinigt. Man erhielt 1,2 g (23 %) des Produktes.
   ¹H-NMR (D₆-DMSO):
   δ= 1,2 (3H); 4,0 (2H); 6,9 (1H); 7,3 (2H); 7,7 (1H) und ca. 12 (breit) ppm.
e) 1-Ethyl-6-nitro-7-(1-pyrrolyl)-chinoxalin-2,3-(1H,4H)-dion
   0,9 g (3,5 mmol) 7-Amino-1-ethyl-6-nitro-chinoxalin-2,3-(1H,4H)-dion und 0,58 g (4,4 mmol) 2,5-Dimethoxytetrahydrofuran wurden in 20 ml Essigsäure für 2 h unter Rückfluß gekocht. Anschließend wurde der Ansatz im Vakuum eingeengt und der Rückstand chromatographisch am Kieselgel (Fließmittel: Toluol/Aceton/Essigsäure = 10:10:1) gereinigt. Man erhielt 0,7 g (68 %) des Produktes. Schmp. > 300°C.
   ¹H-NMR (D₆-DMSO):
   δ= 1,2 (3H); 4,2 (2H); 6,3 (1H); 6,9 (2H); 7,5 (1H); 7,8 (1H) und ca. 12,5 (breit) ppm.

### Beispiel 33

### 9-(2,3-Dimethyl-1-pyrrolyl-4-(ethoxycarbonylmethyl)-benzo[f]chinoxalin-2,3-(1H,4H)-dion

a) N-(Ethoxycarbonylmethyl)-N-(2,4-dinitro-1-naphthyl)-4-methylphenylsulfonsäureamid
   60 g (155 mmol) N-(2,4-Dinitro-1-naphthyl)-4-methylphenylsulfonsäureamid (J. Soc. Chem. 1935, 1855) wurden in 500 ml wasserfreiem Dimethylformamid unter Schutzgas gelöst und portionsweise mit 19,1 g (170 mmol) Kalium-tert.-butanolat versetzt. Man rührte ca. 30 Minuten bei Raumtemperatur und tropfte anschließend 25,9 g (155 mmol) Bromessigsäureethylester, gelöst in 50 ml wasserfreiem Dimethylformamid, zu. Danach wurde das Reaktionsgemisch für 90 Minuten auf 100°C erwärmt. Nach dem Abkühlen wurde alles auf Eiswasser gegossen und die wäßrige Phase mit Essigsäureethylester extrahiert. Die organische Phase wurde getrocknet und im Vakuum eingeengt. Der Rückstand wurde mit einem Gemisch aus 50 ml Toluol und 100 ml Ethanol versetzt und erwärmt. Das anfallende Kristallisat wurde abgesaugt. Man erhielt 55,7 g (76 %) des Produktes. Schmp. 106-107°C.
   ¹H-NMR (D₆-DMSO):
   δ= 1,2 (3H); 2,4 (3H); 4,2 (2H); 4,7 (1H); 4,9 (1H); 7,25 (2H); 7,4 (2H); 7,7 (1H); 7,9 (1H); 8,3 (1H), 8,6 (1H) und 8,8 (1H) ppm.
b) N-(2,4-Dinitro-1-naphthyl)aminoessigsäureethylester
   50 g (106 mmol) des Produktes 33a wurden in 200 ml 90 %iger Schwefelsäure für 1 h bei Raumtemperatur gerührt. Anschließend wurde der Ansatz auf Wasser gegossen und der anfallende Niederschlag abgesaugt. Man erhielt 28,4 g (85 %) des Produktes. Schmp. > 305°C.
   ¹H-NMR (D₆-DMSO):
   δ= 4,0 (2H); 7,6 (1H); 7,9 (1H); 8,6 (1H); 9,0 (1H) und ca. 11 (breit) ppm.
c) N-(2,4-Dinitro-1-naphthyl)-N-(ethoxycarbonylmethyl)-oxalsäuremonoethylesteramid
   22 g (68,9 mmol) des Produktes 33b wurden in 250 ml Pyridin gelöst und bei Raumtemperatur tropfenweise mit 8,1 ml (72,4 mmol) Oxalsäuremonoethylesterchlorid versetzt. Man rührte alles für 1 h und engte danach den Ansatz im Vakuum ein. Der Rückstand wurde zwischen Essigsäureethylester und Wasser verteilt, die organische Phase wurde getrocknet und im Vakuum eingeengt. Man erhielt 21,3 g (74 %) des Produktes. Schmp. 101-102°C.
   ¹H-NMR (D₆-DMSO):
   δ= 0,8 (3H); 1,2 (3H); 3,9 (2H); 4,1 (2H); 4,3 (1H); 4,7 (1H); 7,9-8,1 (2H); 8,4 (1H); 8,6 (1H) und 8,8 (1H) ppm.
d) 9-Amino-4-(ethoxycarbonylmethyl)-benzo[f]chinoxalin-2,3-(1H,4H)-dion
   20 g (47,7 mmol) des Produktes 33e wurden in einem Gemisch aus 10 ml Essigsäure und 300 ml Tetrahydrofuran gelöst und nach Zusatz von 1,5 g Palladium/Kohle (10 %) hydriert. Anschließend wurde filtriert und das Filtrat im Vakuum eingeengt. Man erhielt 13.8 g (93 %) des Produktes.
   Schmp. 294-295°C (Z.).
   ¹H-NMR (D₆-DMSO):
   δ= 1,2 (3H); 4,2 (2H); 5,8 (2H); 6,0 (2H, NH, breit); 6,5 (1H); 7,3 (1H); 7,4 (1H); 7,8 (1H); 8,2 (1H) und 12,0 (1H) ppm.
e) 9-(2,3-Dimethyl-1-pyrrolyl)-4-(ethoxycarbonylmethyl)-benzo[f]chinoxalin-2,3-(1H,4H)-dion
   13,0 g (41,5 mmol) des Produktes 33d und 4,9 ml (41,5 mmol) 2,5-Hexandion wurden in 150 ml Essigsäure für 1 h unter Rückfluß erhitzt. Anschließend wurde der Ansatz im Vakuum eingeengt, der Rückstand zwischen Essigsäurethylester und Wasser verteilt und die organische Phase getrocknet und im Vakuum eingeengt. Der Rückstand wurde chromatographisch an Kieselgel (Fließmittel: Toluol/Aceton 2:1) gereinigt. Man erhielt 8,3 g (52 %) des Produktes. Schmp. 132-133°C (Z.).
   ¹H-NMR (D₆-DMSO):
   δ= 1,2 (3H); 1,8 (6H); 4,2 (2H); 5,1 (2H); 5,9 (2H); 6,9 (1H); 7,3 (1H); 7,5 (1H); 7,6 (1H); 8,1 (1H) und 12,3 (breit) ppm.

### Beispiel 34

### 4-(Carboxymethyl)-9-(2,5-dimethyl-1-pyrrolyl)-benzo[f]chinoxalin-2,3-[1H,4H]-dion

7,0 g (17,9 mmol) des Beispiels 33 wurden in 100 ml Tetrahydrofuran gelöst und mit einer Lösung aus 1,3 g (53,7 mmol) Lithiumhydroxid in 30 ml Wasser versetzt. Alles wurde 4 h bei Raumtemperatur gerührt. Anschließend wurde das organische Lösungsmittel im Vakuum entfernt und die resultierende Wasserphase mit verdünnter Salzsäure verdünnt und mit Essigsäureethylester extrahiert. Diese organische Phase wurde getrocknet und im Vakuum eingeengt. Man erhielt 4,8 g (74 %) des Produktes. Schmp. > 210°C (Z.).
¹H-NMR (D₆-DMSO):
δ= 1,8 (6H); 4,9 (1H); 5,7 (1H); 5,9 (2H); 6,9 (1H); 7,4-7,7 (2H); 7,85+8,5 (1H); 8,1-8,2 (1H) und 12,3 (1H) ppm.

### Beispiel 35

### 2-(9-(2,5-Dimethyl-1-pyrrolyl)-benzo[f]chinoxalin-2,3-(1H,4H)-dion-4-yl)essigsäurebenzylamid

1,3 g (3,6 mmol) des Beispiels 34 und 0,43 ml (3,9 mmol) Benzylamin wurden in 30 ml wasserfreiem Dimethylformamid gelöst. Bei 0°C wurden nacheinander 0,85 ml (3,9 mmol) Phosphorsäurediphenylesterazid, gelöst in 10 ml wasserfreiem Dimethylformamid, und 1,1 ml (7,9 mmol) Triethylamin zugetropft. Man rührte 16 h bei Raumtemperatur. Anschließend wurde alles auf Eiswasser gegeben und mit Essigsäureethylester extrahiert. Die organische Phase wurde getrocknet und im Vakuum eingeengt. Der Rückstand wurde chromatographisch an Kieselgel (Fließmittel: Methylenchlorid/Methanol = 20:3) gereinigt. Man erhielt 1,1 g (70 %) des Produktes.
Schmp. > 290°C.
¹H-NMR (D₆-DMSO):
δ= 1,8 (6H); 4,4 (2H); 4,95 (2H); 5,9 (2H); 6,8 (1H); 7,2-7,5 (8H); 9,05 (1H) und ca. 12,5 (breit) ppm.

### Beispiel 36

### 6-Chlor-1-cyclohexyl-7-(1-pyrrolyl)-chinoxalin-2,3-(1H,4H)-dion

a) 4-Chlor-N-cyclohexyl-2-nitro-anilin
   51,5 g (0,27 Mol) 2,5-Dichlor-nitrobenzol, 22,3 g (0,27 Mol) Cyclohexylamin, 74,6 (0,54 Mol) Kaliumkarbonat und 0,5 g 18-crown-6 wurden in 300 ml Dimethylformamid für 4 h auf 100°C erwärmt. Anschließend wurde das Gemisch auf Wasser gegossen und mit Essigsäureethylester extrahiert. Die organische Phase wurde getrocknet und im Vakuum eingeengt. Der Rückstand wurde aus i-Propanol umkristallisiert, wobei 42,3 g (62 %) des Produktes anfielen.
   Schmp. 101-103°C.
   ¹H-NMR (D₆-DMSO):
   δ= 1,2-2,0 (10H); 3,7 (1H); 7,1 (1H); 7,5 (1H); 8,0 (1H) und 8,05 (1H) ppm.
b) 2-Amino-4-chlor-N-cyclohexyl-anilin
   41,6 g (0,16 Mol) des 4-Chlor--cyclohexyl-2-nitro-anilins wurden in 400 ml Ethanol gelöst, mit 4,2 g Raney-Nickel versetzt und bei 1 bar und 25°C hydriert. Der Ansatz wurde anschließend filtriert und das Filtrat im Vakuum eingeengt. Man erhielt 37,3 g (100 %) des Produktes.
   ¹H-NMR (D₆-DMSO):
   δ= 1,0-2,0 (10H); 3,1 (1H); 4,0-5,0 (3H, breit) und 6,3-6,6 (3H) ppm.
c) 6-Chlor-1-cyclohexyl-chinoxalin-2,3-(1H,4H)-dion
   34,5 g (0,15 Mol) des 2-Amino-4-chlor-N-cyclohexylanilins wurden in 500 ml Oxalsäurediethylester für 4 h unter Rückfluß erwärmt. Nach dem Abkühlen wurde der Niederschlag abgesaugt, mit n-Pentan gewaschen und getrocknet. Man erhielt 26.8 g (63 %) des Produktes.
   Schmp. 265-266°C.
   ¹H-NMR (D₆-DMSO):
   δ= 1,1-1,9 (8H); 2,3-2,5 (2H); 4,4 (1H); 7,1 (2H); 7,6 (1H) und 12 (breit) ppm.
d) 6-Chlor-1-cyclohexyl-7-nitro-chinoxalin-2,3-(1H,4H)-dion
   26,3 g (94 mmol) 6-Chlor-1-cyclohexyl-chinoxalin-2,3-(1H,4H)-dion wurden in 275 ml konzentrierter Schwefelsäure gelöst und anschließend bei 0°C 9,5 g (94 mmol) Kaliumnitrat portionsweise zugefügt. Man rührte dann 30 Min. bei 0°C und 2 h bei 25°C. Das Reaktionsgemisch wurde auf Eiswasser gegossen und der Niederschlag abgesaugt. Man erhielt 29,5 g (97 %) des Produktes. Schmp. > 300°C.
   ¹H-NMR (D₆-DMSO):
   δ= 1,1-1,9 (8H); 2,3-2,5 (2H); 4,4 (1H); 7,3 (1H) und ca. 12,5 (1H) ppm.
e) 7-Amino-6-chlor-1-cyclohexyl-chinoxalin-2,3-(1H,4H)-dion (e1) und
   7-Amino-1-cyclohexyl-chinoxalin-2,3(1H,4H)-dion (e2)
   28,9 g (89mmol) 6-Chlor-1-cyclohexyl-7-nitro-chinoxalin-2,3(1H,4H)-dion wurden in 300 ml Tetrahydrofuran/Methanol/Dimethylformamid (3:3:1) gelöst und nach Zugabe von 3 g Palladium/Kohle (10 %) hydriert. Der Ansatz wurde filtriert, die Kohle mit methanolischer Ammoniak-Lösung gewaschen und die vereinigten Filtrate im Vakuum eingeengt. Der Rückstand wurde chromatographisch am Kieselgel mit dem Fließmittel Toluol/Aceton/Eisessig (10:10:1) getrennt. Man erhielt 2,2 g (8 %) des Produktes e1 und 18,5 g (80 %) des Produktes e2.
   - e1:: ¹H-NMR (D₆-DMSO):
   δ= 1,1-2,0 (8H); 2,3-2,5 (2H); 4,4 (1H); ca. 5,2 (2H, breit); 7,0 (1H); 7,1 (1H) und ca. 11,5 (breit) ppm.
   - e2:: ¹H-NMR (D₆-DMSO):
   δ= 1,1-2,0 (8H); 2,3-2,5 (2H); 4,4 (1H); ca. 5,1 (2H, breit); 6,4 (1H); 6,8 (1H); 6,9 (1H) und ca. 11,5 (breit) ppm.
f) 6-Chlor-1-cyclohexyl-7-(1-propyl-chinoxalin-2,3-(1H,4H)-dion
   1,9 g (6,3 mmol) 7-Amino-6-chlor-1-cyclohexyl-chinoxalin-2,3-(1H,4H)-dion (36 e1) und 1,1 g (7,9 mmol) 2,5-Dimethoxytetrahydrofuran wurden in 40 ml Eisessig für 2 h unter Rückfluß gekocht. Anschließend wurde der Ansatz auf Eiswasser gegossen und der Niederschlag abgesaugt. Man erhielt 2,0 g (90 %) des Beispiels 36.
   Schmp. 216-218°C (Z.).
   ¹H-NMR (D₆-DMSO):
   δ= 1,1-2,0 (8H); 2,3-2,5 (2H); 4,4 (1H); 6,3 (2H); 7,0 (2H); 7,3 (1H); 7,6 (1H) und 12,0 (1H) ppm.

### Beispiel 37

### 1-Cyclohexyl-6-nitro-7-(1-pyrrolyl)-chinoxalin-2,3(1H,4H)-dion

a) 7-Acetamino-1-cyclohexyl-chinoxalin-2,3(1H,4H)-dion
   18,3 g (70 mmol) 7-Acetamino-1-cyclohexyl-chinoxalin-2,3(1H,4H)-dion (Produkt e2 aus Beispiel 36) wurden in 250 ml Essigsäure gelöst und nachdem man eine Spatelspitze 4-(N,N-Dimethylamino)-pyridin zugesetzt hatte, wurden 7,2 g (70 mmol) Essigsäureanhydrid zugetropft. Man rührte noch 30 Min. bei Raumtemperatur. Anschließend wurde der entstandene Niederschlag abgesaugt und getrocknet. Man erhielt 20,8 g (98 %) des Produktes.
   Schmp. 227-230°C (Z.).
   ¹H-NMR (D₆-DMSO):
   δ= 1,1-1,9 (8H); 2,3-2,5 (2H); 4,5 (1H); 7,1 (1H); 7,3 (1H); 8,0 (1H); 10,0 (1H) und ca. 12,0 (breit) ppm.
b) 7-Amino-1-cyclohexyl-6-nitro-chinoxalin-2,3(1H,4H)-dion
   20,6 g (68 mmol) 7-Acetamino-1-cyclohexyl-chinoxalin-2,3(1H,4H)-dion wurden in 250 ml konzentrierter Schwefelsäure gelöst. Anschließend wurden bei 0-5°C 7,2 g (71 mmol) Kaliumnitrat portionsweise zugegeben. Man rührte danach für 30 Min. bei 0°C und 2 h bei Raumtemperatur. Der Ansatz wurde dann auf 1,5 l Eiswasser gegossen und für 4 h auf dem Wasserbad erwärmt. Der entstandene Niederschlag wurde abgesaugt. Das Filtrat wurde mit weniger Ammoniak-Lösung auf pH=6 eingestellt und mit Methylenchlorid extrahiert. Die organische Phase wurde getrocknet und im Vakuum eingeengt. Dieser Rückstand und der erste Niederschlag wurden vereint. Man erhielt 13,4 g (64 %) des Produktes. Schmp. > 260°C.
   ¹H-NMR (D₆-DMSO):
   δ= 1,1-2,0 (8H); 2,3-2,5 (2H); 4,3 (1H); 7,1 (1H); 7,1-7,4 (breit, 2H); 7,7 (1H) und ca. 11,5 (1H) ppm.
c) 1-Cyclohexyl-6-nitro-7-(1-pyrrolyl)-chinoxalin-2,3(1H,4H)-dion
   2,5 g (8,2 mmol) 7-Amino-1-cyclohexyl-6-nitro-chinoxalin-2,3(1H,4H)-dion und 1,4 g (10,3 mmol) 2,5-Dimethoxytetrahydrofuran wurden in 50 ml Essigsäure 2 h unter Rückfluß gekocht. Danach wurde der Ansatz auf Eiswasser gegossen und der Niederschlag abgesaugt und aus wenig heißem Ethanol kristallisiert. Man erhielt 2,0 g (70 %) des Beispiels 37.
   ¹H-NMR (D₆-DMSO):
   δ= 1,1-1,9 (8H); 2,3-2,5 (2H); 4,5 (1H); 6,25 (2H); 7,0 (2H); 7,6 (1H); 7,8 (1H) und ca. 12 (breit) ppm.

### Beispiel 38

### 1-Cyclohexyl-7-(2-methoxycarbonyl-1-pyrrolyl)-6-nitro-chinoxalin-2,3(1H,4H)-dion

4,5 g (14,8 mmol) 7-Amino-1-cyclohexyl-6-nitro-chinoxalin-2,3(1H,4H)-dion (Produkt 37 b) und 3,5 g (14,8 mmol) 1,3-Dimethoxy-2-tetrahydrofuran-1-yl-carbonsäuremethylester wurden in 75 ml Essigsäure für 2 h unter Rückfluß gekocht. Der Ansatz wurde auf Eiswasser gegossen und der Niederschlag abgesaugt. Man erhielt 4,8 g (78 %) des Produktes.
Schmp. 293°C (Z.).
¹H-NMR (D₆-DMSO):
δ= 1,1-1,9 (8H); 2,3-2,5 (2H); 3,6 (3H); 4,5 (1H); 6,4 (1H); 7,1 (1H); 7,2 (1H); 7,8 (1H); 7,9 (1H) und ca. 12 (breit) ppm.

### Beispiel 39

### 9-Cyclohexyl-7,8(6H,9H)-Dioxopyrazino-[5,6-g]-pyrrolo-[1,2-c]-chinoxalin-2(4H)-on

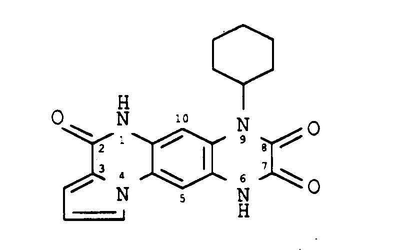

3,15 g (7,6 mmol) 1-Cyclohexyl-7-(2-methoxycarbonyl-1-pyrrolyl)-6-nitro-chinoxalin-2,3-(1H,4H)-dion wurden in 200 ml Essigsäure gelöst und bei 80°C mit 4,3 g (7,6 mmol) Eisenpulver portionsweise versetzt. Man erwärmte noch 2 h unter Rückfluß. Der Ansatz wurde im Vakuum eingeengt und der Rückstand in 2 M Salzsäure dispergiert. Der Niederschlag wurde abgesaugt. Man erhielt 2,4 g (91 %) des Produktes. Schmp. > 300°C.
¹H-NMR (D₆-DMSO):
δ= 1,1-2,0 (8H); 2,3-2,5 (2H); 4,6 (1H); 6,7 (1H); 7,0 (1H); 7,1 (1H); 7,9 (1H); 8,4 (1H); 11,3 (1H) und 12 (1H) ppm.

### Beispiel 40

### 1-(2-Ethyl-1-butyl)-6-nitro-7-(1-pyrrolyl)-chinoxalin-2,3(1H,4H)-dion

a) 4-Chlor-N-(2-ethyl-1-butyl)-2-nitroanilin
   50,0 g (0,26 mol) 2,5-Dichlor-1-nitrobenzol, 71,9 g (0,52 mol) Kaliumkarbonat, 45,6 g (0,455 mol) 2-Ethyl-1-butylamin und 0,5 g (18-crown-6 wurden in 250 ml Dimethylformamid 4 h auf 80°C erwärmt. Anschließend wurde alles auf viel Eiswasser gegossen und der anfallende Niederschlag abgesaugt, getrocknet und am Methanol umkristallisiert. Man erhielt 41,8 g (63 %) des Produktes.
   ¹H-NMR (D₆-DMSO):
   δ= 0,9 (6H); 1,3 (4H); 1,6 (1H); 3,3 (2H); 7,1 (1H); 7,5 (1H); 8,0 (1H) und 8,1 (1H) ppm.
b) 2-Amino-4-chlor-N-(2-ethyl-1-butyl)-anilin
   40 g (0,156 mol) 4-Chlor-N-(2-ethyl-1-butyl)-2-nitroanilin wurden in 500 ml Ethanol gelöst. Die Lösung wurde mit 4 g Raney-Nickel versetzt und bei 1 atm und Raumtemperatur hydriert. Anschließend wurde filtriert und das Filtrat im Vakuum eingeengt. Man erhielt 35 g (98 %) des Produktes.
   ¹H-NMR (D₆-DMSO):
   δ= 0,8 (6H); 1,4 (4H); 1,5 (1H); 2,8 (2H); 4,2 (1H); 4,8 (breit, 2H), 6,3 (1H); 6,4 (1H) und 6,5 (1H) ppm.
c) 6-Chlor-1-(2-ethyl-1-butyl)-chinoxalin-2,3(1H,4H)-dion
   35 g (0,124 mol) 2-Amino-4-chlor-N(2-ethyl-1-butyl)-anilin wurden in 350 ml Oxalsäurediethylester für 2,5 h unter Rückfluß gekocht. Nach dem Abkühlen wurde der Niederschlag abgesaugt und getrocknet. Man erhielt 33 g (76 %) des Produktes. Schmp. 253-255°C.
   ¹H-NMR (D₆-DMSO):
   δ= 0,9 (6H); 1,3 (4H); 1,7 (1H); 4,0 (2H); 7,2 (1H); 7,25 (1H) und 7,3 (1H) ppm.
d) 6-Chlor-1(2-ethyl-1-butyl)-7-nitro-chinoxalin-2,3(1H,4H) -dion
   30 g (0,106 mol) 5-Chlor-1-(2-ethyl-1-butyl)-chinoxalin-2,3(1H,4H)-dion wurden in 350 ml konzentrierter Schwefelsäure gelöst und auf 0°C gekühlt. Man gab danach bei 0°C 10,8 g Kaliumnitrat portionsweise zu und rührte die Reaktionslösung noch 1,5 h. Anschließend wurde alles auf Eiswasser gegossen und der entstandene Niederschlag abgesaugt, mit Wasser gewaschen und getrocknet. Man erhielt 31 g (91 %) des Produktes. Schmp. 232-233°C.
   ¹H-NMR (D₆-DMSO):
   δ= 0,9 (6H); 1,3 (4H); 1,8 (1H); 4,1 (2H); 7,3 (1H); 8,0 (1H) und 12,5 (1H) ppm.
e) 7-Amino-1-(2-ethyl-1-butyl)-chinoxalin-2,3(1H,4H)-dion
   36,0 g (0,112 mol) 6-Chlor-1-(2-ethyl-1-butyl)-7-nitro-chinoxalin-2,3(1H,4H)-dion wurden unter Stickstoff in 500 ml Isopropanol gelöst und mit einer Lösung von 70,6 g (1,12 mol) Ammoniumformiat in 100 ml Wasser versetzt. Nach der Zugabe von 3,5 g Palladium/Kohle (10 %) wurde alles für 1 h bei Rückfluß gekocht. Anschließend wurde filtriert und das Filtrat im Vakuum eingeengt. Der Rückstand wurde zwischen wäßriger Natriumhydrogencarbonat-Lösung und Ether verteilt. Die Ether-Phase wurde abgetrennt, getrocknet und im Vakuum eingeengt.
   Schmp.: 246°C (Z.).
   ¹H-NMR (D₆-DMSO):
   δ= 0,9 (6H); 1,3 (4H); 1,9 (1H); 4,0 (2H); 5,2 (2H); 6,4 (1H); 6,5 (1H) und 6,9 (1H) ppm.
f) 7-Acetamido-1-(2-ethyl-1-butyl)-chinoxalin-2,3(1H,4H) -dion
   29,0 g (0,111 mol) 7-Amino-1-(2-ethyl-1-butyl)-chinoxalin-2,3(1H,4H)-dion wurden in 200 ml Essigsäure gelöst. Nach der Zugabe von einer Spatelspitze 4-N,N-Dimethylamino-pyridin wurden 11,4 g (0,111 mol) Essigsäureanhydrid zugetropft. Man rührte noch 30 Minuten bei Raumtemperatur. Anschließend wurde alles auf Eiswasser gegossen und der anfallende Niederschlag abgesaugt und getrocknet. Man erhielt 22,5 g (67 %) des Produktes.
   Schmp. 168-170°C.
   ¹H-NMR (D₆-DMSO):
   δ= 0,9 (6H); 1,3 (4H); 1,9 (1H); 2,1 (3H); 4,0 (2H); 7,1 (1H); 7,2 (2H); 7,9 (1H); 10,1 (1H) und ca. 11,5 (breit) ppm.
g) 7-Acetamido-1-(2-ethyl-1-butyl)-6-nitro-chinoxalin-2,3(1H,4H)-dion
   21,0 g (69,2 mmol) 7-Acetamido-1-(2-ethyl-1-butyl)-chinoxalin-2,3(1H,4H)dion wurden in 250 ml konzentrierter Schwefelsäure gelöst und bei 0-5°C portionsweise mit 7,0 g (69,2 mmol) Kaliumnitrat versetzt. Man rührte noch 1 h und gab anschließend nochmals 3,5 g (34,6 mmol) Kaliumnitrat zu. Danach rührte man für 16 h bei Raumtemperatur. Anschließend wurde alles auf Eiswasser gegossen und der anfallende Niederschlag abgesaugt und getrocknet. Man erhielt 18,0 g (75 %) des Produktes, das direkt weiterverarbeitet wurde.
h) 7-Amino-1-(2-ethyl-1-butyl)-6-nitro-chinoxalin-2,3(1H,4H)-dion
   18,0 g (51,6 mmol) 7-Acetamido-1-(2-ethyl-1-butyl)-chinoxalin-2,3-dion wurden in einem Gemisch aus 50 ml Ethanol und 300 ml 2 M Salzsäure 1,5 h unter Rückfluß erhitzt. Anschließend wurde die Lösung im Vakuum eingeengt und der Rückstand zwischen Wasser und Essigsäureethylester versetzt. Die organische Phase wurde getrocknet und im Vakuum eingeengt. Man erhielt 13,0 g (82 %) des Produktes.
   ¹H-NMR (D₆-DMSO):
   δ= 0,9 (6H); 1,3 (4H); 1,8 (1H); 3,9 (3H); 6,8 (1H); 7,2-7,6 (breit, 2H); 7,8 (1H) und 11,9 (1H) ppm.
i) 1-(2-Ethyl-1-butyl)-6-nitro-7-(1-pyrrolyl)-chinoxalin-2,3(1H,4H)-dion
   2,5 g (8,2 mmol) 7-Amino-1-(2-ethyl-1-butyl)-6-nitro-chinoxalin-2,3(1H,4H)-dion und 1,1 g (8,2 mmol) 2,5-Dimethoxytetrahydrofuran wurden in 100 ml Essigsäure 1 h unter Rückfluß gekocht. Anschließend wurde alles auf Eiswasser gegossen und der anfallende Niederschlag abgesaugt, mit Wasser gewaschen und getrocknet. Man erhielt 0,8 g (31 %) des Produktes. Schmp. 154-155°C.
   ¹H-NMR (D₆-DMSO):
   δ= 0,8 (6H); 1,3 (4H); 1,8 (1H); 4,1 (2H); 6,3 (2H); 6,9 (2H); 7,3 (1H); 7,8 (1H) und 12,3 (1H) ppm.

### Beispiel 41

### 1-(2-Ethyl-1-butyl)-7-(2-methoxycarbonyl-1-pyrrolyl)-6-nitro-chinoxalin-2,3(1H,4H)-dion

3,0 g (9,8 mmol) 7-Amino-1-(2-ethyl-1-butyl)-6-nitro-chinoxalin-2,3-(1H,4H)-dion (Beispiel 40 h) und 1,8 g (9,8 mmol) (2,5-Dimethoxy-tetrahydrofuran-2-yl)carbonsäuremethylester wurden in 150 ml konzentrierter Essigsäure für 1 h unter Rückfluß gekocht. Anschließend wurde der Ansatz auf Eiswasser gegossen und der anfallende Niederschlag abgesaugt. Man erhielt 1,1 g (28 %) des Produktes. Schmp. 240-241°C.
¹H-NMR (D₆-DMSO):
δ= 0,8 (6H); 1,3 (4H); 1,8 (1H); 3,6 (3H); 4,2 (2H); 6,4 (1H); 7,1 (1H); 7,3 (1H); 7,4 (1H); 8,0 (1H) und ca. 12,5 (1H) ppm.

### Beispiel 42

### 1-Cyclopropyl-6-nitro-7-(1-pyrrolyl)chinoxalin-2,3-(1H,4H) -dion

2,5-Dibrom-1-nitrobenzol und Cyclopropylamin wurden analog zu Beispiel 40 (a-i) umgesetzt.
¹H-NMR (D₆-DMSO):
δ= 0,8 (2H); 1,3 (2H); 3,0 (1H); 6,3 (2H); 6,9 (2H); 7,6 (1H); 7,8 (1H) und 12,5 (1H) ppm.

### Beispiel 43

### 7-Brom-9-(2,5-dimethyl-1-pyrrolyl)-benzo[f]chinoxalin-2,3-[1H,4H]-dion

a) 6-Brom-2-methoxy-1-nitronaphthalin
   Zu 50 g (0,21 Mol) 6-Brom-2-methoxynaphthalin, gelöst in 350 ml Essigsäure, wurden bei Raumtemperatur 10 ml 98 %ige Salpetersäure, gelöst in 70 ml Essigsäure, getropft. Das Reaktionsgemisch wurde auf 50°C erwärmt und nachdem das Produkt auskristallisiert war, abgekühlt. Der Niederschlag wurde abgesaugt. Man erhielt 53,1 g (90 %) des Produktes. Schmp. 156-157°C.
   ¹H-NMR (D₆-DMSO):
   δ= 4,1 (3H); 7,5 (1H); 7,7-7,8 (2H); 8,2 (1H) und 8,3 (1H) ppm.
b) 6-Brom-1-nitro-2-naphthylamin
   10 g (35,4 mmol) des Produktes 43 a wurden in 100 ml Dimethylformamid in Gegenwart von 0,8 Mol Ammoniak in einem Autoklaven 24 h auf 100°C erhitzt. Anschließend wurde der Ansatz im Vakuum eingeengt, der Niederschlag in Wasser dispergiert und abgesaugt. Man erhielt 9,3 g (98 %) des Produktes. Schmp. > 175°C /Z.).
   ¹H-NMR (D₆-DMSO):
   δ= 7,2 (1H); 7,7 (1H); 7,8 (1H); 8,0 (1H); 8,05 (2H) und 8,5 (1H) ppm.
c) 2-Amino-6-Brom-1-naphthylamin
   9 g (33,7 mmol) des Produktes 43 b wurden in 250 ml Ethanol gelöst und nach Zugabe von 2 g Raney-Nickel hydriert. Anschließend wurde filtriert und das Filtrat im Vakuum eingeengt. Man erhielt 7,4 g (92 %) des Produktes.
   Schmp. 159-160°C (Z.).
   ¹H-NMR (D₆-DMSO):
   δ= ca. 5 (4H, breit); 7,0 (2H); 7,3 (1H) und 7,8-7,9 (2H) ppm.
d) 7-Brom-benzo[f]chinoxalin-2,3-(1H,4H)-dion
   7 g (29,5 mmol) des Produktes 43 c wurden in 300 ml Oxalsäurediethylester 3 h unter Rückfluß gekocht. Nach dem Abkühlen wurde der Niederschlag abgesaugt, mit Ethanol gewaschen und getrocknet. Man erhielt 5,9 (69 %) des Produktes.
   ¹H-NMR (D₆-DMSO):
   δ= 7,4 (1H); 7,7 (2H); 8,2 (1H); 8,5 (1H) und 12,3 (2H) ppm.
e) 7-Brom-9-nitro-benzo[f]chinoxalin-2,3-(1H,4H)-dion
   5,5 g (18,9 mmol) des Produktes 43 d wurden in 150 ml Essigsäure suspendiert. 25 ml 65 %ige Salpetersäure wurden bei Raumtemperatur zugetropft und danach der Ansatz 30 Minuten auf 100°C erwärmt. Nach dem Abkühlen wurde der Niederschlag abgesaugt. Man erhielt 4,7 g (74 %) des Produktes.
   ¹H-NMR (D₆-DMSO):
   δ= 7,65 (1H); 8,4 (1H); 8,75 (1H); 9,0 (1H) und 11,8 (breit) ppm.
f) 9-Amino-7-Brom-9-benzo[f]chinoxalin-2,3-(1H,4H)-dion
   4,4 g (13,1 mmol) des Produktes 43 e wurden in einem Gemisch aus 100 ml Methanol und 200 ml Tetrahydrofuran gelöst und nach Zugabe von 1 g Raney-Nickel hydriert. Anschließend wurde filtriert und das Filtrat im Vakuum eingeengt. Man erhielt 3,7 g (93 %) des Produktes.
   Schmp. > 260°C.
   ¹H-NMR (D₆-DMSO):
   δ= ca. 5,9 (2H, breit); 6,6 (1H); 7,6 (1H); 8,0 (1H); 8,3 (1H) und ca. 12 (breit) ppm.
g) 7-Brom-9-(2,5-dimethyl-1-pyrrolyl)-benzo[f]chinoxalin-2,3-(1H,4H)-dion
   3,1 g (10,1 mmol) des Produktes 43 f und 1,2 ml (10,1 mmol) 2,5-Hexandion wurden in 150 ml Essigsäure für 30 Minuten unter Rückfluß gekocht. Anschließend wurde der Ansatz im Vakuum eingeengt, der Rückstand in Wasser dispergiert und abgesaugt. Man erhielt 2,8 g (75 %) des Produktes. Schmp. > 270°C.
   ¹H-NMR (D₆-DMSO):
   δ= 1,8 (6H); 5,9 (2H); 6,9 (2H); 7,3 (1H); 7,8 (1H); 8,6 (1H) und 12,2 (breit) ppm.

### Beispiel 44

### 7-Chlor-6-(2,5-dimethyl-1-pyrrolyl)-1-(methoxycarbonyl-methyl)-chinoxalin-2,3-(1H,4H)dion

a) N-(5-Chlor-2-nitro-phenyl)glycin
   25 g (0,13 Mol) 2,4-Dichlor-1-nitrobenzol, 19,5 g (0,26 Mol) Glycin und 18 g (0,13 Mol) Kaliumkarbonat wurden in 200 ml Dimethylformamid 3 h auf 120°C erhitzt. Anschließend wurde alles auf Eiswasser gegossen, mit 1 M Salzsäure angesäuert und mit Essigsäureethylester extrahiert. Die organische Phase wurde getrocknet und im Vakuum eingeengt. Der Rückstand wurde chromatographisch an Kieselgel (Fließmittel:Toluol/Aceton/Essigsäure=20:10:1) gereinigt. Man erhielt 19,7 g (66 %) des Produktes. Schmp. 147-148°C.
   ¹H-NMR (D₆-DMSO):
   δ= 4,2 (2H); 6,7 (1H); 7,0 (1H); 8,1 (1H); 8,4 (1H) und ca. 12,5 (breit) ppm.
b) N-(2-Amino-5-chlor-phenyl)glycin
   19,5 g (84,6 mmol) N-(5-chlor-2-nitro-phenyl)glycin wurden in 250 ml Ethanol gelöst, mti 5 g Raney-Nickel versetzt und bei 45-50°C und 1 bar hydriert. Anschließend wurde filtriert und das Filtrat im Vakuum einrotiert. Man erhielt 15,6 g (93 %) des Produktes.
   ¹H-NMR (D₆-DMSO):
   δ= 3,7 (2H); 6,2 (1H); 6,5-6,8 (3H) und 10,2 (1H) ppm.
c) 1-(Carboxymethyl)-7-chlor-chinoxalin-2,3-(1H,4H)-dion
   15 g (75 mmol) N-(2-Amino-5-chlorphenyl)glycin und 21 ml (150 mmol) Triethylamin wurden in 250 ml wasserfreiem Tetrahydrofuran gelöst. Bei 0°C wurde eine Lösung aus 18,2 ml (16,4 mmol) Oxalsäuremonoethylesterchlorid und 30 ml wasserfreim Tetrahydrofuran zugetropft. Man rührte eine Stunde bei 0°C und weitere zwei bei Raumtemperatur. Anschließend wurde der Ansatz im Vakuum eingeengt und der Rückstand zwischen Wasser und Methylenchlorid verteilt. Die organische Phase wurde getrocknet und im Vakuum eingeengt, der Rückstand aus wenig Ethanol kristallisiert. Dieses Produkt wurde in einem Gemisch aus 200 ml Ethanol und 250 ml konzentrierter Salzsäure 1 h unter Rückfluß gekocht. Anschließend wurde alles auf Eis gegossen und der Niederschlag abgesaugt. Man erhielt 13,7 g (93 % des Produktes). Schmp. > 285°C.
   ¹H-NMR (D₆-DMSO):
   δ= 4,9 (2H); 7,0-7,3 (2H); 7,4 (1H); 12,3 (1H) und ca. 13 (breit) ppm.
d) 1-Carboxymethyl-7-chlor-6-nitro-chinoxalin-2,3-(1H,4H)-dion
   13,0 g (51 mmol) 1-(Carboxymethyl)-7-chlor-chinoxalin-2,3-(1H,4H)-dion wurden in 150 ml konzentrierter Schwefelsäure gelöst und bei 0°C 4,3 g (51 mmol) Natriumnitrat portionsweise zugefügt. Es wurde 2 h bei Raumtemperatur gerührt. Anschließend wurde auf Eiswasser gegossen und der anfallende Niederschlag abgesaugt. Man erhielt 10,2 g (67 %) des Produktes. Schmp. > 280°C.
   ¹H-NMR (D₆-DMSO):
   δ= 5,0 (2H); 7,8 (1H); 7,9 (1H) und 12,5 (1H) ppm.
e) 7-Chlor-1-(methoxycarbonylmethyl)-6-nitro-chinoxalin-2,3-(1H,4H)-dion
   10 g (33,4 mmol) 1-Carboxymethyl-7-chlor-chinoxalin-2,3-(1H,4H)-dion wurden in einem Gemisch aus 2 ml konzentrierter Schwefelsäure und 200 ml Ethanol für 1 h unter Rückfluß gekocht. Nach dem Abkühlen wurde der Niederschlag abgesaugt. Man erhielt 11,5 g des Produktes. Schmp. > 260°C.
   ¹H-NMR (D₆-DMSO):
   δ= 1,25 (3H); 4,2 (2H); 5,0 (2H); 7,8 (1H); 7,9 (1H) und 12,5 (1H) ppm.
f) 6-Amino-7-chlor-(1-methoxycarbonylmethyl)-chinoxalin-2,3-(1H,4H)-dion
   10 g (30,5 mmol) 7-Chlor-(1-methoxycarbonylmethyl)-6-nitro-chinoxalin-2,3-(1H,4H)-dion wurden in 300 ml Dimethylformamid gelöst und nach Zugabe von 5 g Raney-Nickel bei 1 bar und Raumtemperatur hydriert. Anschließend wurde filtriert und das Filtrat im Vakuum eingeengt. Man erhielt 8,6 g (95 %) des Produktes. Schmp. > 260°C.
   ¹H-NMR (D₆-DMSO):
   δ= 1,2 (3H); 4,2 (2H); 4,85 (2H); 5,45 (2H); 6,6 (1H); 7,25 (1H) und 12 (1H) ppm.
g) 7-Chlor-6-(2,5-dimethyl-1-pyrrolyl)-1-(methoxycarbonylmethyl)-chinoxalin-2,3-(1H,4H)-dion
   6,0 g (20,2 mmol) 6-Amino-7-chlor-(1-methoxy-carbonylmethyl)-chinoxalin-2,3-(1H,4H)-dion und 2,4 ml (20,2 mmol) Hexan-2,5-dion wurden in 75 ml konzentriertem Eisessig für 0,5 h unter Rückfluß gekocht. Danach wurde alles auf Eiswasser gegossen und der Niederschlag abgesaugt. Man erhielt 5,5 g (74 %) des Produktes.
   ¹H-NMR (D₆-DMSO):
   δ= 1,2 (3H); 1,9 (6H); 4,2 (2H); 5,0 (2H); 5,8 (2H); 7,1 (1H); 7,8 (1H) und 12,3 (breit) ppm.

### Beispiel 45

### 1-(Carboxymethyl)-7-chlor-6-(2,5-dimethyl-1-pyrrolyl)-chinoxalin-2,3-(1H,4H)-dion

4,0 g (10,6 mmol) 7-Chlor-6-(2,5-dimethyl-1-pyrrolyl)-1-(methoxycarbonylmethyl)-chinoxalin-2,3-(1H,4H)-dion (Beispiel 44) wurden in 100 ml Tetrahydrofuran gelöst und mit einer Lösung aus 0,76 g (31,9 mmol) Lithiumhydroxid in 15 ml Wasser versetzt. Man rührte 2 h bei Raumtemperatur. Anschließend wurde das organische Lösungsmittel im Vakuum entfernt und die wäßrige Phase mit 1 M Salzsäure angesäuert. Der anfallende Niederschlag wurde abgesaugt. Man erhielt 3,1 g (84 %) des Produktes. Schmp. > 265°C.
¹H-NMR (D₆-DMSO):
δ= 1,9 (6H); 4,9 (2H); 5,8 (2H); 7,1 (1H); 7,7 (1H) und 12,5 (1H) ppm.

### Beispiel 46

### 2-(7-Chlor-6-(2,5-dimethyl-1-pyrrolyl)-chinoxalin-2,3-(1H,4H)-dion-1-yl)essigsäurebenzylamid

1,3 g (3,7 mmol) 1-(Carboxymethyl)-7-chlor-6-(2,5-dimethyl-1-pyrrolyl)-chinoxalin-2,3-(1H,4H)-dion und 1,6 ml (12,3 mmol) Benzylamin wurden in 30 ml Dimethylformamid gelöst, und bei 0°C wurde eine Lösung aus 0,89 ml (4,1 mmol) Phosphorsäurediphenylesterazid und 10 ml Dimethylformamid zugetropft. Alles wurde 16 h bei Raumtemperatur gerührt. Anschließend wurde auf Eiswasser gegossen und die wäßrige Phase mit Essigsäureethylester extrahiert. Die organische Phase wurde getrocknet und im Vakuum eingeengt. Der anfallende Rückstand wurde chromatographisch an Kieselgel (Fließmittel: Toluol/Aceton= 1/1) gereinigt. Man erhielt 1,1 g (67 %) des Produktes. Schmp. 244°C.
¹H-NMR (D₆-DMSO):
δ= 1,8 (6H); 4,3 (2H); 4,9 (2H); 5,8 (2H); 7,1 (1H); 7,2-7,4 (5H); 7,5 (1H); 8,7 (1H) und 12,5 (1H) ppm.

### Beispiel 47

### 1-(Carboxymethyl)-9-(2,5-dimethyl-1-pyrrolyl)-benzo[f]chinoxalin-2,3-(1H,4H)-dion

13,0 g (33,2 mmol) des Beispiels 31 wurden in 200 ml Tetrahydrofuran gelöst und mit einer Lösung aus 2,4 g (99,6 mmol) Lithiumhydroxid in 100 ml Wasser versetzt. Alles wurde 2 h bei Raumtemperatur gerührt. Anschließend wurde das Tetrahydrofuran im Vakuum entfernt und die wäßrige Phase durch Zugabe von 11 ml konzentrierter Salzsäure angesäuert. Der ausgefallene Niederschlag wurde abgesaugt. Man erhielt 10,7 g (89 %) des Produktes.
Schmp. 236-237°C (Z.).
¹H-NMR (D₆-DMSO):
δ= 1,8 (6H); 5,1 (2H); 5,9 (2H); 6,9 (1H); 7,6 (1H); 7,8 (2H); 8,7 (1H); 12,5 (1H) und ca. 13,5 (breit) ppm.

### Beispiel 48

### 2-[9-(2,5-Dimethyl-1-pyrrolyl)-benzo[f]chinoxalin-2,3-(1H,4H)-dion-1-yl]essigsäurebenzylamid

1,5 g (4,1 mmol) des Beispiels 47 und 0,5 ml (4,5 mmol) Benzylamin wurden in 30 ml wasserfreiem Dimethylformamid gelöst. Bei 0°C tropfte man nacheinander 1 ml (4,5 mmol) Phosphorsäurediphenylesterazid, gelöst in 10 ml Dimethylformamid, und 1,25 ml (9,1 mmol) Triethylamin zu. Danach wurde noch 3 h bei Raumtemperatur gerührt. Anschließend wurde die Reaktionslösung zwischen 2 M Salzsäure und Essigsäureethylester verteilt. Die organische Phase wurde getrocknet und im Vakuum eingeengt. Der anfallende Rückstand wurde noch aus Ethanol/Tetrahydrofuran neutralisiert.
Ausbeute: 1,25 g (67 %); Schmp. 243-244°C.
¹H-NMR (D₆-DMSO):
δ= 1,8 (6H); 4,3 (2H); 5,1 (2H); 5,9 (2H); 6,9 (1H); 7,1-7,4 (5H); 7,5-7,8 (3H); 8,7 (2H) und 12,5 (1H) ppm.

### Beispiel 49

### 2-[9-(2,5-Dimethyl-1-pyrrolyl)-benzo[f]chinoxalin-2,3-(1H,4H)-dion-1-yl]essigsäurephenylamid

1,15 g (3,2 mmol) des Beispiels 47 und 0,32 ml (3,5 mmol) Anilin wurden analog Beispiel 48 umgesetzt. Das so erhaltene Produkt wurde chromatographisch an Kieselgel (Fließmittel: Toluol/Aceton/Essigsäure = 20:10:1) gereinigt. Ausbeute: 0,37 g (27 %).
Schmp. > 260°C.
¹H-NMR (D₆-DMSO):
δ= 1,8 (6H); 5,5 (2H); 6,0 (2H); 7,1 (2H); 7,3 (2H); 7,5 (2H); 7,8 (1H); 8,0 (1H); 8,2 (1H); 9,9 (1H) und 10,4 (1H) ppm.

### Beispiel 50

### 1-(Benzyloxycarbonylmethyl)-9-(2,5-dimethyl-1-pyrrolyl)-benzo[f]chinoxalin-2,3-(1H,4H)-dion

1,3 g (3,6 mmol) des Beispiels 47 und 0,41 ml (3,9 mmol) Benzylalkohol wurden analog Beispiel 48 umgesetzt. Das so erhaltene Produkt wurde chromatographisch am Kieselgel (Fließmittel: Toluol/Aceton/Essigsäure = 10:10:1) gereinigt. Ausbeute: 1,0 g (63 %).
¹H-NMR (D₆-DMSO):
δ= 1,8 (6H); 5,2 (2H); 5,3 (2H); 5,9 (2H); 6,9 (1H); 7,3 (5H); 7,5 (1H); 7,7 (1H); 7,75 (1H); 8,8 (1H) und ca. 12,5 (breit) ppm.

### Beispiel 51

### 2-[9-(2,5-Dimethyl-1-pyrrolyl)-benzo[f]chinoxalin-2,3-(1H,4H)-dion-1-yl]essigsäureethylamid

0,8 g (2,0 mmol) des Beispiels 31 wurden in 200 ml einer 10 %igen ethanolischen Ethylamin-Lösung für 40 h unter Rückfluß gekocht. Danach wurde im Vakuum eingeengt und der Rückstand chromatographisch an Kieselgel (Fließmittel: Toluol/Aceton/Essigsäure = 20:10:1) gereinigt. Ausbeute: 0,41 g (52 %). Schmp. > 285°C.
¹H-NMR (D₆-DMSO):
δ= 0,95 (3H); 1,8 (6H); 3,1 (2H); 4,9 (2H); 5,9 (2H); 6,9 (1H); 7,4 (1H); 7,55 (1H); 7,7 (1H); 8,2 (1H); 8,8 (1H) und 12,4 (breit) ppm.

### Beispiel 52

### 1-(Ethoxycarbonylmethyl)-6-nitro-7-(1-pyrrolyl)-chinoxalin-2,3(1H,4H)-dion

a) N-(4-Chlor-2-nitro-phenyl)-glycin
   26,2 g (0,137 Mol) 2,5-Dichlor-1-nitrobenzol, 20,6 g (0,274 Mol) Glycin und 18,9 g (0,137 Mol) Kaliumkarbonat wurden in 200 ml Diethylenglykol für 1 h bei 120°C erwärmt. Nach dem Abkühlen wurden 100 ml Wasser zugegeben und die Lösung mit 1 M Salzsäure angesäuert. Der Niederschlag wurde abgesaugt. Man erhielt 17,1 g (54 %) des Produktes.
   ¹H-NMR (D₆-DMSO):
   δ= 4,2 (2H); 6,9 (1H); 7,5 (1H); 8,1 (1H); 8,4 (1H) und ca. 13 (breit) ppm.
b) N-(4-Chlor-2-nitrophenyl)aminoessigsäureethylester
   87,1 g (0,38 Mol) N-(4-Chlor-2-nitrophenyl)glycin wurden in 500 ml 10 %iger ethanolischer Schwefelsäure suspendiert und auf 80°C erwärmt. Die entstandene klare Lösung goß man auf 1,5 l Eiswasser und neutralisierte anschließend die Lösung mit konzentrierter Ammoniak-Lösung und Natriumhydrogenkarbonat-Lösung. Der Niederschlag wurde abgesaugt. Man erhielt 84,4 g (89 %) des Produktes.
   ¹H-NMR (D₆-DMSO):
   δ= 1,2 (3H); 4,2 (2H); 4,3 (2H); 7,0 (1H); 7,5 (1H); 8,0 (1H) und 8,4 (1H) ppm.
c) N-(4-Chlor-2-nitrophenyl)-N-(ethoxycarbonylmethyl)-oxalsäuremonoethylesteramid
   86,1 g (0,33 Mol) der Verbindung von Bsp. 52 b wurden in 350 ml Pyridin gelöst und bei Raumtemperatur 67,6 g (0,495 Mol) Oxalsäuremonoethylesterchlorid zugetropft. Alles wurde 16 h bei Raumtemperatur gerührt. Anschließend wurde der Ansatz auf Eiswasser gegossen und mit 4 M Salzsäure angesäuert. Der Niederschlag wurde abfiltriert und man erhielt 120 g verunreinigtes Produkt.
   ¹H-NMR (D₆-DMSO):
   δ= 1,1-1,3 (6H); 4,0 (2H); 4,2 (2H); 4,5 (1H); 4,6 (1H); 7,7 (1H); 8,0 (1H) und 8,3 (1H) ppm.
d) 6-Chlor-1-(ethoxycarbonylmethyl)-chinoxalin-2,3-(1H,4H)-dion
   101,3 g (0,28 Mol) N-(4-Chlor-2-nitrophenyl)-N-(ethoxycarbonylmethyl)-oxalsäuremonoethylesteramid wurden in 1 l Essigsäure gelöst und auf 80°C erwärmt. Man gab danach portionsweise 15,8 g (0,28 Mol) Eisenpulver zu. Nach 2 h wurden erneut 15,8 g (0,28 Mol) Eisenpulver zugegeben. Eine halbe Stunde später wurde der Ansatz auf Eiswasser gegossen und mit 4 M Salzsäure sauer gestellt. Der Niederschlag wurde abgesaugt und man erhielt 75,7 g (95 %) des Produktes.
   ¹H-NMR (D₆-DMSO):
   δ= 1,2 (3H); 4,2 (2H); 5,0 (2H); 7,2 (2H); 7,3 (1H) und 12,3 (1H) ppm.
e) 6-Chlor-1-(ethoxycarbonylmethyl)-7-nitro-chinoxalin-2,3-(1H,4H)-dion
   69,8 g (0,25 Mol) 6-Chlor-1-(ethoxycarbonylmethyl)-chinoxalin-2,3-(1H,4H)dion wurden in 625 ml konzentrierter Schwefelsäure gelöst und bei 0°C wurden 25 g (0,25 Mol) Kaliumnitrat portionsweise zugegeben. Danach wurde die Kühlung entfernt und der Ansatz weiterhin gerührt. Nach vollständigem Umsatz wurde alles auf Eiswasser gegossen und der Niederschlag abgesaugt. Man erhielt 77,8 g (95 %) des Produktes.
   ¹H-NMR (D₆-DMSO):
   δ= 1,3 (3H); 4,2 (2H); 5,0 (2H); 7,3 (1H); 8,2 (1H) und 12,5 (1H) ppm.
f) 7-Amino-1-(ethoxycarbonylmethyl)-chinoxalin-2,3-(1H,4H)-dion
   84,3 g (0,26 Mol) 6-Chlor-1-(ethoxycarbonylmethyl)-7-nitro-chinoxalin-2,3-(1H,4H)-dion wurden in 1,5 l Isopropanol suspendiert und nacheinander 194,2 g (3,1 Mol) Ammoniumformiat, gelöst in 500 ml Wasser, und 8,5 g Palladium/Kohle (10 %ig) zugegeben. Alles wurde für 4 h auf 75°C erwärmt. Nach dem Abkühlen wurde filtriert und der Filterkuchen dreimal mit 800 ml Dimethylformamid extrahiert. Die vereinigten Dimethylformamidphasen wurden im Vakuum eingeengt und der Rückstand mit Wasser gewaschen. Man erhielt 53,2 g (79 %) des Produktes.
   ¹H-NMR (D₆-DMSO):
   δ= 1,2 (3H); 4,2 (2H); 4,8 (2H); 5,2 (breit, 2H); 6,4 (1H); 6,5 (1H); 6,9 (1H) und 12 (1H) ppm.
g) 7-Acetamido-1-(ethoxycarbonylmethyl)-chinoxalin-2,3-(1H,4H)-dion
   52,95 g (0,2 Mol) 7-Amino-1-(ethoxycarbonylmethyl)-chinoxalin-2,3-(1H,4H)-dion und eine Spatelspitze 4-(N,N-Dimethylamino)-pyridin wurden in einem Gemisch aus 500 ml Eisessig und 300 ml Tetrahydrofuran suspendiert und auf 50°C erwärmt. Danach wurden 20,5 g (0,2 Mol) Essigsäureanhydrid zugetropft und alles für 2 h auf 50°C erwärmt. Der Niederschlag wurde abgesaugt. Man erhielt 57,8 g (94 %) des Produktes.
   ¹H-NMR (D₆-DMSO):
   δ= 1,2 (3H); 2,05 (3H); 4,2 (2H); 4,9 (2H); 7,1 (1H); 7,3 (1H); 7,55 (1H); 10,0 (1H) und 12 (1H) ppm.
h) 7-Amino-1-(carboxymethyl)-6-nitro-chinoxalin-2,3-(1H,4H)-dion
   57,5 g (0,19 Mol) 7-Acetamido-1-(ethoxycarbonylmethyl)-chinoxalin-2,3-(1H,4H)-dion wurden in 575 ml konzentrierter Schwefelsäure gelöst. Die Lösung wurde auf 0°C gekühlt, und es wurden 19,0 g (0,19 Mol) Kaliumnitrat portionsweise zugegeben. Anschließend wurde die Kühlung entfernt und alles gerührt. Nach vollständigem Umsatz wurde der Ansatz in 2 l Eiswasser gegossen und alles 2 h auf dem Wasserbad erhitzt. Danach wurde der pH mit wäßriger Ammoniak-Lösung auf ca. 4-5 eingestellt und der Niederschlag abgesaugt. Das Filtrat wurde im Vakuum eingeengt und der Rückstand chromatographisch an Kieselgel (Laufmittel: Methanol; Tetrahydrofuran:Wasser = 5:4:1/+ 2,5 % Eisessig) gereinigt. Das so erhaltene Produkt wurde mit dem ersten Niederschlag vereinigt. Man erhielt 36,9 g (70 %) Produkt.
   ¹H-NMR (D₆-DMSO):
   δ= 4,5 (2H); 6,7 (1H); 7,3 (2H) und 7,7 (1H) ppm.
i) 7-Amino-1-(ethoxycarbonylmethyl)-6-nitro-chinoxalin-2,3-(1H,4H)-dion
   30,7 g (0,11 Mol) 7-Amino-1-(ethoxycarbonylmethyl)-6-nitro-chinoxalin-2,3-(1H,4H)-dion wurden in 500 ml 10 %iger ethanolischer Schwefelsäure suspendiert und alles 2 h unter Rückfluß erhitzt. Der Ansatz wurde danach auf Eiswasser gegossen und der entstandene Niederschlag abgesaugt. Man erhielt 29,3 g (87 %) des Produktes.
   ¹H-NMR (D₆-DMSO):
   δ= 1,2 (3H); 4,2 (2H); 4,8 (2H); 6,7 (1H); 7,0-7,6 (breit, 2H); 7,8 (1H) und 12,0 (1H) ppm.
h) 1-(Ethoxycarbonylmethyl)-6-nitro-7-(1-pyrrolyl).chinoxalin-2,3-(1H,4H)-dion
   30 g (97 mmol) 7-Amino-1-(ethoxycarbonylmethyl)-6-nitro-chinoxalin-2,3-(1H,4H)-dion und 16,1 g (121,5 mmol) 2,5-Dimethoxytetrahydrofuran wurden in 500 ml Essigsäure 1 h bei 80°C erwärmt. Danach wurde der Ansatz auf Eiswasser gegossen und der Niederschlag abgesaugt. Man erhielt 28,8 g (83 %) des Produktes.
   ¹H-NMR (D₆-DMSO):
   δ= 1,2 (3H); 4,2 (2H); 5,1 (2H); 6,2 (2H); 6,9 (2H); 7,5 (1H); 7,8 (1H) und 12,5 (1H) ppm.

### Beispiel 53

### 1-(Carboxymethyl)-6-nitro-7-(1-pyrrolyl)-chinoxalin-2,3-(1H,4H)-dion

24,5 g (68 mmol) 1-(Ethoxycarbonylmethyl)-6-nitro-7-(1-pyrrolyl)-chinoxalin-2,3-(1H,4H)-dion (Beispiel 52) wurden in 500 ml Tetrahydrofuran gelöst und mit einer Lösung von 6,6 (270 mmol) Lithiumhydroxid in 100 ml Wasser versetzt. Alles wurde für 16 h bei Raumtemperatur gerührt. Anschließend wurde das Tetrahydrofuran im Vakuum entfernt und die wäßrige Phase filtriert. Das Filtrat wurde mit 1 M Salzsäure angesäuert und der entstandene Niederschlag abgesaugt. Man erhielt 17,5 g (77 %) des Produktes.
¹H-NMR (D₆-DMSO):
δ= 5,0 (2H); 6,2 (2H); 6,9 (2H); 7,5 (1H); 7,9 (1H); 12,5 (1H) und 13,5 (breit) ppm.

### Beispiel 54

### 1-(2-Ethoxycarbonylethyl)-7-(1-pyrrolyl)-6-trifluormethyl-chinoxalin-2,3-(1H,4H)-dion

a) 3-N-(2-Nitro-4-trifluormethylphenyl)amino-propionsäure
   60 g (0,27 Mol) 2-Chlor-1-nitro-5-trifluormethylbenzol, 50 g (0,56 Mol) β-Alanin und 41 g (0,3 Mol) Kaliumcarbonat wurden in einem Gemisch aus 300 ml Dimethylformamid und 50 ml Wasser für 6 h unter Rückfluß gekocht. Danach wurde alles auf Eiswasser gegossen, die wäßrige Phase mit Salzsäure angesäuert und der anfallende Niederschlag abgesaugt. Man erhielt 73 g (99 %) des Produktes.
   ¹H-NMR (D₆-DMSO):
   δ= 2,7 (2H); ca. 3,4 (breit); 3,7 (2H); 7,3 (1H); 7,8 (1H); 8,2 (1H); 8,6 (1H) und ca. 12,5 (breit) ppm.
b) 3-N-(2-Nitro-4-trifluormethylphenyl)amino-propionsäureethylester
   73 g (0,26 Mol) des Produktes 54 a wurden in 500 ml 10 %iger ethanolischer Schwefelsäure suspendiert und, bis eine klare Lösung entstand, auf 80°C erhitzt. Anschließend wurde die Lösung auf Eiswasser gegossen, mit wäßrigen Ammoniak- und Natriumhydrogenkarbonat-Lösungen neutralisiert und der anfallende Niederschlag abgesaugt. Man erhielt 71,2 g (89 %) des Produktes.
   ¹H-NMR (D₆-DMSO):
   δ= 1,2 (3H); 2,7 (2H); 3,7 (2H); 4,1 (2H); 7,3 (1H); 7,8 (1H); 8,3 (1H) und 8,5 (1H) ppm.
c) N-(2-Ethoxycarbonylethyl)-N-(2-nitro-4-trifluormethylphenyl)oxalsäuremonoethylesteramid
   40 g (0,13 Mol) des Produktes 54 b wurden in 200 ml Pyridin gelöst und bei Raumtemperatur mit 19,6 g (0,14 Mol) Oxalsäuremonoethylesterchlorid tropfenweise versetzt. Man rührte für 7 h und gab weitere 19,6 g (0,14 Mol) Oxalsäuremonoethylesterchlorid zu. Nach 16 stündigem Rühren gab man vorsichtig 30 ml Wasser zu und engte alles im Vakuum ein. Der Rückstand wurde zwischen verdünnter Salzsäure und Essigsäureethylester verteilt und die organische Phase getrocknet und im Vakuum eingeengt. Man erhielt 56 g verunreinigtes Produkt.
   ¹H-NMR (D₆-DMSO):
   δ= 0,9-1,4 (6H); 2,7 (2H); 3,8-4,3 (6H); 7,9 (1H); 8,3 (1H) und 8,5 (1H) ppm.
d) 1-(2-EThoxycarbonylethyl)-6-trifluormethylchinoxalin-2,3-(1H,4H)-dion
   55 g (0,135 Mol) des Produktes 54c wurden in 500 ml Essigsäure gelöst und auf 80°C erwärmt. Danach wurden 15,4 g (0,275 Mol) Eisen-Pulver portionsweise zugegeben und der Ansatz für 2-3 h auf 100°C erwärmt. Anschließend wurde alles auf Eiswasser gegossen, die wäßrige Phase mit Salzsäure angesäuert und der anfallende Niederschlag abgesaugt. Dieser Rückstand wurde mit wenig Ethanol behandelt und erneut abgesaugt. Man erhielt 30,3 g (67 %) des Produktes.
   ¹H-NMR(D₆-DMSO):
   δ= 1,2 (3H), 2,7 (2H), 4,1 (2H), 4,4 (2H), 7,5 (1H), 7,55 (1H), 7,65 (1H) und ca. 12,3 (1H) ppm.
e) 1-(2-Ethoxycarbonylethyl)-7-nitro-6-trifluormethylchinoxalin-2,3-(1H,4H)-dion
   26 g (78 mmol) des Produktes 54 d wurden in 250 ml konzentrierter Schwefelsäure gelöst und bei 0°C portionsweise 9,5 g (94 mmol) Kaliumnitrat zugefügt. Danach wurde noch 2 h bei Raumtemperatur gerührt. Anschließend wurde alles auf Eiswasser gegossen und der entstandene Niederschlag abgesaugt. Man erhielt 27,7 g (94 %) des Produktes.
   ¹H-NMR(D₆-DMSO):
   δ= 1,2 (3H), 2,7 (2H), 4,1 (2H), 4,4 (2H), 7,6 (1H), 8,25 (1H) und ca. 12,5 (1H) ppm.
f) 7-Amino-1-(2-ethoxycarbonylethyl)-6-trifluormethyl-chinoxalin-2,3-(1H,4H)-dion
   26 g (69 mmol) des Produktes 54 e wurden in einem Gemisch aus 500 ml Tetrahydrofuran und 100 ml Ethanol in der Siedehitze gelöst. Diese Lösung wurde langsam zu einer siedenden Lösung von 54 g (310 mmol) Natriumdithionit in 600 ml Wasser getropft. Man kochte die Lösung noch ca. 15 Minuten und entfernte danach das organische Lösungsmittel im Vakuum. Die wäßrige Phase wurde mehrmals mit Essigsäureethylester extrahiert, die vereinigten organischen Phasen getrocknet und im Vakuum eingeengt. Man erhielt 12,7 g (53 %) des Produktes.
   Schmp. 262 - 263°C.
   ¹H-NMR(D₆-DMSO):
   δ= 1,2 (3H), 2,7 (2H), 4,1 (2H), 4,2 (2H), ca. 5,5 (2H), 6,9 (1H), 7,2 (1H) und ca. 11,8 (1H) ppm.
g) 1-(2-Ethoxycarbonylethyl)-7-(1-pyrrolyl)-6-trifluormethyl-chinoxalin-2,3-(1H,4H)-dion
   6 g (17,4 mmol) des Produktes 54 f und 3 ml (23 mmol) 2,5-Dimethoxytetrahydrofuran wurden in 150 ml Essigsäure ca. 1 h auf 80°C erwärmt. Anschließend wurde der Ansatz auf Eiswasser gegossen und der anfallende Niederschlag abgesaugt. Man erhielt 6,1 g (89 %) des Produktes. Schmp. 98 - 120°C (Z.).
   ¹H-NMR(D₆-DMSO):
   δ= 1,2 (3H), 2,7 (2H), 4,0 (2H), 4,4 (2H), 6,2 (2H), 6,9 (2H), 7,6 (2H) und 12,3 (1H) ppm.

### Beispiel 55

### 1-(2-Carboxyethyl)-7-(1-pyrrolyl)-6-trifluormethyl-chinoxalin-2,3-(1H,4H)-dion

5 g (12,7 mmol) des Beispiels 54 wurden in 100 ml Tetrahydrofuran gelöst und mit einer Lösung von 0,91 g (38 mmol) Lithiumhydroxid in 100 ml Wasser versetzt. Man rührte 2 h und gab anschließend 20 ml 2M Salzsäure zu. Das organische Lösungsmittel wurde im Vakuum entfernt und die resultierende wäßrige Phase mit Essigsäureethylester extrahiert. Die organische Phase wurde getrocknet und im Vakuum eingeengt. Man erhielt 4,5 g (94 %) des Produktes.
Schmp. 190 - 192°C.
¹H-NMR(D₆-DMSO):
δ= 2,6 (2H), 4,3 (2H), 6,2 (2H), 6,9 (2H), 7,55 (1H), 7,6 (1H) und 12,5 (breit, 2H) ppm.

### Beispiel 56

### 3-(7-(1-Pyrrolyl)-6-trifluormethyl-chinoxalin-2,3-(1H,4H)-dion-1-yl)-propionsäurebenzylamid

1,5 g (4 mmol) von Beispiel 55 und 0,45 ml (4 mmol) Benzylamin wurden in 30 ml wasserfreien Diemthylformamid gelöst und nacheinander mit 0,9 ml (4,2 mmol) Phosphorsäurediphenylesterazid, gelöst in 100 ml Dimethylformamid, und 1 ml (7,2 mmol) Triethylamin versetzt. Alles wurde für 16 h Raumtemperatur gerührt. Anschließend wurde der Ansatz auf Eiswasser gegossen, die wäßrige Phase mit verdünnter Salzsäure angesäuert und der angefallene Niederschlag abgesaugt. Man erhielt 1,47 g (74 %) des Produktes.
Schmp. 234 - 235°C
¹H-NMR(D₆-DMSO):
δ= 2,5 (2H), 4,2 (2H), 4,4 (2H), 6,2 (2H), 6,9 (2H), 7,1 (2H), 7,2 (3H), 7,5 (1H), 7,6 (1H), 8,5 (1H) und 12,5 (1H) ppm.

### Beispiel 57

### 7-(2,5-Dimethyl-1-pyrrolyl)-1-(2-ethoxycarbonylethyl)-6-trifluormethylchinoxalin-2,3-(1H,4H)-dion

6 g (17,4 mmol) des Beispiels 54 f und 2,7 ml (23 mmol) Acetonylaceton wurden in 100 ml Essigsäure für 1 h auf 20°C erwärmt. Anschließend wurde mit viel Wasser verdünnt und mit Essigsäureethylester extrahiert. Die organische Phase wurde getrocknet und im Vakuum eingeengt. Der Rückstand wurde chromatographisch an Kieselgel (Fließmittel: Toluol/Tetrahydrofuran = 1/1) gereinigt. Man erhielt 4 g (55 %) des Produktes. Schmp. 233 - 235°C
¹H-NMR(D₆-DMSO):
δ= 1,1 (3H), 2,1 (6H), 2,7 (2H), 4,0 (2H), 4,4 (2H), 5,8 (2H), 7,6 (1H), 7,65 (1H) und ca. 12,5 (breit) ppm.

### Beispiel 58

### 1-(2-Carboxyethyl)-7-(2,5-dimethyl-1-pyrrolyl)-6-trifluormethyl-chinoxalin-2,3-(1H,4H)-dion

3 g (7,1 mmol) des Beispiels 57 wurden in 100 ml Tetrahydrofuran gelöst und mit 0,51 g (21,3 mmol) Lithiumhydroxid, gelöst in 100 ml Wasser, versetzt. Alles wurde ca. 1 h bei Raumtemperatur gerührt. Anschließend wurde mit verdünnter Salzsäure sauer gestellt und das organische Lösungsmittel im Vakuum entfernt. Der ausfallende Niederschlag wurde abgesaugt, wobei man 2,8 g (100 %) des Produktes erhielt. Schmp. 248 - 250°C (Z).
¹H-NMR(D₆-DMSO):
δ= 1,9 (6H), 2,6 (2H), 4,3 (2H), 5,8 (2H), 7,6 (1H), 7,65 (1H) und ca. 12,5 (breit, 2H) ppm.

### Beispiel 59

### 3-(7-(2,5-Dimethyl-1-pyrrolyl)-6-trifluormethyl-chinoxalin-2,3-(1H,4H)-dion-1-yl)-propionsäure-benzylamid

1,2 g (3 mmol) von Beispiel 58 wurden analog Beispiel 56 mit Benzylamin umgesetzt. Man erhielt 1,25 g (86 %) des Produktes.
Schmp. 260 - 262°C.
¹H-NMR(D₆-DMSO):
δ= 1,85 (6H), 2,55 (2H), 4,2 (2H), 4,4 (2H), 5,8 (2H), 7,1 (2H), 7,2 (3H), 7,5 (1H), 7,6 (1H), 8,5 (1H) und 12,4 (1H) ppm.

### Beispiel 60

0,48 g (1,5 mmol) des Produktes 31g und 0,2 g (1,5 mmol) 2,5-Dimethoxytetrahydrofuran wurden in 5 ml Essigsäure 0,5 h unter Rückfluß gekocht. Anschließend wurde alles im Vakuum eingeengt und der Rückstand mit Eiswasser behandelt. Der Niederschlag wurde abgesaugt. Man erhielt 0,45 g (81 %) des Produktes. Schm.: 239-240°C.
¹H-NMR (D₆-DMSO):
δ = 1,2 (3H), 4,2 (2H), 5,15 (2H), 6,3 (2H), 7,1 (2H), 7,5 (1H), 7,6 (1H), 7,7 (2H), 8,7 (1H) und 12,5 (breit) ppm.

### Beispiel 61

### 1-(Carboxymethyl)-9-(2,5-dimethyl-1-pyrrolyl)-benzo[f]chinoxalin-2,3-(1H,4H)-dion

0,43 g (1,2 mmol) des Beispiels 60 wurde in 5 ml Tetrahydrofuran gelöst und mit einer Lösung aus 0,088 mg (3,6 mmol) Lithiumhydroxid in 3 ml Wasser versetzt. Alles wurde 1 h bei Raumtemperatur gerührt. Anschließend wurde das Tetrahydrofuran im Vakuum entfernt, der Rückstand mit Wasser verdünnt, mit verdünnter Salzsäure sauer gestellt und der anfallende Niederschlag abgesaugt. Ausbeute: 0,36 g (91 %). Schmp.: > 250°C.
¹H-NMR (D₆-DMSO):
δ = 5,1 (2H), 6,35 (2H), 7,1 (2H), 7,5 - 7,7 (4H), 8,7 (1H), 12,5 (1H) und ca. 13,3 (breit) ppm.

### Beispiel 62

### 2-(6-Nitro-7-pyrrol-1-yl-chinoxalin-2,3-(1H,4H)-dion-1-yl)essigsäure(2-phenylethyl)amid

1,5 g (4,5 mmol) des Beispiels 53 und 0,65 g (5,4 mmol) 2-Phenylethylamin wurden analog Beispiel umgesetzt. Man erhielt 1,3 g (68 %) des produktes. Schmp.: 298°C (Z.).
¹H-NMR (D₆-DMSO):
δ = 2,6 (2H), 3,2 (2H), 4,8 (2H), 6,3 (2H), 6,9 (ZH), 7,1 - 7,25 (5H), 7,3 (1H), 7,85 (1H), 8,25 (1H) und 12,5 (1H) ppm.

### Beispiel 63

### 1-Cyclohexyl-7-(3-formyl-1-pyrrolyl)-6-nitro-chinoxalin-2,3(1H,4H)-dion

5,1 g (16,7 mmol) des Produktes 28e und 3,35 g (20,9 mmol) 2,5-Dimethoxy-tetrahydrofuran-3-yl-carbaldehyd wurden in 100 ml Essigsäure 2 h unter Rückfluß gekocht. Anschließend wurde das noch warme Reaktionsgemisch filtriert und das Filtrat auf viel Eiswasser gegossen. Der anfallende Niederschlag wurde abgesaugt und man erhielt 5,4 g (85 %) des Produktes. Schmp.: 300°C (Z.).
¹H-NMR (D₆-DMSO):
δ = 1,1 - 2,5 (10H), 4,5 (1H), 6,7 (1H), 7,2 (1H), 7,95 (1H), 8,0 (2H), 9,8 (1H) und 12,5 (1H) ppm.

### Beispiel 64

### 1-(2-Ethyl-1-butyl)-7-(3-formyl-1-pyrrolyl)-6-nitro-chinoxalin-2,3(1H,4H)-dion

3,0 g (9,8 mmol) des Produktes 5h und 1,6 g (9,8 mmol) 2,5-Dimethoxytetrahydrofuran-3-yl-carbaldehyd wurden in 150 ml Essigsäure 1 h unter Rückfluß gekocht.

Anschließend wurde der Ansatz auf Eiswasser gegossen und der anfallende Niederschlag abgesaugt. Man erhielt 0,9 g (25 %) des Produktes. Schmp.: 240-242°C (Z.).
¹H-NMR (D₆-DMSO):
δ = 0,9 (6H), 1,3 (4H), 1,8 (1H), 4,1 (2H), 6,7 (1H), 7,2 (1H), 7,5 (1H), 7,95 (1H), 8,0 (1H), 9,8 (1H) und 12,5 (1H) ppm.

### Beispiel 65

### 1-Cyclohexyl-6-(3-formyl-1-pyrrolyl)-7-nitrochinoxalin-2,3-(1H,4H)-dion

4 g (13 mmol) des Produktes 37b, 2,3 g (14 mmol) 2,5-Dimethoxytetrahydrofuran-3-yl-carbaldehyd und eine Spatelspitze 4-Toluolsulfonsäure wurden in 200 ml Dimethylformamid/Toluol (1:1) am Wasserabscheider unter Rückfluß erhitzt. Nach vollständigem Umsatz (DC-Kontrolle) wurde alles im Vakuum eingeengt und der Rückstand zwischen Essigsäureethylester und Wasser verteilt. Die organische Phase wurde getrocknet und im Vakuum eingeengt. Dieser Rückstand wurde chromatographisch an Kieselgel (Fließmittel: Toluol/Aceton/Eisessig = 40/20/1) gereinigt. Man erhielt 1,1 g (22 %) des Produktes. Schmp.: 176° (Z.).
¹H-NMR (D₆-DMSO):
δ = 1,1 - 2,5 (10H), 4,5 (1M), 6,7 (1H), 7,2 (1H), 7,25 (1H), 8,3 (1H), 9,8 (1H) und 12,5 (1H) ppm.

### Beispiel 66

### 1-(2,2-Dimethylpropyl)-7-(3-formyl-1-pyrrolyl)-6-nitro-chinoxalin-2,3(1H,4H)-dion

a) 4-Chlor-N-(2,2-dimethylpropyl)-2-nitro-anilin
   57,6 g (0,3 Mol) 2,5-Dichlornitrobenzol, 26,2 g (0,3 Mol) 2,2-Dimethylpropylamin, 82,9 g (0,6 Mol) Kaliumkarbonat und 0,5 g Natriumjodid wurden in 400 ml Dimethylformamid 10 h auf 80°C erwärmt. Anschließend wurde der Ansatz auf viel Wasser gegeben und mit Essigsäureethylester extrahiert. Die organische Phase wurde getrocknet und im Vakuum eingeengt. Der Rückstand wurde chromatographisch an Kieselgel (Fließmittel: Petrolether/Toluol = 16/1) gereinigt. Man erhielt 32,3 g (50 %) des Produktes. Schmp.: 78°C.
   ¹H-NMR (D₆-DMSO):
   δ = 1,0 (9H), 3,2 - 3,4 (2H), 7,2 (1H), 7,55 (1H), 8,05 (1H) und 8,2 (1H) ppm.
b) 2-Amino-4-chlor-N-(2,2-dimethylpropyl)anilin 35,3 g (0,145 Mol) des Produktes 3a wurden in 400 ml Methanol gelöst und nach Zugabe von 4 g Raney-Nickel bei Raumtemperatur und 1 bar hydriert. Danach wurde filtriert und das Filtrat im Vakuum eingeengt. Man erhielt 29,7 g (96 %) des Produktes.
   ¹H-NMR (D₆-DMSO):
   δ = 1,0 (9H), 4,1 (1H), 5,0 (2H), 6,4 (2H) und 6,5 (1H) ppm.
c) 6-Chlor-1-(2,2-dimethyl-propyl)-chinoxalin-2,3-(1H,4H)-dion
   29,2 g (0,137 Mol) des Produktes 3b wurden in 400 ml Oxalsäurediethylester für 3 h unter Rückfluß gekocht. Nach dem Abkühlen wurde der Niederschlag abgesaugt. Man erhielt 28,7 g (78 %) des Produktes. Schmp.: 298 - 299°C.
   1^{1H-NMR (D}₆-DMSO):
   δ = 0,9 (9H), 4,1 (2H), 7,1 (2H), 7,6 (1H) und 12,2 (1H) ppm.
d) 6-Chlor-1-(2,2-dimethylpropyl)-7-nitro-chinoxalin-2,3-(1H,4H)-dion
   28,4 g (0,1 Mol) des Produktes 3c wurden in 250 ml konzentrierter Schwefelsäure gelöst. Bei 0 - 5°C wurden dann 10,8 g (0,1 Mol) Kaliumnitrat portionsweise zugegeben. Anschließend rührte man alles noch 2 h bei Raumtemperatur. Der Ansatz wurde danach auf Eis gegossen und der anfallende Niederschlag abgesaugt. Man erhielt 32,4 g (98 %) des Produktes.
   Schmp.: 270 - 271°C.
   ¹H-NMR (D₆-DMSO):
   δ = 0,9 (9H), 4,1 (2H), 7,3 (1H), 8,3 (1H) und 12,5 (1H) ppm.
e) 7-Amino-1-(2,2-dimethylpropyl)-chinoxalin-2,3(1H,4H)-dion
   32,1 g (0,1 Mol) des Produktes 3d wurden in 500 ml Isopropanol suspendiert und mit 65 g (1,0 Mol) Ammoniumformiat, gelöst in 250 ml Wasser, versetzt. Man kochte alles für 1,5 h unter Rückfluß.
   Anschließend wurde das Isopropanol im Vakuum entfernt und der anfallende Niederschlag abgesaugt. Man erhielt 22,5 g (89 %) des Produktes. Schmp.: 260 - 262°C (Z.). ¹H-NMR (D₆-DMSO): δ = 1,0 (9H), 4,0 (2H), 5,1 (2H), 6,4 (1H), 6,7 (1H), 6,9 (1H) und 11,8 (1H) ppm.
f) 7-Acetamido-1-(2,2-dimethylpropyl)-chinoxalin-2,3(1H,4H)-dion
   22,3 g (90 mmol) des Produktes 3e und 0,1 g 4-(N,N-Dimethylamino)pyridin wurden in 400 ml Essigsäure gelöst und mit einer Lösung von 9,2 g (90 mmol) Essigsäureanhydrid in 25 ml Essigsäure tropfenweise versetzt. Danach rührte man noch 2 h bei 30 - 40°C. Der angefallene Niederschlag wurde abgesaugt. Man erhielt 23,8 g (91 %) des Produktes. Schmp.: 294 - 295°C.
   ¹H-NMR (D₆-DMSO): 1,0 (9H), 2,1 (3H), 4,0 (2H), 7,1 (1H), 7,3 (1H), 8,0 (1H), 10,0 (1H) und 12,0 (1H) ppm.
g) 7-Amino-1-(2,2-dimethylpropyl)-6-nitro-chinoxalin-2,3(1H,4H)-dion
   23,8 g (82 mmol) des Produktes 3f wurden in 250 ml konzentrierter Schwefelsäure gelöst und bei 0 bis 5°C mit 8,3 g (82 mmol) Kaliumnitrat portionsweise versetzt. Danach rührte man noch 2 h bei Raumtemperatur. Anschließend wurde der Ansatz auf Eis gegossen und die Suspension 4 h auf dem Wasserbade erhitzt. Der anfallende Niederschlag wurde abgesaugt. Man erhielt 19,8 g (82 %) des Produktes. Schmp.: > 300°C.
   ¹H-NMR (D₆-DMSO):
   δ = 1,0 (9H), 3,8 - 4,2 (3H), 7,0 (1H), 7,7 (1H) und 11,8 (1H) ppm.
h) 1-(2,2-Dimethylpropyl)-7-(3-formyl-1-pyrrolyl)-6-nitro-chinoxalin-2,3(1H,4H)-dion
   8 g (27 mmol) des Produktes 40 h und 5,5 g (34 mmol) 2,5-Dimethoxytetrahydrofuran-3-ylcarbaldehyd wurden in 100 ml Essigsäure für 2 h unter Rückfluß gekocht. Danach wurde der Ansatz filtriert und das Filtrat auf Eiswasser gegossen. Der anfallende Niederschlag wurde abgesaugt und chromatographisch an Kieselgel (Fließmittel: Toluol/Aceton/Essigsäure = 20:10:1) gereinigt. Man erhielt 5,7 g (56 %) des Produktes.
   Schmp.: 262-264°C (Z.).
   ¹H-NMR (CDCl₃):
   δ = 0,95 (5H), 4,2 (2H), 6,7 (1H), 7,1 (1H), 7,85 (1H), 7,9 (1H), 8,0 (1H), 9,8 (1H) und 12,5 (1H) ppm.

### Beispiel 67

### 1-(2,2-Dimethylpropyl)-7-(3-hydroxy-iminomethyl-1-pyrrolyl)-6-nitro-chinoxalin-2,3(1H,4H)-dion

2 g (5,4 mmol) des Beispiels 66, 0,75 g (11 mmol) Hydroxylammoniumchlorid und 0,89 g (11 mmol) Natriumacetat wurden in 60 ml Ethanol/Wasser (5:1) für 2 h unter Rückfluß gekocht. Nach dem Abkühlen wurde das ausgefallene Produkt abgesaugt. Man erhielt 1,7 g (82 %). Schmp.: 251°C (Z.).
¹H-NMR (CDCl₃):
δ = 0,95 (9H), 4,2 (2H), 6,5-6,6 (1H), 6,9+7,2 (1H), 7,5+7,6 (1H), 7,7-8,2 (3H), 10,6+11,1 (1H) und 12,4 (1H) ppm.
Laut ¹H-NMR liegt ein E/Z-Gemisch vor.

### Beispiel 68

### 7-(3-Cyano-1-pyrrolyl)-1-(2,2-dimethylpropyl)-6-nitro-chinoxalin-2,3(1H,4H)-dion

1,57 g (4 mmol) des Beispiels 67 wurden in 25 ml Essigsäureanhydrid 4 h unter Rückfluß gekocht. Danach wurde der Ansatz auf Eiswasser gegossen und mit Methylenchlorid extrahiert. Die organische Phase wurde getrocknet und im Vakuum eingeengt. Der Rückstand wurde aus Methanol/Ether kristallisiert. Man erhielt 0,55 g (63 %) des Produktes. Schmp.: 220-223°C.
¹H-NMR (D₆-DMSO):
δ = 1,0 (9H), 4,2 (2H), 6,7 (1H), 7,2 (1H), 7,75 (1H), 7,9 (1H), 8,0 (1H) und 12,5 (1H) ppm.

### Beispiel 69

### 1-Cyclohexyl-7-(3-hydroxyiminomethyl-1-pyrrolyl)-6-nitro-chinoxalin-2,3(1H,4H)-dion

1,75 g (4,6 mmol) des Beispiels 65 und 0,32 g (4,6 mmol) Hydroxylammoniumchlorid wurden in 50 ml Ethanol 2 h unter Rückfluß gekocht. Alles wurde im Vakuum eingeengt und der Rückstand chromatographisch an Kieselgel (Fließmittel: Toluol/Aceton/Essigsäure = 20/10/1) gereinigt. Man erhielt 1,7 g (94 %) des Produktes.
Schmp.: 234°C.

### Beispiel 70

### 1-(Ethoxycarbonylmethyl)-7-(3-formyl-1-pyrrolyl)-6-trifluormethyl-chinoxalin-2,3-(1H,4H)-dion

a) Oxalsäuremonoethylester-N-(2-nitro-4-trifluormethyl-phenyl)amid
   51,5 g (0,25 Mol) 2-Nitro-4-trifluormethylanilin, 45 ml (0,32 Mol) Triethylamin und 0,1 g N,N-Dimethylaminopyridin wurden unter einer Stickstoffatmosphäre in 500 ml wasserfreiem Tetrahydrofuran gelöst. Bei 0 - 5°C tropfte man 44,4 g (0,32 Mol) Oxalsäuremonoethylesterchlorid zu. Anschließend rührte man bis zum vollständigen Umsatz bei Raumtemperatur (Dünnschichtchromatographische Kontrolle). Danach wurde alles im Vakuum eingeengt und der Rückstand zwischen Wasser und Essigsäureethylester verteilt. Die organische Phase wurde getrocknet und im Vakuum eingeengt. Das Rohprodukt wurde aus Ethanol umkristallisiert. Man erhielt 68,2 g (89 %) des Produktes. ¹H-NMR (CDCl₃):
   δ = 1,5 (3H), 4,5 (2H), 8,0 (1H), 8,6 (1H), 9,05 (1H) und 12,2 (1H) ppm.
b) Oxalsäuremonoethylester-(N-(ethoxycarbonylmethyl)-N-(2-nitro-4-trifluormethylphenyl)amid
   70 g (0,23 Mol) des Produktes 70a wurden unter einer Stickstoffatmosphäre in 1 l wasserfreiem Tetrahydrofuran gelöst. Bei Raumtemperatur wurden 34,8 g (0,31 Mol) Kalium-tert.-butanolat portionsweise zugegeben. Nachdem man 30 Minuten gerührt hatte, wurden 42,1 g (0,25 Mol) Bromessigsäureethylester zugetropft. Anschließend wurde alles 2 h bei Raumtemperatur gerührt. Danach wurde der Ansatz im Vakuum eingeengt und der Rückstand zwischen Essigsäureethylester und Wasser verteilt. Die organische Phase wurde getrocknet und im Vakuum eingeengt. Man erhielt 63 g (70 %) des Rohproduktes, das direkt weiterverarbeitet wurde.
c) 1-(Ethoxycarbonylmethyl)-6-trifluormethyl-chinoxalin-2,3-(1H,4H)-dion
   63 g (0,16 Mol) des Produktes 70b wurden in 1 l Essigsäure gelöst und unter Rückfluß gekocht. Dazu gab man portionsweise 54 g (0,97 Mol) Eisenpulver. Nachdem man noch 1 h erwärmt hatte, kühlte man den Ansatz ab und filtrierte. Das Filtrat wurde im Vakuum eingeengt und der Rückstand mit Wasser behandelt. Der anfallende Festkörper wurde abgesaugt und aus Ethanol umkristallisiert. Man erhielt 48,2 g (95 %) des Produktes. Schm.: 250-251°C.
   ¹H-NMR (D₆-DMSO):
   δ = 1,25 (3H), 4,7 (2H), 5,0 (2H), 7,5 (3H) und 12,4 (1H) ppm.
d) 1-(Ethoxycarbonylmethyl)-7-nitro-6-trifluormethyl-chinoxalin-2,3-(1H,4H)-dion
   500 ml konzentrierter Schwefelsäure gelöst und bei 0°C portionsweise mit 15 g (0,149 Mol) Kaliumnitrat versetzt. Man rührte weitere 30 Minuten und goß anschließend den Ansatz auf Eiswasser. Die wäßrige Phase wurde mit Essigsäureethylester extrahiert. Die organische Phase wurde im Vakuum konzentriert, der anfallende Niederschlag abgesaugt und aus Ethanol umkristallisiert. Man erhielt 45,9 g (89 %) des Produktes.
   ¹H-NMR (D₆-DMSO):
   δ = 1,25 (3H), 4,2 (2H), 5,0 (2H), 7,7 (1H), 8,25 (1H) und 12,7 (1H) ppm.
e) 7-Amino-1-(ethoxycarbonylmethyl)-6-trifluormethyl-chinoxalin-2,3-(1H,4H)-dion
   43 g (0,12 Mol) des Produktes 70d wurden in 300 ml Dimethylformamid gelöst und nach Zugabe von 2 g Palladium/Kohle (10 %) bei Raumtemperatur und 1 bar hydriert. Anschließend wurde filtriert und das Filtrat im Vakuum eingeengt. Der Rückstand wurde mit Ethanol behandelt und abgesaugt. Man erhielt 37,1 g (95 %) des Produktes. Schmp.: > 250°C.
   ¹H-NMR (D₆-DSO):
   δ = 1,25 (3H), 4,2 (2H), 4,85 (2H), 5,5 (2H), 6,6 (1H), 7,2 (1H) und 12,0 (1H) ppm.
f) 1-(Ethoxycarbonylmethyl)-7-(3-formyl-1-pyrrolyl)-6-trifluormethyl-chonoxalin-2,3-(1H,4H)-dion
   25 g (75,5 mmol) des Produktes 70e und 12 g (75,5 mmol 2,5-Dimethoxytetrahydrofuran-3-yl-carbaldehyd wurden in 300 ml Essigsäure 1 h auf 85°C erwärmt. Anschließend wurde alles im Vakuum eingeengt und chromatographisch an Kieselgel (Fließmittel: Methylenchlorid (Aceton = 3/1) gereinigt. Man erhielt 20,3 g (66 %) des Produktes. Schmp.: 236-237°C.
   ¹H-NMR (D₆-DMSO):
   δ = 1,2 (3H), 4,2 (2H), 5,0 (2H), 6,6 (1H), 7,05 (1H), 7,65 (1H), 7,8 (2H), 9,8 (1H) und 12,3 (1H) ppm.

### Beispiel 71

### 1(Carboxymethyl)-7(3-formyl-1-pyrrolyl)-6-trifluormethyl-chinoxalin-2,3(1H,4H)-dion

1,2 g (2,9 mmol) des Beispiels 70e wurden in 100 ml Tetrahydrofuran gelöst und mit 0,21 g (8,8 mmol) Lithiumhydroxid, in 15 ml Wasser gelöst, versetzt. Man rührte alles 2 h bei Raumtemperatur. Anschließend wurde das organische Lösungsmittel im Vakuum entfernt und die wäßrige Phase eingesäuert. Der anfallende Niederschlag wurde abgesaugt. Man erheilt 0,83 g (75 %) des Produktes.
Schmp.: > 250°C.
¹H-NMR (D₆-DMSO):
δ = 4,9 (2H), 6,65 (1H), 7,1 (1H), 7,65 (1H), 7,75 (1H), 8,0 (1H), 9,8 (1H), 12,5 (1H) und ca. 13,3 (breit) ppm.

### Beispiel 72

### 1-Carboxymethyl-7-(2,5-dimethyl-1-pyrrolyl)-6-trifluormethylchinoxalin-2,3(1H,4H)-dion

a) 1-(Methoxycarbonylmethyl)-7-(2,5-dimethyl-1-pyrrolyl)-6-trifluormethyl-chinoxalin-2,3-(1H,4H)-dion (Beispiel 23)
   5,1 g (15,4 mmol) des Produktes 70e und 1,8 ml (15,4 mmol) 2,5-Hexandion wurden in 100 ml Essigsäure 0,5 h unter Rückfluß gekocht. Danach wurde der Ansatz im Vakuum eingeengt und chromatographisch an Kieselgel (Fließmittel: Methylenchlorid/Methanol = 10/1) gereinigt. Das Rohprodukt wurde mit Wasser behandelt und abgesaugt. Man erhielt 5,3 g (85 %) des Produktes. Schmp.: 262-263°C.
   ¹H-NMR (D₆-DMSO):
   δ = 1,2 (3H), 1,85 (6H), 4,15 (2H), 5,1 (2H), 5,7 (2H), 7,45 (1H), 7,7 (1H) und 12,5 (1H) ppm.
b) 1-Carboxymethyl-7-(2,5-dimethyl-1-pyrrolyl)-6-trifluormethyl-chinoxalin-2,3(1H,4H)-dion
   3,3 g (8,1 mmol) des Produktes 72a wurden in 100 ml Tetrahydrofuran gelöst und 0,58 g (24,2 mmol) Lithiumhydroxid, gelöst in 25 ml Wasser, versetzt. Man rührte 1 h bei Raumtemperatur. Danach wurde das organische Lösungsmittel im Vakuum entfernt und die resultierende wäßrige Phase mit 2 M Salzsäure angesäuert. Der Niederschlag wurde abgesaugt. Man erhielt 28 g (92 %) des Produktes. Schmp.: > 270°C.
   ¹H-NMR (D₆-DMSO):
   δ = 1,85 (6H), 5,0 (2H), 5,8 (2H), 7,4 (1H), 7,7 (1H), 12,5 (1H) und ca. 13,3 (breit) ppm.

### Beispiel 73

### 2(7-(2,5-Dimethyl-1-pyrrolyl)-6-trifluormethyl-chinoxalin-2,3(1H,4H)-dion-1-yl)-essigsäurebenzylamid

1,6 g (4,2 mmol) der Verbindung des Beispiels 72 und 0,5 ml (4,6 mmol) Benzylamin wurden unter einer Stickstoffatmosphäre in 50 ml wasserfreiem Dimethylformamid gelöst. Bei 0°C wurden nacheinander 1 ml (4,6 mmol) Phosphorsäurediphenylesteracid, gelöst in 10 ml wasserfreiem Dimethylformamid, und 1,3 ml (9,2 mmol, Triethylamin zugetropft. Danach wurde 16 h bei Raumtemperatur gerührt. Anschließend wurde der Ansatz mit Wasser verdünnt, angesäuert und mit Essigsäureethylester extrahiert. Die organische Phase wurde getrocknet und im Vakuum eingeengt. Der Rückstand wurde aus Ethanol umkristallisiert. Man erhielt 1,1 g (56 %) des Produktes. Schmp.: > 250°C.
¹H-NMR (D₆-DMSO):
δ = 1,8 (6H), 4,25 (2H), 4,9 (2H), 5,8 (2H), 7,1-7,3 (5H), 7,65 (1H), 8,65 (1H) und 12,4 (1H) ppm.

### Beispiel 74

### 1-Ethoxycarbonylmethyl-7-(3-hydroxyiminomethyl-1-pyrrolyl)-6-trifluormethylchinoxalin-2,3(1H,4H)-dion

1,2 g (2,9 mmol) der Verbindung des Beispiels 70, 0,4 g (6 mmol) Hydroxylammoniumchlorid und 0,48 g (6 mmol) Natriumacetat wurden in 45 ml H₂O/EtOH (2:1) 30 Minuten unter Rückfluß gekocht. Das organische Lösungsmittel wurde im Vakuum entfernt und die resultierende wäßrige Phase mit Essigsäureethylester extrahiert. Die organische Phase wurde getrocknet und im Vakuum eingeengt. Der Rückstand wurde aus Ethanol umkristallisiert, so daß man 1,6 g (70 %) des Produktes erhielt. Schmp.: > 250°C.
¹-NMR (D₆-DMSO):
δ = 1,2 (3H), 4,15 (2H), 5,0 (2H), 6,6 (1H), 6,9 (1H), 7,3 (1H), 7,6-7,8 (3H), 11,1 (1H) und 12,5 (1H) ppm.

### Beispiel 75

### 7-3-Benzyloxyiminomethyl-1-pyrrolyl)-1-(ethoxycarbonyl-methyl)-6-trifluormethyl-chinoxalin-2,3-(1H,4H)-dion

1,1 g (2,7 mmol) der Verbindung des Beispiels 70, 0,86 g (5,4 mmol) o-Benzylhydroxylammoniumchlorid und 0,44 g (5,4 mmol) Natriumacetat wurden in 4,5 ml EtOH/H₂O (1:2) 30 Minuten unter Rückfluß gekocht. Danach wurde das Ethanol im Vakuum entfernt, der anfallende Niederschlag abgesaugt und aus Ethanol umkristallisiert. Man erhielt 1,15 g (84 %) des Produktes. Laut ¹H-NMR liegt ein E/Z-Gemisch vor. Schmp.: 142-147°C.
¹H-NMR (D₆-DMSO):
δ = 1,2 (3H), 4,2 (2H), 5,0 (2H), 5,1+5,2 (2H), 6,5+6,7 (1H), 7,0 (1H), 7,2-8,2 (9H) und 12,5 (1H) ppm.

### Beispiel 76

### 1-Carboxymethyl-7-(3-hydroxyiminomethyl-1-pyrrolyl)-6-trifluormethyl-chinoxalin-2,3(1H,4H)-dion

1,6 g (3,8 mmol) der Verbindung des Beispiels 74 wurden in 100 ml Tetrahydrofuran gelöst und mit 0,4 g (15,1 mmol) Lithiumhydroxid, gelöst in 25 ml Wasser, versetzt. Alles wurde 2 h bei Raumtemperatur gerührt. Danach wurde das Tetrahydrofuran im Vakuum entfernt und die wäßrige Phase angesäuert. Der anfallende Niederschlag wurde abgesaugt. Schmp.: > 225°C.
¹H-NMR (D₆-DMSO):
δ = 4,8 (2H), 6,5 (1H), 6,9 (1H), 7,3 (1H), 7,5 (1H), 7,6 (1H), 7,7 (1H), 11,0 (1H) und 12,5 (1H) ppm.

### Beispiel 77

### 7-(3-Benzyloxyiminomethyl-1-pyrrolyl)-1-carboxymethyl-6-trifluormethyl-chinoxalin-2,3(1H,4H)-dion

1,2 g (2,3 mmol) des Beispiels 74 wurden analog Beispiel 76 mit 0,2 g (7 mmol) Lithiumhydroxid verseift. Man erhielt 1,0 g (90 %) des Produktes.
¹H-NMR (D₆-DMSO):
δ = 4,9 (2H), 5,1+5,2 (1H), 6,4+6,6 (1H), 6,9 (1H), 7,2-8,2 (9H), 12,5 (1H) und 13,5 (breit) ppm.
Laut ¹H-NMR liegt ein E/Z-Gemisch vor.

### Beispiel 78

### 7-(3-Cyano-1-pyrrolyl)-1-cyclohexyl-6-nitro-chinoxalin-2,3(1H,4H)-dion

1,75 g (4,4 mmol) des Beispiels 69 wurden in 25 ml Essigsäureanhydrid für 5 h unter Rückfluß gekocht. Anschließend wurde alles auf Eiswasser gegossen und mit Methylenchlorid extrahiert. Die organische Phase wurde mit wäßriger Natriumhydrogenkarbonat-Lösung gewaschen, dann getrocknet und im Vakuum eingeengt. Man erhielt 1,2 g (70 %) des Produktes. Schmp.: 304-305°C.
¹H-NMR (D₆-DMSO):
δ = 1,0-2,5 (10H), 4,4 (1H), 6,7 (1H), 7,2 (1H), 7,8 (1H), 8,0 (2H) und 12,3 (1H) ppm.

### Beispiel 79

### 1-Cyclohexyl-6-methylsulfonyl-7-(1-pyrrolyl)-chinoxalin-2,3(1H,4H)-dion

a) 1-Cyclohexyl-6-methylsulfonyl-chinoxalin-2,3-(1H,4H)-dion
   23 g (85,7 mmol) N¹-Cyclohexyl-4-mesyl-1,2-benzoldiamin (Dr. Schelz et al., Dyes and Pigments 1983, 4, 305-320) wurden in 200 ml Oxalsäurediethylester für 2 h unter Rückfluß gekocht. Nach dem Abkühlen wurde der Niederschlag abgesaugt. Man erhielt 18,5 g (67 %) des Produktes. Schmp.: > 300°C.
   ¹H-NMR (D₆-DMSO):
   δ = 1,2-2,5 (10H), 3,2 (3H), 4,5(1H), 7,6-7,7 (2H), 7,75 (1H) und 12,3 (1H) ppm.
b) 1-Cyclohexyl-6-methylsulfonyl-7-nitro-chinoxalin-2,3-(1H,4H)-dion
   9 g (28 mmol) des Produktes 79a wurden in 90 ml konzentrierter Schwefelsäure gelöst und bei 0°C mit 2,7 g (31,8 mmol) Natriumnitrat portionsweise versetzt. Danach wurde 3 h bei Raumtemperatur gerührt. Der Ansatz wurde auf Eiswasser gegossen und der anfallende Niederschlag abgesaugt. Dieser wurde anschließend chromatographisch an Kieselgel (Fließmittel: Toluol/Aceton/Essigsäure = 10·10·1) gereinigt. Man erhielt 3,8 g (37 %) des Produktes. Schmp.: > 280°C.
   ¹H-NMR (D₆-DMSO):
   δ = 1,1-2,5 (10H), 3,5 (3H), 4,5 (1H), 7,8 (1H), 8,3 (1H) und 12,5 (1H) ppm.
c) 7-Amino-1-cyclohexyl-6-methylsulfonyl-chinoxalin-2,3(1H,4H)-dion
   2,3 g (6,3 mmol) des Produktes 79b wurden in 100 ml Tetrahydrofuran gelöst und nach Zugabe von 0,3 g Palladium/Kohle (10 %) hydriert. Danach wurde filtriert und das Filtrat im Vakuum eingeengt. Man erhielt 2,0 g eines Rohproduktes, das direkt weiterumgesetzt wurde.
d) 1-Cyclohexyl-6-methylsulfonyl-7(1-pyrrolyl)-chinoxalin-2,3(1H,4H)-dion
   2,5-Dimethoxytetrahydrofuran wurden in 50 ml Essigsäure 30 Minuten unter Rückfluß gekocht. Anschließend wurde alles im Vakuum eingeengt und der Rückstand aus wenig Ethanol umkristallisiert. Man erhielt 0,9 g (38 %) des Produktes.
   Schmp.: > 280°C.
   ¹H-NMR (D₆-DMSO):
   δ = 1,0-2,5 (10H), 3,3 (3H), 4,5 (1H), 6,25 (2H), 7,0 (2H), 7,6 (1H) und 7,7 (1H) ppm.

### Beispiel 80

### 1-(Ethoxycarbonylmethyl)-7-(3-formyl-1-pyrrolyl)-6-nitrochinoxalin-2,3(1H,4H)-dion

5,0 g (1,6 mMol) des Produktes von Beispiel 52i und 2,9 g (1,8 mMol) 2,5-Dimethoxy-tetrahydrofuran-3-ylcarbaldehyd wurden in 100 ml 80-90°C heiße Essigsäure eingetragen. Man rührte für 30 Minuten. Anschließend wurde alles auf Eiswasser gegossen und der Niederschlag abgesaugt. Man erhielt 5,3 g (86%) des Produktes.
¹H-NMR (D₆-DMSO):
δ = 1,2 (3H), 4,15 (3H), 4,15 (2H), 5,05 (2H), 6,7 (1H), 7,15 (1H), 7,8 (1H), 7,9 (1H), 8,0 (1H), 9,8 (1H) und 12,3 (breit) ppm.

### Beispiel 81

### 7-(3-(N-Benzylaminomethylen)-1-pyrrolyl)-1-(ethoxycarbonylmethyl)-6-trifluormethyl-chinoxalin-2,3-(1H,4H)-dion

1,2 g (2,9 mMol) der Verbindung des Beispiels 70, 0,31 g (2,9 mMol) Benzylamin und 0,2 ml (2,9 mMol) Essigsäure wurden in 50 ml Ethanol gelöst und bei Raumtemperatur mit 0,18 g (2,9 mMol) Natriumcyanoborhydrid portionsweise versetzt. Man rührte 16 h. Anschließend wurde alles im Vakuum eingeengt, die organische Phase getrocknet und schließlich wieder im Vakuum eingeengt. Der Rückstand wurde chromatographisch an Kieselgel (Fließmittel: Methylenchlorid: Methanol = 5:1) gereinigt. Man erhielt 0,87 g (60%) des Produktes.
¹H-NMR (D₆-DMSO):
δ = 1,2 (3H), 3,8 (2H), 3,9 (2H), 4,1 (2H), 5,0 (2H), 6,3 (1H), 7,85 (1H), 7,9 (1H), 7,2-7,8 (7H) und 12,3 (breit) ppm.

### Beispiel 82

### 7-(3-Benzyloxyiminomethyl-1-pyrrolyl)-1-(carboxymethyl)-6-nitro-chinoxalin-2,3(1H,4H)-dion

1,5 g (3,9 mMol) des Beispiels 80, 1,2 g (7,8 mMol) O-Benzylhydroxylaminhydrochlorid und 0,6 g (7,8 mMol) Natriumacetat wurden in 45 ml Ethanol/Wasser (1:2) für 2 h unter Rückfluß gekocht. Anschließend wurde mit Wasser verdünnt und mit Essigsäureethylester extrahiert. Die organische Phase wurde getrocknet und im Vakuum eingeengt. Man erhielt 1,7 g eines Öls, das in 50 ml Tetrahydrofuran gelöst und danach mit 0,27 g Lithiumhydroxid, gelöst in 50 ml Wasser, versetzt wurde. Man ließ 2 h bei Raumtemperatur rühren. Anschließend wurde das Tetrahydrofuran im Vakuum entfernt und die resultierende wäßrige Phase mit 2 M Salzsäure angesäuert. Danach wurde mit Essigsäureethylester extrahiert, die organische Phase getrocknet und im Vakuum eingeengt. Der Rückstand wurde chromatographisch an Kieselgel (Fließmittel: Methylenchlorid/Methanol = 2/1) gereinigt. Man erhielt 0,17 g (10 %) des Produktes, das als E/Z-Gemisch vorlag.
¹H-NMR (D₆-DMSO):
δ = 4,5+4,6 (2H), 5,1+5,15 (2H), 6,4-8,5 (11H) und 12,2 (breit) ppm.

### Beispiel 83

### 7-(3-Formyl-1-pyrrolyl)-6-nitro-chinoxalin-2,3(1H,4H)-dion

2,0 g (9,0 mMol) des Produktes 5c und 1,4 g (9,0 mMol) 2,5-Dimethoxy-tetrahydrofuran-3-ylcarbaldehyd wurden in 50 ml Essigsäure 1 h unter Rückfluß gekocht. Der Ansatz wurde anschließend auf Wasser gegossen und der anfallende Niederschlag abgesaugt. Dieser Rückstand wurde nach Zugabe von etwas Aktivkohle und Kieselgel in 60 ml Dimethylformamid/Tetrahydrofuran (1/5) aufgekocht. Die Suspension wurde filtriert und das Filtrat in Vakuum eingeengt. Der Rückstand wurde in Wasser suspendiert und abgesaugt. Man erhielt 0,3 g (13 % d.Th.) des Produktes. Schmp. > 250°C
¹H-NMR (D₆-DMSO):
δ = 6,65 (1H), 7,1 (1H), 7,15 (1H), 7,9 (1H), 7,95 (1H), 9,7 (1H) und ca. 12,3 (breit) ppm.

### Beispiel 84

### 6-Nitro-7-(1-pyrrolyl)-chinoxalin-2,3-(1H,4H)-dion-1-yl-essigsäureethylamid

1,5 g (4,5 mMol) des Beispiels 53 und 0,44 g (5,4 mMol) Ethylaminhydrochlorid wurden analog Beispiel 48 umgesetzt. Man erhielt 0,9 g (56 %) des Produktes.
Schmp. > 300°C
¹H-NMR (D₆-DMSO):
δ = 1,0 (3H), 3,1 (2H), 4,8 (2H), 6,3 (2H), 7,9 (2H), 7,3 (1H), 7,9 (1H), 8,2 (1H) und 12,5 (1H) ppm.

### Beispiel 85

### 6-Nitro-7-(1-pyrrolyl)-chinoxalin-2,3-(1H,4H)-dion-1-yl-essigsäurephenylamid

1,5 g (4,5 mMol) des Beispiels 53 und 0,5 g (5,5 mMol) Anilin wurden analog Beispiel 48 umgesetzt. Man erhielt 0,9 g (49 %) des Produktes.
Schmp. > 300°C
¹H-NMR (D₆-DMSO):
δ = 5,1 (2H), 6,3 (2H), 6,9 (2H), 7,1 (1H), 7,3 (2H), 7,6 (3H), 7,8 (1H), 10,4 (1H) und 12,3 (1H) ppm.

### Beispiel 86

### 6-Nitro-7-(1-pyrrolyl)-chinoxalin-2,3-(1H,4H)-dion-1-yl-essigsäurebenzylamid

1,5 g (4,5 mMol) des Beispiels 53 und 0,58 g (5,4 mMol) Benzylamin wurden analog Beispiel 48 umgesetzt. Man erhielt 1,2 g (63 %) des Produktes.
Schmp. > 300°C
¹H-NMR (D₆-DMSO):
δ = 4,3 (2H), 5,0 (2H), 6,3 (2H), 6,9 (2H), 7,2-7,5 (6H), 7,9 (1H), 8,8 (1H) und 12,5 (1H) ppm.

### Beispiel 87

### 7-(3-(N-Benzylaminomethylen)-1-pyrrolyl)-1-(Carboxymethyl)-6-trifluormethyl-chinoxalin-2,3(1H,4H)-dion

0,9 g (1,8 mMol) des Beispiels 81 wurden in 30 ml Tetrahydrofuran gelöst und mit 0,2 g (8,1 mMol) Lithiumhydroxid, in 25 ml Wasser gelöst, versetzt. Man rührte 2 h bei Raumtemperatur. Anschließend wurde das Tetrahydrofuran im Vakuum entfernt, die resultierende Wasserphase mit 1 M Salzsäure neutralisiert und der Niederschlag abgesaugt. Man erhielt 0,8 g (90 %) des Produktes.
Schmp. > 300°C
¹H-NMR (D₆-DMSO):
δ = 3,9 (2H), 4,0 (2H), 4,65 (2H), 6,35 (1H), 6,9 (1H), 7,05 (1H), 7,2 (1H), 7,3-7,5 (5H), 7.7 (1H) und ca. 12 (1H) ppm.

### Beispiel 88

### 1-(Ethoxycarbonylmethyl)-7-(3-(hydroxymethyl)-1-pyrrolyl)-6-trifluormethyl-chinoxalin-2,3-(1H,4H)-dion

1,1 g (3,3 mMol) des Produktes 70 e und 0,54 g (3,3 mMol) (2,5-Dimethoxy-tetrahydrofuran-3-yl)-methanol wurden in 70 ml Essigsäure 5 h unter Rückfluß gekocht. Anschließend wurde alles im Vakuum eingeengt und der Rückstand in Methylenchlorid gelöst. Durch Zugabe von Petrolether wurde das Produkt ausgefällt, das danach abgesaugt wurde. Man erhielt 1,3 g (45 %).
Schmp. > 185-186°C
¹H-NMR (D₆-DMSO):
δ = 1,2 (3H), 4,15 (2H), 4,95 (2H), 5,05 (2H), 6,25 (1H), 6,9 (1H), 7,0 (1H), 7,6 (1H), 7,6 (2H) und 12,5 (breit) ppm.

### Beispiel 89

### 7-(3-Benzoylaminomethyl-1-pyrrolyl)-1-(carbethoxymethyl)-6-trifluormethyl-chinoxalin-2,3-(1H,4H)-dion

a) N-(2,5-Dimethoxytetrahydrofuran-3-ylmethyl)-benzoesäureamid
   2 g (12,4 mMol) 2,5-Dimethoxyl-3-aminomethyl-tetrahydrofuran und 3,4 ml (24,8 mMol) Triethylamin wurden in 50 ml wasserfreiem Tetrahydrofuran gelöst. Bei 0°C wurden 1,7 g (12,4 mMol) Benzoylchlorid, gelöst in 20 ml wasserfreiem Tetrahydrofuran, zugetropft. Man rührte 1 h. Der Ansatz wurde filtriert und das Filtrat im Vakuum eingeengt. Der Rückstand wurde aus Ether/Petrolether umgefällt. Man erhielt 2,4 g des Produktes, das ungereinigt weiter eingesetzt wurde.
b) 7-(3-Benzoylaminomethyl-1-pyrrolyl)-1-(carbethoxymethyl)-6-trifluormethyl-chinoxalin-2,3-(1H,4H)-dion
   1 g (3,0 mMol) des Produktes 70 e und 1,6 g (6,0 mMol) des Produktes 89 a wurden in 70 ml Essigsäure 30 Minuten bei 100°C gerührt. Nach dem Abkühlen wurde der Niederschlag abgesaugt und mit Wasser gewaschen. Man erhielt 1,1 g (71 %) des Produktes.
   Schmp. > 210-211°C
   ¹H-NMR (D₆-DMSO):
   δ = 1,2 (3H), 4,15 (2H), 4,35 (2H), 5,05 (2H), 6,2 (1H), 6,8 (2H), 7,4-7,6 (5H), 7,7-7,8 (2H), 8,8 (1H) und 12,5 (1H) ppm.

### Beispiel 90

### 7-(3-Benzoylaminomethyl-1-pyrrol)-1-(carbethoxymethyl)-6-trifluormethyl-chinoxalin-2,3-(1H,4H)-dion

1 g (1,9 mMol) des Beispiels 89 wurden in 40 ml Tetrahydrofuran gelöst und mit 0,14 g (5,8 mMol) Lithiumhydroxid, gelöst in 10 ml Wasser, versetzt. Man rührte alles 24 h bei Raumtemperatur. Anschließend wurde mit 1 M Salzsäure angesäuert und das ausgefallene Produkt abgesaugt. Man erhielt 0,8 g (85 %) des Produktes.
¹H-NMR (D₆-DMSO):
δ = 4,35 (2H), 4,9 (2H), 6,25 (1H), 6,8 (1H), 6,85 (1H), 7,4-7,55 (5H), 7,9 (2H), 8,8 (1H) und 12,4 (breit) ppm.

### Beispiel 91

### 7-(3-(Acetylaminomethyl)-1-pyrrolyl)-1-(carbethoxymethyl)-6-trifluormethyl-chinoxalin-2,3-(1H,4H)-dion

a) N-(2,5-Dimethoxytetrahydrofuran-3-ylmethyl)-essigsäureamid
   4.0 g 2,5-Dimethoxy-3-aminomethyl-tetrahydrofuran und 1,75 ml Acetylchlorid wurden analog dem Produkt 89 a umgesetzt. Man erhielt 3,7 g des verunreinigten Produktes, das sofort weiter umgesetzt wurde.
b) 7-(3-Acetylaminomethyl-1-pyrrolyl)-1-(carbethoxymethyl)-6-trifluormethyl-chinoxalin-2,3-(1H,4H)-dion
   1,5 g (4,5 mMol) des Produktes 70 e wurden mit 3,7 g des Produktes 91 a in 70 ml Essigsäure 30 Minuten unter Rückfluß gekocht. Anschließend wurde alles im Vakuum eingeengt. Der Rückstand wurde aus Ethanol unkristallisiert. Man erhielt 1,6 g (79 %) des Produktes.
   Schmp. > 250°C
   ¹H-NMR (D₆-DMSO):
   δ = 1,2 (3H), 4,1-4,3 (3H), 4,1-4,3 (4H), 5,0 (2H), 6,15 (1H), 6,8 (2H), 7,5 (1H), 7,6 (1H), 8,2 (1H) und 12,5 (breit) ppm.

### Beispiel 92

### 7-(3-Acetylaminomethyl-1-pyrrolyl)-1-(carbethoxymethyl)-6-trifluormethyl-chinoxalin-2,3-(1H,4H)-dion

1,3 g (2,9 mMol) des Beispiels 91 wurden in 50 ml Tetrahydrofuran suspendiert und mit 0,21 g (8,6 mMol) Lithiumhydroxid, gelöst in 25 ml Wasser, versetzt. Man rührte 1,5 h bei Raumtemperatur. Anschließend wurde das Tetrahydrofuran im Vakuum entfernt und die resultierende wäßrige Phase mit 1 M Salzsäure angesäuert. Der Niederschlag wurde abgesaugt, wobei man 1,0 g (85 %) des Produktes erhielt.
Schmp. > 250-251°C
¹H-NMR (D₆-DMSO):
δ = 1,8 (3H), 4,1 (3H), 4,9 (2H), 6,2 (1H), 6,8 (2H), 7,4 (1H), 7,6 (1H), 8,1 (1H), 12,4 (1H) und 13,3 (breit) ppm.

### Beispiel 93

### 1-(Carboxylmethyl)-7-(3-formyl-1-pyrrolyl)-6-nitro-chinoxalin-2,3(1H,4H)-dion

1,7 g (4,4 mMol) des Beispiels 80 wurden in 50 ml Tetrahydrofuran gelöst und mit 0,32 g 813,4 mMol) Lithiumhydroxid analog Beispiel 90 hydrolysiert. Man erhielt 1,1 g (69 %) des Produktes.
¹H-NMR (D₆-DMSO):
δ = 4,9 (2H), 6,6 (1H), 7,15 (1H), 7,7 (1H), 7,9 (1H), 8,0 (1H), 9,7 (1H) und 12,8 (breit) ppm.

### Beispiel 94

### 9-(3-Benzoylaminomethyl-1-pyrrolyl)-1-(ethoxycarbonylmethyl)-benzo[f]chinoxalin-2,3-(1H,4H)-dion

2,0 g (6,3 mMol) des Produktes 34 g und 2,5 g (9,6 mMol) des Tetrahydrofuran-Derivats 89 a wurden in 50 ml Essigsäure ca. 10 Minuten auf 100°C erwämrt. Anschließend wurde alles im Vakuum eingeengt, der Rückstand mit Ethanol behandelt und der Niederschlag abgesaugt. Man erhielt 2,5 g (80 %) des Produktes. Schmp. > 250°C
¹H-NMR (D₆-DMSO):
δ = 1,2 (3H), 4,15 (3H), 4,25 (2H), 5,1 (2H), 6,3 (1H), 7,0 (2H), 7,3-7,75 (7H), 7,85 (2H), 8,7 (1H), 8,85 (1H) und 12,5 (breit) ppm.

### Beispiel 95

### 9-(2-Benzoylaminomethyl-1-pyrrolyl)-1-(ethoxycarbonylmethyl)-benzo[f]chinoxalin-2,3-(1H,4H)-dion

a) N-(2,5-Dimethoxytetrahydrofuran-2-ylmethyl)-benzoesäureamid
   10 g (62 mMol) 2-Aminomethyl-2,5-dimethoxy-tetrahydrofuran und 17 ml (123 mMol) Triethylamin wurden in 100 ml wasserfreiem Tetrahydrofuran gelöst. Bei 0°C tropfte man 7,2 ml (62 mMol) Benzoylchlorid zu und rührte alles 1 h bei 0°C. Der Ansatz wurde filtriert und das Filtrat im Vakuum eingeengt. Man erhielt 17 g des Rohproduktes, das direkt weiter umgesetzt wurde.
b) 9-(2-Benzoylaminomethyl-1-pyrrolyl)-1-(ethoxycarbonylmethyl)-benzo[f]chinoxalin-2,3-(1H,4H)-dion
   2 g (6,4 mMol) des Produktes 34 g und 2,5 g (9,6 mMol) des obigen Produktes 95 a wurden in 100 ml Essigsäure analog Beispiel 94 umgesetzt. Man erhielt 2,7 g (86 %) des Produktes.
   Schmp. > 206-207°C
   ¹H-NMR (D₆-DMSO):
   δ = 1,2 (3H), 4,0-4,3 (4H), 5,0 (2H), 6,75 (2H), 6,9 (1H), 7,15 (1H), 7,2-7,7 (8H), 7,9 (1H), 8,45 (1H), 8,7 (1H) und 12,5 (breit) ppm.

### Beispiel 96

### 9-(2-Benzoylaminomethyl-1-carbonylmethyl)-benzo[f]chinoxalin-2,3-(1H,4H)-dion

1,4 g (2,8 mMol) des Beispiels 95 und 0,2 g (8,5 mMol) Lithiumhydroxid wurden analog Beispiel 90 umgesetzt. Man erhielt 1,1 g (84 %) des Produktes.
Schmp. > 180°C
¹H-NMR (D₆-DMSO):
δ = 4,1-4,3 (2H), 4,8 (1H), 5,0 (1H), 6,3 (2H), 6,9 (1H), 7,2 (1H), 7,3 (2H), 7,45 (1H), 7,55 (3H), 7,7 (1H), 7,8 (1H), 8,4 (1H), 12,5 (1H) und ca. 13,3 (breit) ppm.

## Patentansprüche

1. Chinoxalin-2,3-(1H,4H)-dione der allgemeinen Formel I 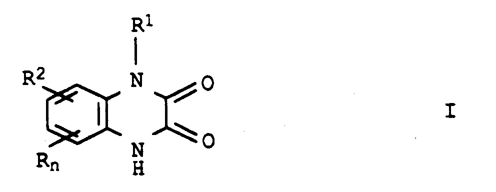 und ihre tautomeren und enantiomeren Formen sowie ihre physiologisch verträglichen Salze, wobei die Variablen folgende Bedeutung haben:
R¹ - Wasserstoff,
- ein cycloaliphatischer Rest mit bis zu 8 C-Atomen,
- eine Phenylgruppe,
- ein aliphatischer Rest mit 1 bis 12 C-Atomen, der einen oder zwei gleiche oder verschiedene der Substituenten Phenyl, Cyclopentyl, Cyclohexyl -CO-R³, -CO-O-R³, -CO-NH-R³, -OR³, -NR⁷R³, 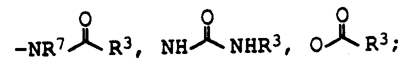 =N-OR³, -CN
tragen kann, wobei R³ und R⁷ unabhängig voneinander Wasserstoff, C₁-C₄-Alkyl, Phenyl, Benzyl, 1-Phenylethyl oder 2-Phenylethyl bedeuten, und
wobei die in R¹ enthaltenen oder R¹ darstellenden aliphatischen und aromatischen Cyclen bis zu drei gleiche oder verschiedene der folgenden Substituenten tragen können C₁-C₄-Alkyl, C₁-C₄-Halogenalkyl, C₁-C₄-Alkoxy, C₁-C₄-Halogenalkoxy, Halogen, Nitro, Cyano, -CO-OR⁹, -CO-NH-R⁹, -OH, 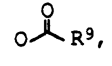 -NHR⁹, -NR⁹R¹⁰, 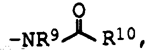 =N-OR⁹, =O;
wobei R⁹ und R¹⁰ unabhängig voneinander Wasserstoff, C₁-C₄-Alkyl, Phenyl, Benzyl, 1-Phenylethyl und 2-Phenylethyl bedeuten,
R² - eine Pyrrol-1-ylgruppe, die einen oder zwei der folgenden Substituenten tragen kann:
C₁-C₄-Alkyl, Phenyl, Phenylsulfonyl, Nitro, Cyano oder einen Substituent aus der Gruppe
-CO-O-R³, -CO-NH-R³, -CH₂-O-R³, -O-R³, -CH=NO-R³, -C(O)R³, 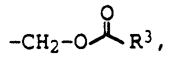 -CH₂NR⁷R³, 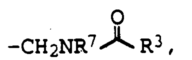 -CH=CH-R⁸, -CH=N-R³,
wobei R⁸ -COOR³, -CONH-R³, CN und Phenyl sein kann;
R - gleiche oder verschiedene der folgenden Reste: C₁-C₄-Alkyl, C₁-C₄-Alkoxy, Trifluormethyl, Trichlormethyl, Trifluormethoxy, Trichlormethoxy, Fluor, Chlor, Brom, Jod, Nitro, Cyano und eine der Gruppen
-CO-O-R³, -CO-NH-R³, -SO₂R³ und 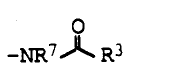 sowie einen anellierten Benzolring, der seinerseits bis zu drei der für R genannten Reste tragen kann;
n - eine ganze Zahl von 0 bis 3, für den anellierten Benzolring 0 oder 1.

2. Chinoxalin-2,3-(1H,4H)-dione nach Anspruch 1, in der die Reste in der Formel I die folgende Bedeutung haben:
R¹ - Wasserstoff, C₁-C₄-Alkyl,
- eine der Gruppen
-(CH₂)ₘ-R⁴
wobei m für einen Wert von 0 bis 2 steht und R⁴ die Cyclohexylgruppe oder die Phenylgruppe bedeutet, welche jeweils ein oder zwei Fluor,
Chlor, Brom, Trifluormethyl, Trifluormethoxy, C₁-C₄-Alkyl, -OR³, -OC(O)R³, -NHR³, -NH-C(O)R³, =O, =N-O-R³ oder eine Nitrogruppe als Substituenten tragen kann, oder
- die Gruppe -CHR⁵-(CH₂)ₘ-CO-O-R⁶ oder -CHR⁵-(CH₂)ₘ-CO-NH-R⁶, wobei R⁵ Wasserstoff, R⁴ oder C₁-C₄-Alkyl und R⁶ Wasserstoff, C₁-C₄-Alkyl oder -(CH₂)ₘ-R⁴ bedeuten;
R² - eine Pyrrol-1-ylgruppe, die einen oder zwei der folgenden Substituenten tragen kann:
C₁-C₄-Alkyl, C₁-C₄-Alkoxy, Phenyl, Formyl, Acetyl, Propionyl und eine der Gruppen
-CH=NOR³, -CH₂N(R⁷)C(O)R³, -CH₂NR⁷R³, -CH₂OC(O)R³, -CO-O-R³ und -CO-NH-R³;
wobei R³ und R⁷ unabhängig voneinander Wasserstoff, C₁-C₄-Alkyl, Phenyl, Benzyl und 2-Phenylethyl sein können;
R - C₁-C₄-Alkyl, Fluor, Chlor, Brom, Nitro, Trifluormethyl, Trifluormethoxy oder einen anellierten Benzolring, der seinerseits einen für R genannten Rest tragen kann;
n - 0 oder 1.

3. Chinoxalin-2,3-(1H,4H)-dione der allgemeinen Formel I' 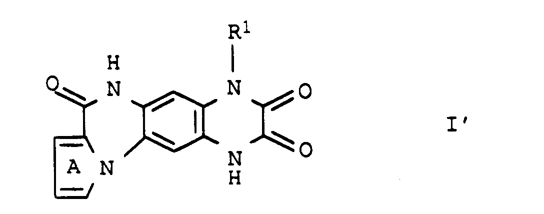 und ihre tautomeren und enantiomeren Formen sowie ihre physiologisch verträglichen Salze, wobei R¹ die in Anspruch 1 genannte Bedeutung hat und wobei der Ring A die in Anspruch 1 als Substituenten an der Pyrrol-1-ylgruppe genannten Reste tragen kann.

4. Verfahren zur Herstellung der Chinoxalin-2,3-(1H,4H)-dione I gemäß Anspruch 1 oder 2, dadurch gekennzeichnet, daß man ein Aminophenylen-1,2-diamin II 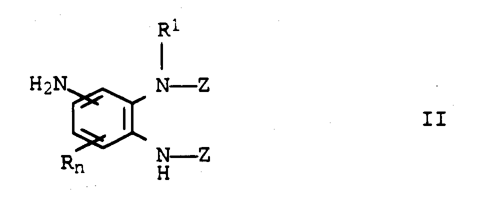 wobei Z für eine als Schutzgruppe fungierende Acylgruppe steht, mit Bernsteindialdehyd oder seinem acyclischen oder cyclischen Acetal oder Hemiacetal (Verbindung III), wobei die C-Atome von III je einen der für die Pyrrol-1-ylgruppe in Anspruch 1 genannten Substituenten tragen können, in an sich bekannter Weise zu der Verbindung IV 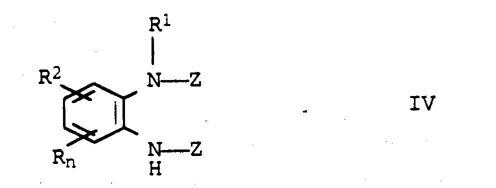 umsetzt, die Schutzgruppe Z in an sich bekannter Weise abspaltet und den Ringschluß zu I in an sich bekannter Weise mit Oxalsäure oder einem ihrer funktionellen Derivate vornimmt, oder
daß man II in schutzgruppenfreier Form zunächst mit Oxalsäure oder einem ihrer funktionellen Derivate in die entsprechenden Aminochinoxalin-2,3-(1H,4H)-dione überführt und diese danach mit III zu I umsetzt.

5. Verfahren zur Herstellung der Chinoxalin-2,3(1H,4H)-dione I' gemäß Anspruch 3, dadurch gekennzeichnet, daß man die Nitrogruppe einer gemäß Anspruch 4 erhältlichen Verbindung I'' 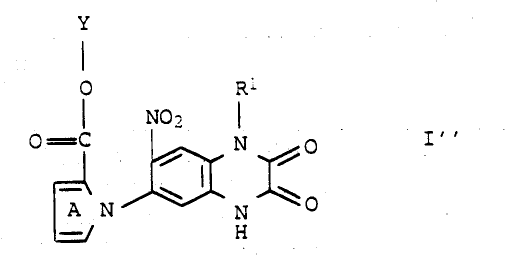 wobei Y Wasserstoff oder eine C₁-C₄-Alkylgruppe bedeutet, unter sauren Bedingungen reduziert.

6. Chinoxalin-2,3(1H,4H)-dione I und I' gemäß den Ansprüchen 1 bis 5 zur Verwendung als Arzneimittel für die Human- und Veterinärmedizin.

7. Verwendung der Chinoxalin-2,3(1H,4H)-dione I und I' gemäß den Ansprüchen 1 bis 5 zur Herstellung von Arzneimitteln zur Behandlung neurodegenerativer Erkrankungen und neurotoxischer Störungen des zentralen Nervensystems sowie zur Herstellung von Spasmolytika, Antiepileptika, Anxiolytika und Antidepressiva.

8. Arzneimittelzubereitungen, enthaltend neben den üblichen Arzneimittelhilfsstoffen eine therapeutisch wirksame Menge der Chinoxalin-2,3(1H,4H)-dione I und I' gemäß den Ansprüchen 1 bis 5.

## Claims

1. A 2,3(1H,4H)-quinoxalinedione of the formula I 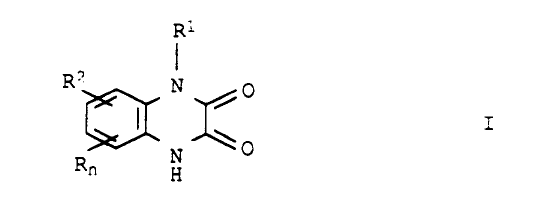 and its tautomers and enantiomers and its physiologically tolerated salts, where
R¹ is hydrogen, a cycloaliphatic radical with up to 8 carbons, phenyl, an aliphatic radical which has from 1 to 12 carbons and can have one or two identical or different substituents from phenyl, cyclopentyl, cyclohexyl, -CO-R³, -CO-O-R³, -CO-NH-R³, -OR³, -NR⁷R³, 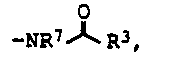 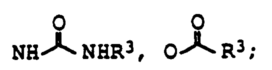 =N-OR³, -CN, where R³ and R⁷ independently of one another are hydrogen, C₁-C₄-alkyl, phenyl, benzyl, 1-phenylethyl or 2-phenylethyl, and where the aliphatic and aromatic rings present in R¹ or representing R¹ can have up to three identical or different substituents from the following: C₁-C₄-alkyl, C₁-C₄-haloalkyl, C₁-C₄-alkoxy, C₁-C₄-haloalkoxy, halogen, nitro, cyano, -CO-OR⁹, -CO-NH-R⁹, -OH, 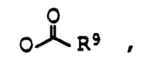 -NHR⁹, -NR⁹R¹⁰, 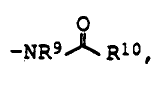 =N-OR⁹, =O; where R⁹ and R¹⁰ independently of one another are hydrogen, C₁-C₄-alkyl, phenyl, benzyl, 1-phenylethyl and 2-phenylethyl,
R² is 1-pyrrolyl which can have one or two of the following substituents: C₁-C₄-alkyl, phenyl, phenylsulfonyl, nitro, cyano or a substituent from the group consisting of -CO-O-R³, -CO-NH-R³, -CH₂-O-R³, -O-R³, -CH=NO-R³, -C(O)R³, 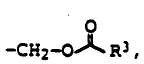 -CH₂NR⁷R³, 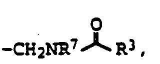 -CH=CH-R⁸, -CH=N-R³, where R⁸ can be -COOR³, -CONH-R³, CN and phenyl;
R radicals are identical or different and are the following: C₁-C₄-alkyl, C₁-C₄-alkoxy, trifluoromethyl, trichloromethyl, trifluoromethoxy, trichloromethoxy, fluorine, chlorine, bromine, iodine, nitro, cyano and -CO-O-R³, -CO-NH-R³, -SO₂R³ and 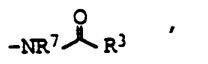 and a fused-on benzene ring which in turn can carry up to three of the radicals mentioned for R;
n is an integer from 0 to 3, for the fused-on benzene ring 0 or 1.

2. A 2,3(1H,4H)-quinoxalinedione as claimed in claim 1, where
R¹ is hydrogen, C₁-C₄-alkyl,
- (CH₂)ₘ-R⁴ where m is from 0 to 2 and R⁴ is cyclohexyl or phenyl which can each be mono- or di-substituted by fluorine, chlorine, bromine, trifluoromethyl, trifluoromethoxy, C₁-C₄-alkyl, -OR³, -OC(O)R³, -NHR³ -NH-C(O)R³, =O, =N-O-R³ or nitro, or
- CHR⁵-(CH₂)ₘ-CO-O-R⁶ or -CHR⁵-(CH₂)ₘ-CO-NH-R⁶ where R⁵ is hydrogen, R⁴ or C₁-C₄-alkyl and R⁶ is hydrogen, C₁-C₄-alkyl or -(CH₂)ₘ-R⁴;
R² is 1-pyrrolyl which can have one or two of the following substituents: C₁-C₄-alkyl, C₁-C₄-alkoxy, phenyl, formyl, acetyl, propionyl and -CH=NOR³, -CH₂N(R⁷)C(O)R³, -CH₂NR⁷R³, -CH₂OC(O)R³, -CO-O-R³ and -CO-NH-R³; where R³ and R⁷ independently of one another can be hydrogen, C₁-C₄-alkyl, phenyl, benzyl and 2-phenylethyl;
R is C₁-C₄-alkyl, fluorine, chlorine, bromine, nitro, trifluoromethyl, trifluoromethoxy or a fused-on benzene ring which in turn can carry a radical mentioned for R;
n is 0 or 1.

3. A 2,3(1H,4H)-quinoxalinedione of the formula I' 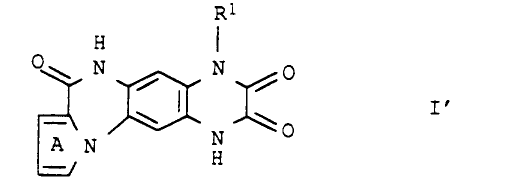 and its tautomers and enantiomers and its physiologically tolerated salts, where R¹ has the meaning specified in claim 1, and where ring A can be substituted as mentioned in claim 1 for 1-pyrrolyl.

4. A process for preparing a 2,3(1H,4H)-quinoxalinedione I as claimed in claim 1 or 2, which comprises reacting an aminophenylene-1,2-diamine II 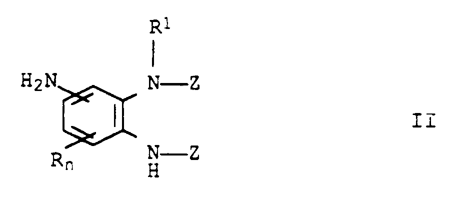 where Z is an acyl protective group, with succinaldehyde or its acyclic or cyclic acetal or hemiacetal (compound III), it being possible for each of the carbons of III to be substituted as mentioned for 1-pyrrolyl in claim 1, in a conventional manner to give a compound IV 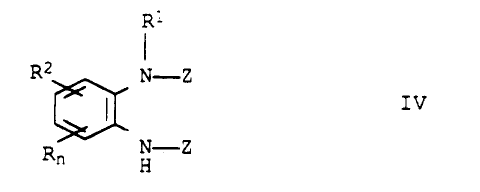 eliminating the protective group Z in a conventional manner and effecting the ring closure to I in a conventional manner with oxalic acid or one of its functional derivatives, or
initially converting II in unprotected form with oxalic acid or one of its functional derivatives into the corresponding amino-2,3(1H,4H)-quinoxalinediones and then reacting the latter with III to give I.

5. A process for preparing a 2,3(1H,4H)-quinoxalinedione I' as claimed in claim 3, which comprises reducing the nitro group of a compound I'', obtainable as claimed in claim 4, 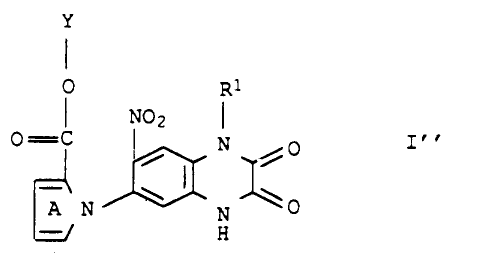 where Y is hydrogen or C₁-C₄-alkyl, under acidic conditions.

6. A 2,3(1H,4H)-quinoxalinedione I or I' as claimed in claims 1 to 5 for use as drug in human and veterinary medicine.

7. The use of a 2,3(1H,4H)-quinoxalinedione I or I' as claimed in claims 1 to 5 for producing drugs for the treatment of neurodegenerative disorders and neurotoxic disturbances of the central nervous system and for producing spasmolytics, antiepileptics, anxiolytics and antidepressants.

8. A pharmaceutical composition which, besides conventional pharmaceutical ancillary substances, contains a therapeutically effective amount of a 2,3(1H,4H)-quinoxalinedione I or I' as claimed in claims 1 to 5.

## Revendications

1. Quinoxaline-2,3-(1H,4H)-dione de la formule générale I 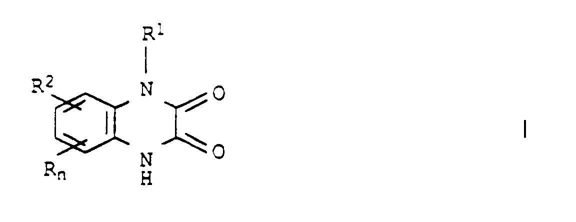 et leurs formes tautomériques et énantiomériques, ainsi que leurs sels physiologiquement compatibles, où les variables ont les significations qui suivent:
R¹ représente - un atome d'hydrogène,
- un radical cycloaliphatique comptant jusqu'à 8 atomes de carbone,
- un radical phényle,
- un radical aliphatique comptant de 1 à 12 atomes de carbone, qui peut porter un ou deux substituants identiques ou différents choisis parmi les radicaux phényle, cyclopentyle, cyclohexyle,
-CO-R³, -CO-O-R³, -CO-NH-R³, -OR³, -NR⁷R³, 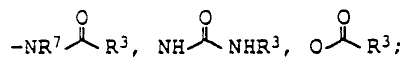 =N-OR³, -CN
où R³ et R⁷ représentent, chacun indépendamment l'un de l'autre, un atome d'hydrogène, un radical alkyle en C₁ à C₄, phényle, benzyle, 1-phényléthyle ou 2-phényléthyle et
où les cycles ou noyaux aliphatiques et aromatiques contenus dans R¹ ou représentant R¹ peuvent porter jusqu'à 3 substituants identiques ou différents, choisis parmi les suivants : alkyle en C₁ à C₄, halogénoalkyle en C₁ à C₄, alcoxy en C₁ à C₄, halogénoalcoxy en C₁ à C₄, halogéne, nitro, cyano, -CO-OR⁹, -CO-NH-R⁹,-OH, 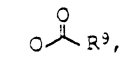 -NHR⁹, -NR⁹R¹⁰, 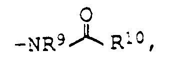 =N-OR⁹, =O; où R⁹ et R¹⁰ représentent, chacun indépendamment l'un de l'autre, un atome d'hydrogène, un radical alkyle en C₁ à C₄, phényle, benzyle, 1-phényléthyle et 2-phényléthyle,
R² représente un radical pyrrole-1-yle, qui peut porter un ou deux des substituants suivants : alkyle en C₁ à C₄, phényle, phénylsulfonyle, nitro, cyano, ou un substituant choisi dans le groupe des radicaux qui suivent :
-CO-O-R³, -CO-NH-R³, -CH₂-O-R³, -O-R³, -CH=NO-R³, -C(O)R³, 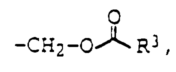 -CH₂NR⁷R³, 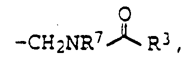 -CH=CH-R⁸, -CH=N-R³,
où R⁸ peut représenter un radical -COOR³, -CONH-R³, CN et phényle,
R représente des radicaux identiques ou différents, choisis parmi ceux qui suivent : alkyle en C₁ à C₄, alcoxy en C₁ à C₄, trifluorométhyle, trichlorométhyle, trifluorométhoxy, trichlorométhoxy, fluor, chlore, brome, iode, nitro, cyano et l'un des radicaux qui suivent:
-CO-O-R³, -CO-NH-R³, -SOR₂R³ et 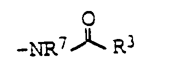 ainsi qu'un noyau benzénique annelé, qui, de son côté, peut porter jusqu'à 3 des radicaux cités à propos de R,
n représente un nombre entier dont la valeur varie 0 à 3, pour le noyau benzénique annelé, 0 ou 1.

2. Quinoxaline-2,3-(1H,4H)-dione suivant la revendication 1, dans lesquelles les restes dans la formule I possèdent les significations qui suivent:
R¹ représente un atome d'hydrogène, un radical alkyle en C₁ à C₄, l'un des radicaux
-(CH₂)ₘ-R⁴
où m a une valeur qui varie de 0 à 2 et R⁴ représente le radical cyclohexyle ou le radical phényle, qui peut à chaque fois porter un ou deux atomes de fluor, de chlore, de brome, ou un ou deux radicaux trifluorométhyle, trifluorométhoxy, alkyle en C₁ à C₄, -OR³, OC(O)R³, -NHR³, -NH-C(O)R³, =O, =N-O-R³ ou un groupe nitro, à titre de substituants, ou bien
le radical -CHR⁵-(CH₂)ₘ-CO-O-R⁶ ou -CHR⁵-(CH₂)ₘ-CO-NH-R⁶ ou R⁵ représente un atome d'hydrogène, R⁴ ou un radical alkyle en C₁ à C₄, et R⁶ représente un atome d'hydrogène, un radical alkyle en C₁ à C₄, ou -(CH₂)ₘ-R⁴,
R² représente un groupe pyrrole-1-yle, qui peut porter un ou deux des substituants suivants : alkyle en C₁ à C₄, alcoxy en C₁ à C₄, phényle, formyle, acétyle, propionyle et l'un des radicaux -CH=NOR³, - CH₂N(R⁷)C(O)R³, -CH₂NR⁷R³, -CH₂OC(O)R³, -Cl-O-R³ et -CO-NH-R³,
où R³ et R⁷ peuvent représenter, chacun indépendamment l'un de l'autre, un atome d'hydrogène, un radical alkyle en C₁ à C₄, phényle, benzyle et 2-phényléthyle,
R représente un radical alkyle en C₁ à C₄, un atome de fluor, de chlore, de brome, un radical nitro, trifluorométhyle, trifluorométhoxy, ou un noyau benzénique annelé, qui, à son tour, peut porter un reste cité à propos de R,
n est égal à 0 ou 1.

3. Quinoxaline-2,3-(1H,4H)-dione de la formule générale I' 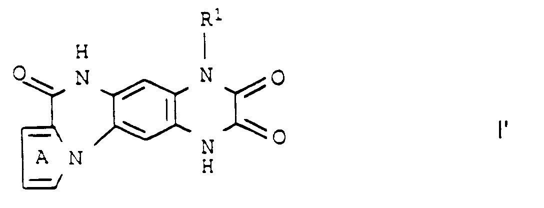 et leurs formes tautomériques énantiomériques, ainsi que leurs sels physiologiquement compatibles, où R¹ possède les significations qui lui ont été attribuées dans la revendication 1 et où le noyau A peut porter les restes cités à titre de substituants dans la revendication 1 sur le groupe pyrrole-1-yle.

4. Procédé de préparation des quinoxaline-2,3-(1H,4H)-dione 1 suivant la revendication 1 ou 2, caractérisé en ce que l'on convertit une aminophénylène-1,2-diamine II 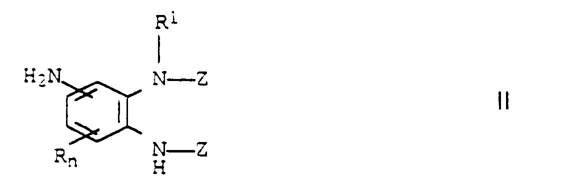 où Z représente un radical acyle servant de groupe protecteur avec le dialdéhyde succinique ou son acétal ou hémiacétal (composé III) acyclique ou cyclique, où les atomes de carbone de III peuvent porter l'un des substituants cités dans la revendication 1 pour le radical pyrrole-1-yle de manière en soi connue, en le composé IV 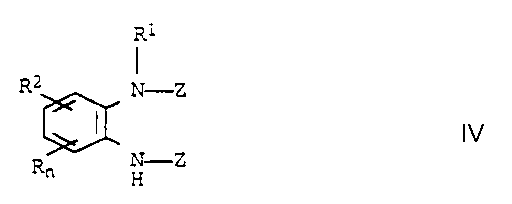 on sépare le groupe protecteur Z de manière en soi connue et on entreprend la fermeture du cycle ou cyclisation en I, de manière en soi connue, à l'aide d'acide oxalique ou d'un de ses dérivés fonctionnels, ou bien on convertit II sous forme dépourvue de groupes protecteurs d'abord avec l'acide oxalique ou un de ses dérivés fonctionnels en les aminoquinoxaline-2,3-(1H,4H)-diones correspondantes et on convertit ensuite ces dernières avec III en I.

5. Procédé de préparation des quinoxaline-2,3-(1H,4H)-dione I' suivant la revendication 3, caractérisé en ce que l'on réduit le groupe nitro d'un composé I'' que l'on peut obtenir suivant la revendication 4 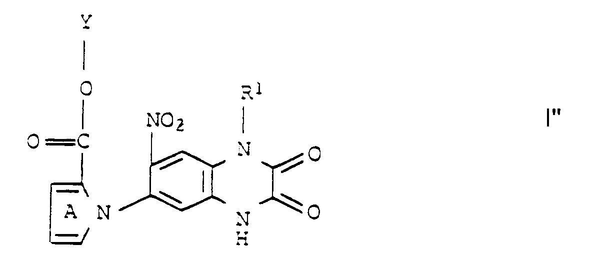 dans laquelle Y représente un atome d'hydrogène ou un groupe alkyle en C₁ à C₄, dans des conditions acides.

6. Quinoxaline-2,3-(1H,4H)-dione I et I' suivant les revendications 1 à 5, en vue de leur utilisation à titre de médicaments en médecine humaine et vétérinaire.

7. Utilisation des quinoxaline-2,3-(1H,4H)-dione I et I' suivant les revendications 1 à 5, en vue de la fabrication de médicaments destinés au traitement de maladies neurodégénératives et de perturbations neurotoxiques du système nerveux central, ainsi que pour la préparation de spasmolytiques, d'anti-épileptiques, d'anxiolytiques et d'antidépressifs.

8. Préparations médicamenteuses, qui contiennent, outre les adjuvants de médicaments habituels, une proportion thérapeutiquement active des quinoxaline-2,3-(1H,4H)-dione I et I' suivant les revendications 1 à 5.
